(19) Europäisches Patentamt
European Patent Office
Office européen des brevets

(11) EP 0 475 527 B1

(12) EUROPEAN PATENT SPECIFICATION

(45) Date of publication and mention of the grant of the patent:
**25.02.1998 Bulletin 1998/09**

(21) Application number: **91202290.2**

(22) Date of filing: **06.09.1991**

(51) Int Cl.6: **C07D 243/04**, C07D 245/06, C07D 295/14, C07D 401/04, C07D 401/06, C07D 405/04, C07D 405/06, C07D 405/12, C07D 403/06, C07D 409/04, C07D 409/06 // C07D471/04, C07D487/04, C07D495/04, C07D213/38, C07D333/20, C07C211/27, C07C233/35, A61K31/55

(54) **Aryl-fused and hetaryl-fused-2,4-diazepine and 2,4-diazocine antiarrhythmic agents**

Arylkondensierte und heteroarylkondensierte 2,4-Diazepine und 2,4-Diazocine als antiarrhythmische Mittel

Aryl condensées et hétéroaryl condensées-2,4-diazépines et 2,4-diazocines comme agents anti-arythmiques

(84) Designated Contracting States:
**AT BE CH DE DK ES FR GB GR IT LI LU NL SE**

(30) Priority: **10.09.1990 US 580065**
**13.06.1991 US 743853**

(43) Date of publication of application:
**18.03.1992 Bulletin 1992/12**

(73) Proprietor: **SANOFI**
**75374 Paris Cédex 08 (FR)**

(72) Inventors:
- **Johnson, Robert Ed**
**New York, New York 10016 (US)**
- **Schlegel, Donald Charles**
**New York, New York 10016 (US)**

(74) Representative: **Le Guen, Gérard et al**
**CABINET LAVOIX**
**2, place d'Estienne d'Orves**
**75441 Paris Cédex 09 (FR)**

(56) References cited:
**EP-A- 0 066 303**
**EP-A- 0 389 765**
**DE-A- 1 770 135**
**US-A- 3 696 093**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 475 527 B1

Printed by Jouve, 75001 PARIS (FR)

## Description

The present invention relates to novel 4,5-dihydro-1H-2,4-aryl fused diazepines, and benzodiazocines to related diamines and aminoamides, to processes for preparing them and to methods and compositions for treating cardiac arrhythmias in mammals utilizing said 4,5-dihydro-1H-2,4-aryl fused diazepines, and benzodiazocines.

U.S. Patent 3,696,093 discloses a single 3,4-disubstituted benzodiazepine: 3,4-dimethyl-4,5-dihydro-1H-2,4-benzodiazepine hydrochloride.

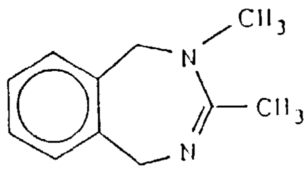

Also disclosed are 4,5-dihydro-1H-2,4-benzodiazepines monosubstituted in the 3 position with benzyl, dimethylaminoethyl, amino, 1-piperidinylmethyl, and phenyl. The compounds are said to be useful as cardiovascular agents, for example, in the treatment or management of the various forms of hypertension or of congestive heart failure. The patent does not disclose antiarrhythmic properties for the genus, and the single example of a disubstituted benzodiazepine was found to be inactive as an antiarrhythmic when tested in the protocol used to evaluate the compounds of the present invention.

Japanese application 59/013766 (CA 101:23612m) discloses a series of 1,2,4-trisubstituted-tetrahydrobenzodiazepines of general structure

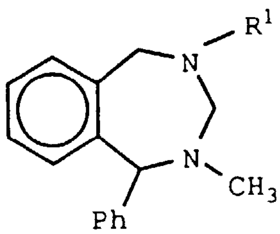

wherein $R^1$ is lower alkyl or phenethyl (opt. substd. with lower alkoxy). The compounds are said to be analgesics.

Elslager et al [J. Het. Chem. 5, 609-613 (1968)] describe the synthesis of a series of tetrahydrothiazolo-[3,2-b][2,4] benzodiazepines. The authors state that "None of the compounds possessed appreciable biological activity." As intermediates in the synthesis, they disclose

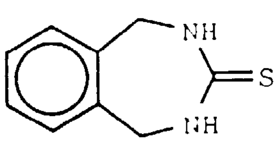

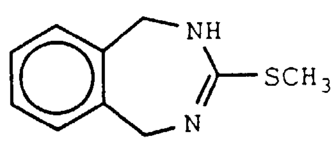

1,2,4,5-tetrahydro-3H-2,4-benzodiazepine-3-thione and 2,5,dihydro-3-(methylthio)-1H-2,4-benzodiazepine hydroiodide.

EP 66 303 concerns pharmaceuticals which may act as antiarrhythmic agents. These compounds are 2-azidomethyl-2,3-dihydro-3H-5-phenyl-1,4-benzodiazepine derivatives.

DE 1 770 135 discloses pharmaceuticals acting upon the cardiovascular system because of their antihypertensive properties which are 4,5-dihydro-1H-2,4-benzodiazepine derivatives of the formula :

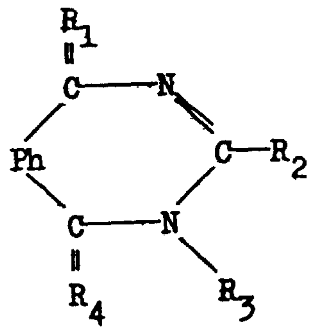

In the proposed examples, the total number of carbon atoms in $R_4$ plus $R_3$ plus $R_1$ is zero.

The present invention relates to compounds of the formula XXXVI:

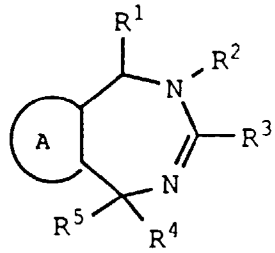

XXXVI

wherein

A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; benzyl; di-(lower-alkyl)amino, pyrrolidino; piperidino; morpholino; phenyl; or phenyl substituted with amino, nitro or lower-alkyl sulfonamido;

$R_3$ is $Y_p-(CH_2)_m-X_n-R^8$ wherein

Y is -NH-, -O-, -S-, or

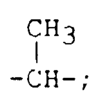

p is zero or one;

m is an integer from zero to seven;

X is -S-, -O-, $-SO_2-$,

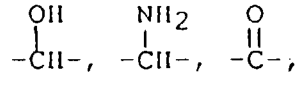

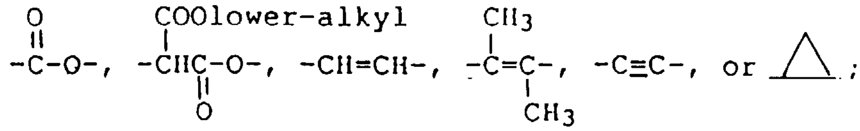

n is zero or one; and

$R^8$ is hydrogen;lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)- amino; di-(lower-alkyl) amino; or a 5- or 6- membered heterocycle containing one or two nitrogens, said heterocycle being unsubstituted or substituted with one lower-alkyl group; or X and $R^8$ taken together are cyclohexylidine;

$R^4$ is hydrogen; lower-alkyl; allyl; lower-alkoxylower-alkyl; acetyl; lower-alkoxy-carbonyl, lower-alkoxy-carbonyl-alkyl; or α-hydroxy-lower .-alkyl; and

$R^5$ is hydrogen; lower-alkyl; naphthyl; thienyl; pyridinyl; benzyl; phenyl; or phenyl having one or two substituents chosen from the group consisting of lower-alkyl,lower-alkoxy, halogen, hydroxyl, amino, di-(lower-alkyl)amino, lower-alkylsulfonamido and lower acylamino;

with the proviso that the total number of carbon atoms in $R^1$ plus $R^2$ plus $R^4$ plus $R^5$ must be five or greater.

The compounds of formula XXXVI are useful as antiarrhythmic agents.

Lower-alkyl as used herein describes linear, branched, or cyclic saturated carbon chains of eight or fewer carbon atoms; lower-alkoxy as used herein describes linear or branched alkyloxy substituents containing eight or fewer carbon atoms; halogen describes bromine, chlorine or fluorine.

In the text that follows, the substituents R are defined when initially presented and maintain that definition whenever they occur subsequently. Preferred compounds of the invention are those of the formula

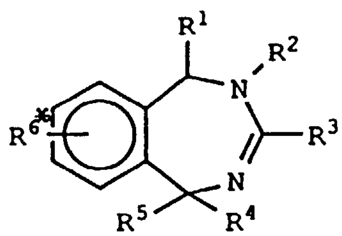

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; benzyl; di(lower-alkyl)amino, pyrrolidino; piperidino; morpholino; phenyl; phenyl substituted with amino, nitro or lower-alkyl sulfonamido;

$R_3$ is $Y_p-(CH_2)_m-X_n-R^8$ wherein

Y is -NH-, -O-, -S-, or

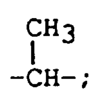

p is zero or one;

m is an integer from zero to seven;

X is -S-, -O-, , $-SO_2-$,

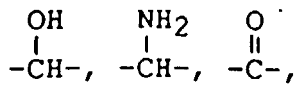

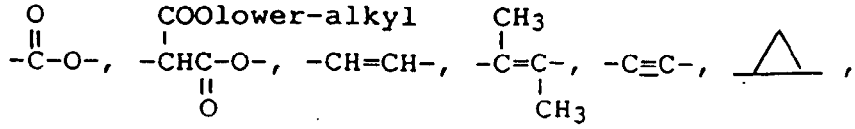

n is zero or one; and

$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)-amino; di-(lower-alkyl) amino; or a 5- or 6- membered heterocycle containing one or two nitrogens, said heterocycle being unsubstituted or substituted with one lower-alkyl group; or X and $R^8$ taken together are cyclohexylidine;

$R^4$ is hydrogen; lower-alkyl; allyl; lower-alkoxylower-alkyl; acetyl; lower-alkoxy-carbonyl, lower-alkoxy-carbonyl-alkyl ; or α-hydroxy-lower .-alkyl; and

$R^5$ is hydrogen; lower-alkyl; naphthyl; thienyl; pyridinyl; benzyl; phenyl; or phenyl having one or two substituents chosen from the group consisting of lower-alkyl,lower-alkoxy, halogen, hydroxyl, amino, di-(lower-alkyl)amino, lower-alkylsulfonamido and lower acylamino; and

$R^{6*}$ is one or two substituents chosen from the group consisting of hydrogen, lower-alkyl, lower-alkoxy, amino, halogen, nitro and lower-alkylsulfonamido;

with the proviso that the total number of carbon atoms in $R^1$ plus $R^2$ plus $R^4$ plus $R^5$ must be five or greater.

Particularly preferred compounds are :

4,5-Dihydro-4-methyl-1-phenyl-3-(2-phenylethyl)-1H-2,4-benzodiazepine,
R-(+)-4,5-Dihydro-4-methyl-1-phenyl-3-(2-phenyléthyl)-1H-2,4-benzodiazepine,
N-[4-[2-(4,5-Dihydro-4-methyl-1-phenyl-1H-2,4-benzodiazepin-3-yl)ethyl]phenyl]methanesulfonamide,
N-[4-[2-(4,5-Dihydro-3-ethyl-1-phenyl-1H-2,4-benzodiazepin-4-yl)ethyl]phenyl]methanesulfonamide,
N-[4,5-Dihydro-4-methyl-1-phenyl-3-(2-phenylethyl)-1H-2,4-benzodiazepin-8-yl]methanesulfonamide,
1-(4-Chlorophenyl)-4,5-dihydro-4-methyl-3-(2-phenylethyl)-1H-2,4-benzodiazepine,
3-[2-(4-Chlorophenyl)ethyl]-4,5-dihydro-4-methyl-1-phenyl-1H-2,4-benzodiazepine,
3-[2-(4-Chlorophenyl)ethyl] -4,5-dihydro-1,4-dimethyl-1-phenyl-1H-2,4-benzodiazepine,
N,N-Diethyl-4-[4,5-dihydro-5-methyl-1-phenyl-1H-2,4-benodiazepin-4-yl]benzenesulfonamide
4,5-Dihydro-1-(4-hydroxyphenyl)-4-methyl-3-(2-phenylethyl)-1H-2,4-benzodiazepine,
4,5-Dihydro-3-[2-(4-hydroxyphenyl)ethyl]-4-methyl-1-phenyl-1H-2,4-benzodiazepine or acid-additional salts thereof according to claim 2.

A second genus of preferred compounds comprises the compounds of formula :

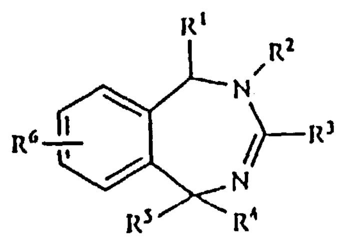

I

wherein

$R^1$ is hydrogen, lower-alkyl, phenyl, phenyl having one or two substituents chosen from the group consisting of

lower-alkyl and lower-alkoxy, naphthyl, thienyl, pyridinyl, or benzyl;
$R^2$ is hydrogen; lower-alkyl; benzyl; phenyl; phenyl substituted with halogen, lower-alkyl or lower-alkoxy; or $R^2$ is $-CH_2CH_2R^7$ where $R^7$ is lower-alkoxy; phenyl, benzyl, di-(lower-alkyl)amino, pyrrolidino, piperidino or morpholino;
$R^3$ is $Y_p-(CH_2)_m-X_n-R^8$ wherein
Y is -NH-, -O-, -S-, or

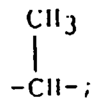

p is zero or one;
m is an integer from zero to seven;
X is -S-, -O-, $-SO_2-$,

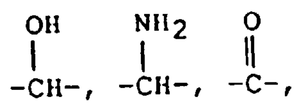

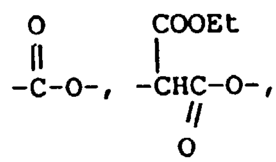

or -CH=CH-
n is zero or one; and
$R^8$ is lower-alkyl; phenyl; phenyl having one or two substituents chosen from the group consisting of halogen, lower-alkyl, and lower-alkoxy; or when n is zero and m is other than zero, $R^8$ is additionally hydrogen; halogen; benzyl(lower-alkyl)-amino; di-(lower-alkyl)amino; or a 5- or 6-membered heterocycle containing one or two nitrogens, said heterocycle being unsubstituted or substituted with one lower-alkyl group;
$R^4$ is hydrogen; lower-alkyl; allyl; lower-alkoxylower-alkyl; acetyl; lower-alkoxycarbonyl-alkyl; lower alkyl carboxyl; or $\alpha$-hydroxy-lower-alkyl;
$R^5$ is hydrogen; lower-alkyl; phenyl; phenyl having one or two substituents chosen from the group consisting of lower-alkyl,lower-alkoxy, halogen, amino, di-(loweralkyl)amino, and lower acylamino; naphthyl; thienyl; pyridinyl; or benzyl; and
$R^6$ is one or two substituents chosen from the group consisting of hydrogen, lower-alkyl, lower-alkoxy, and halogen;

with the proviso that the total number of carbon atoms in $R^1$ plus $R^2$ plus $R^4$ plus $R^5$ must be five or greater.

The compounds of formula III

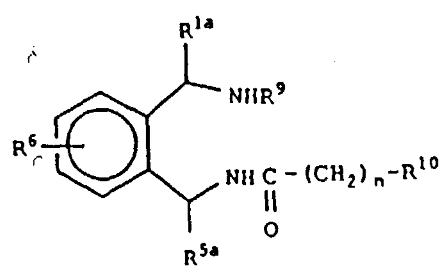

III

wherein

$R^{1a}$ is hydrogen, lower-alkyl or phenyl;
$R^{5a}$ is hydrogen; phenyl; phenyl having one or two substituents chosen from the group consisting of halogen, lower-alkyl and lower-alkoxy;
naphthyl; thienyl; pyridinyl; or benzyl;
$R^9$ is hydrogen, lower-alkyl, benzyl, phenethyl or [di-(lower-alkyl)amino]lower-alkyl;
$R^{10}$ is hydrogen; lower-alkyl; phenyl; phenyl substituted with halogen, lower-alkyl, lower-alkylsulfonamido or lower-alkoxy; phenoxy; phenoxy substituted with halogen, lower-alkyl or lower-alkoxy; benzyl; or $R^{10}$ is a 5-or 6-membered heterocycle containing one or two nitrogens, optionally substituted with a lower alkyl group; and
n is zero or one,

are all useful as intermediates in the synthesis of compounds of formula I.
The compounds of formula XXXVII

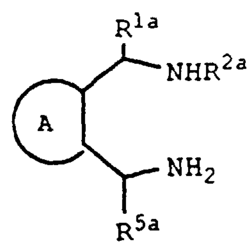

XXXVII

wherein

$R^{2a}$ is lower-alkyl; benzyl; phenyl; phenyl substituted with halogen, lower-alkyl or lower-alkoxy; or
$R^{2a}$ is $-CH_2CH_2R^7$ where $R^7$ is lower-alkoxy, phenyl, benzyl, di(lower-alkyl)amino, pyrrolidino, piperidino, or morpholino; and at least one of $R^{1a}$ and $R^{5a}$ is phenyl, substituted phenyl, benzyl, naphthyl, thienyl or pyridinyl,

are useful as intermediates in the synthesis of compounds of formula XXXVI,
The invention also relates to processes for the production of benzodiazepines of formula V

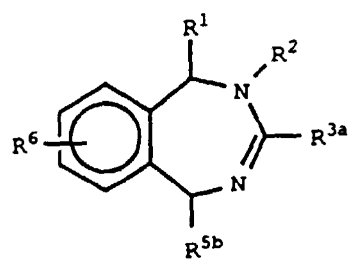

V

which comprises a) reacting a compound of formula VId

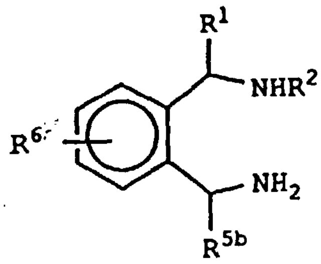

VI d

wherein $R^1$ is hydrogen, lower-alkyl, benzyl, naphthyl, thienyl, pyridinyl, phenyl, or phenyl having one or two substituents chosen from the group consisting of lower-alkyl and lower-alkoxy;
$R^2$ is hydrogen; lower-alkyl; benzyl; phenyl; phenyl substituted with halogen, lower-alkyl or lower-alkoxy; or
$R^2$ is $-CH_2CH_2R^7$ where $R^7$ is lower-alkoxy, benzyl, di-(lower-alkyl)amino, pyrrolidino, piperidino morpholino, phenyl, or phenyl substituted with amino, nitro or lower-alkylsulfonamido;
$R^{3a}$ is $(Y^a)_p-(CH_2)_m-(X^a)_n-R^8$ wherein
$Y^a$ is -O-, -S- or

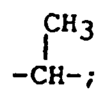

p is zero or one;
m is an integer from zero to seven;
$X^a$ is -S-, $-SO_2-$, -O-, or -CH=CH-;
n is zero or one; and
$R^8$ is hydrogen;lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)-amino; di-(lower-alkyl) amino; or a 5- or 6- membered heterocycle containing one or two nitrogens, said heterocycle being unsubstituted or substituted with one lower-alkyl group; or X and $R^8$ taken together are cyclohexylidine;
$R^{5b}$ is hydrogen; lower-alkyl; phenyl; phenyl having one or two substituents chosen from the group consisting of lower-alkyl, lower-alkoxy, and halogen; naphthyl; thienyl; pyridinyl; or benzyl;
$R^6$ is one or two substituents chosen independently from the group consisting of hydrogen, lower-alkyl, lower-alkoxy, halogen, nitro, and lower-alkylsulfonamido;

1) with an iminoether, or a salt thereof, of formula

$$R^{3a}C(OR^{12})NH$$

wherein $R^{12}$ is methyl or ethyl;
2) with an orthoester of formula $R^{3a}C(OR^{12})_3$
3) with an ester of formula $R^{3a}COOR^{12}$ in the presence of trialkylaluminum, or
b) reacting a compound of formula XXVII or XXVIII

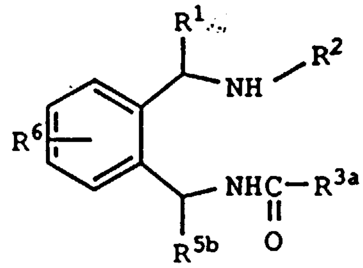

XXVII

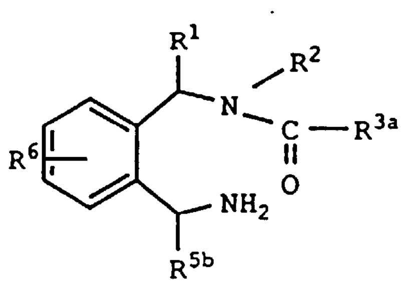

XXVIII

with a trialkylaluminum.

The invention also relates to a process for preparing a compound of formula Ia

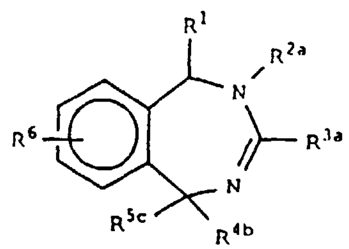

Ia

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;
$R^{2a}$ is lower-alkyl; benzyl; phenyl; phenyl substituted with halogen, lower-alkyl or lower-alkoxy; or
$R^{2a}$ is $-CH_2CH_2R^7$ where $R^7$ is lower-alkoxy, phenyl, benzyl, di-(lower-alkyl)amino, pyrrolidino, piperidino, or morpholino;
$R^{3a}$ is $(Y^a)_p-(CH_2)_m-(X^a)_n-R^8$ wherein
$Y^a$ is -O-, -S- or

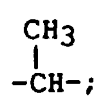

p is zero or one;
m is an integer from zero to seven;
$X^a$ is -S-, $-SO_2-$, -O- or -CH=CH-;
n is zero or one; and
$R^8$ is hydrogen;lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)amino; di(lower-alkyl)-amino; or
$R^8$ is a 5- or 6-membered heterocycle containing one or two nitrogens, said heterocycle being unsubstituted or substituted with one lower-alkyl group; or X and $R^8$ taken together are cyclohexylidine;
$R^{4b}$ is lower-alkyl; allyl; lower-alkoxy-lower-alkyl; acetyl; lower-alkoxy-carbonyl; lower-alkyl-carboxy-alkyl; or α-hydroxy-lower-alkyl;
$R^{5c}$ is phenyl; phenyl having one or two substituents chosen from the group consisting of lower-alkyl, lower-alkoxy, and halogen; naphthyl; thienyl; or pyridinyl; and
$R^6$ is one or two substituents chosen independently from the group consisting of hydrogen, lower-alkyl, lower-alkoxy, halogen, nitro and lower-alkylsulfonamido ;

which comprises reacting a compound of formula Va

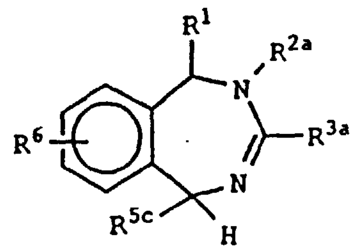

Va

with a strong base and reacting with a) a lower-alkyl aldehyde or b) a compound of formula $R^{4b}$ Z wherein Z is a group subject to nucleophilic displacement.

The invention also relates to a process for preparing a compound of formula VII

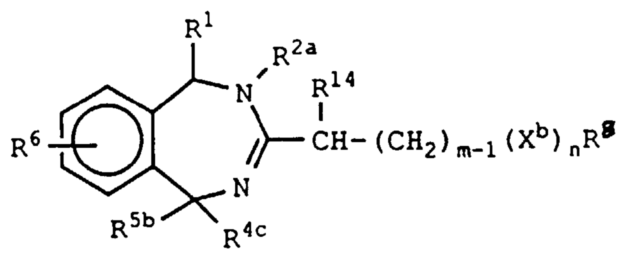

VII

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;
$R^{2a}$ is lower-alkyl; benzyl; phenyl; phenyl substituted with halogen, lower-alkyl or lower-alkoxy; or
$R^{2a}$ is $-CH_2CH_2R^7$ where $R^7$ is lower-alkoxy, phenyl, benzyl, di-(lower-alkyl)amino, pyrrolidino, piperidino, or morpholino;
m is an integer from zero to seven;
$X^b$ is -S-, $-SO_2-$, -O-, -CH=CH-,

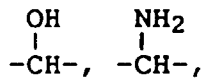

or

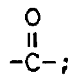

n is zero or one;

$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)amino; di(lower-alkyl)-amino; or

$R^8$ is a 5- or 6-membered heterocycle containing one or two nitrogens, said heterocycle being unsubstituted or substituted with one lower-alkyl group; or X and $R^8$ taken together are cyclohexylidine;

$R^{4c}$ is lower-alkyl , allyl, or lower-alkoxylower-alkyl;

$R^{5c}$ is phenyl; naphthyl; thienyl; pyridinyl; or phenyl having one or two substituents chosen from the group consisting of lower-alkyl, lower-alkoxy, and halogen;

$R^6$ is one or two substituents chosen independently from the group consisting of hydrogen, lower-alkyl, lower-alkoxy, halogen; nitro and lower-alkylsulfonamido; and

$R^{14}$ is hydrogen or methyl,

which comprises reacting a compound of formula VIIa

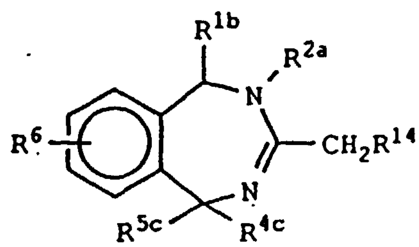

VIIa

with a strong base followed by reaction with an electrophile chosen from the group consisting of $R^8COOR^{12}$, $R^8CHNR^{12}$, $R^8CHO$, $R^8SSR^8$, $R^8(X^b)_n(CH_2)_{m-1}Z$, wherein $R^{12}$ is methyl or ethyl and Z is a group subject to nucleophilic displacement.

The compounds of formula VIa :

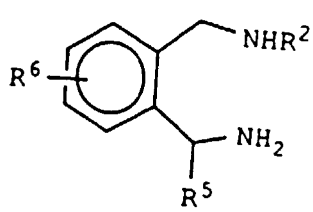

VIa

may be prepared by reacting a compound of formula VIII or IX

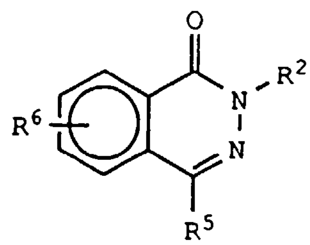

VIII

or

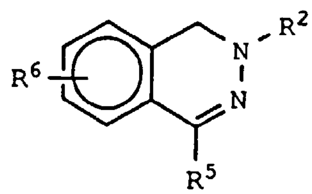

IX

with an excess of diborane.

The compounds of formula VIb

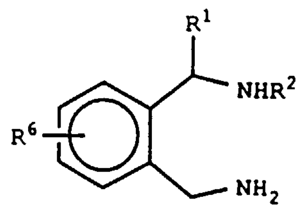

VIb

may be prepared by reacting a compound of formula X or XI

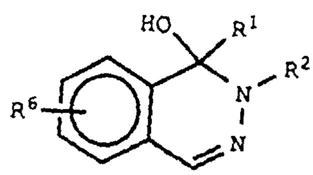

X

or

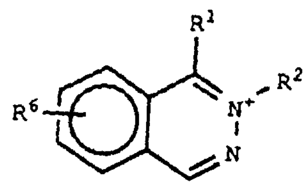

XI

with an excess of diborane.

The compounds of formula IVa

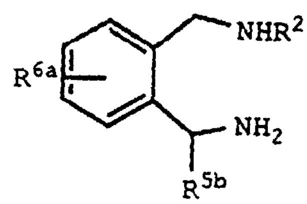

IVa

may be prepared by reducing a compound of formula VIIIa

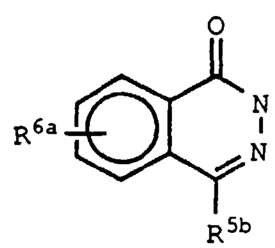

VIIIa

sequentially with an aluminum hydride, hydrogen in the presence of a noble-metal catalyst and hydrogen in the presence of a nickel catalyst.

Other aspects of the invention comprise the use of the diazepines of the invention for the preparation of a medicament for the treatment of cardiac arrhythmia in a patient in need of such treatment.

A general synthesis of compounds of the invention sharing general formula XXXVI may be outlined as shown in Scheme A.

Scheme A

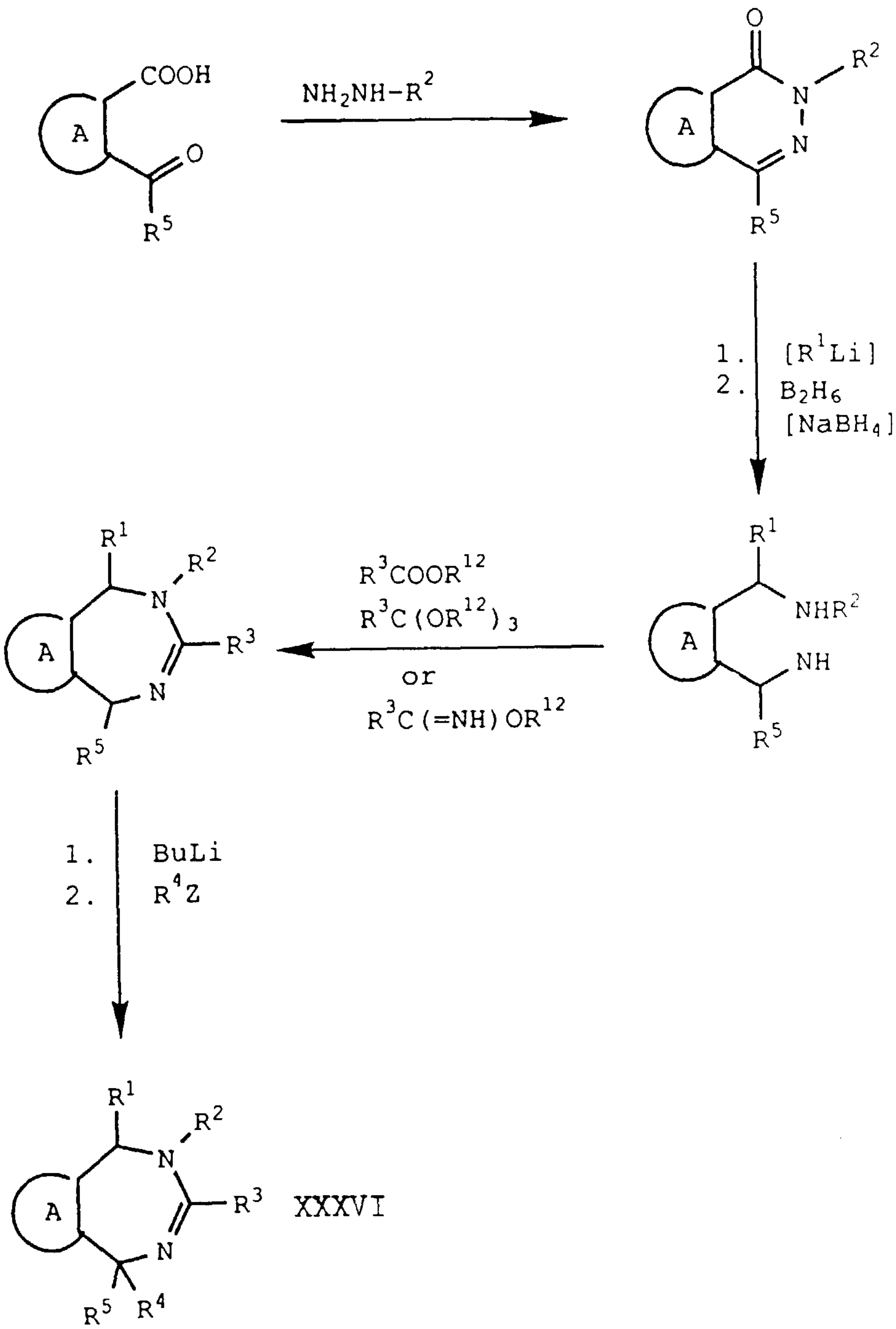


XXXVI

This is more particularly illustrated in Scheme B wherein A is phenyl or substituted phenyl:

Scheme B

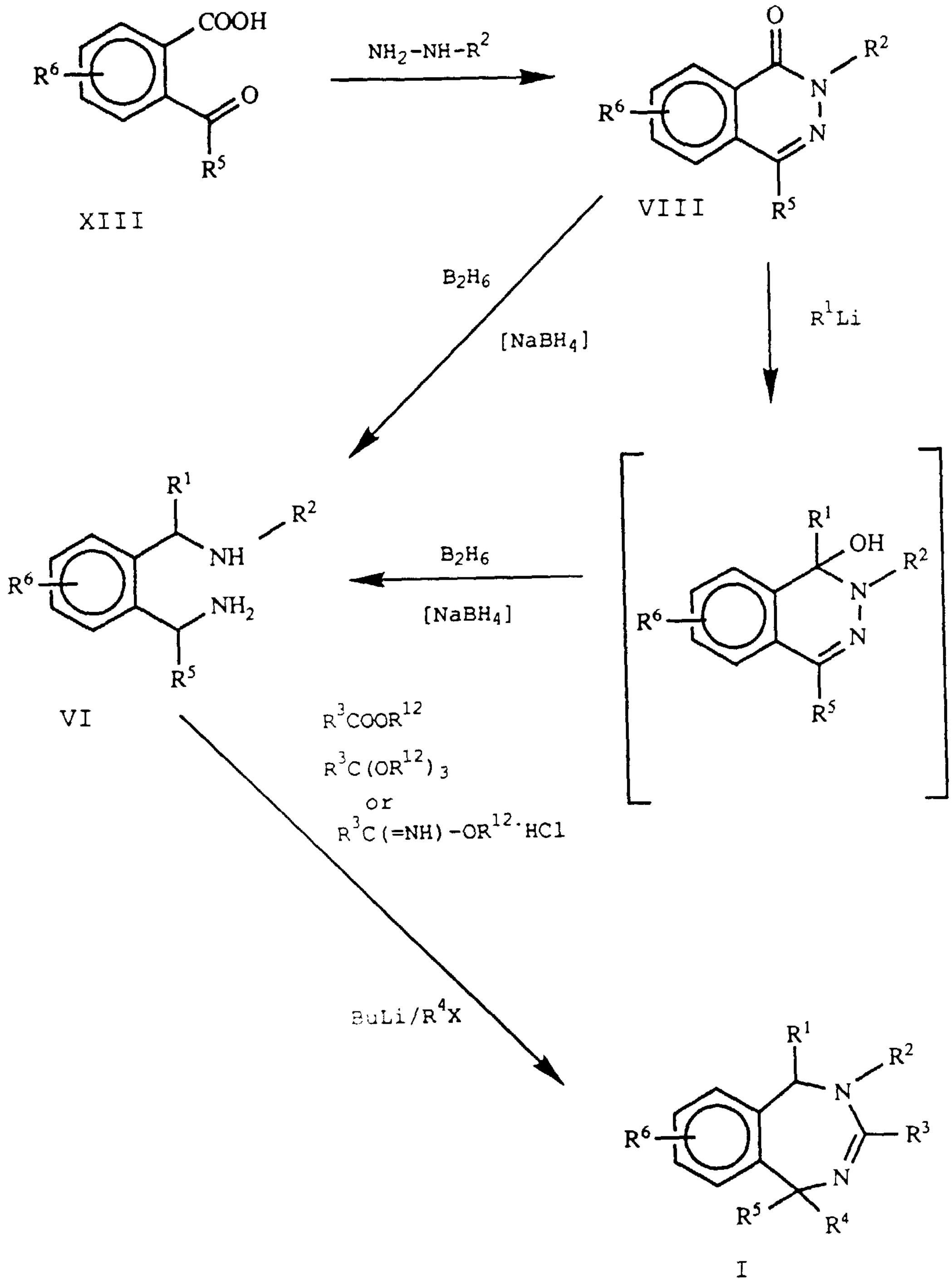


A suitably substituted γ-oxo-acid of formula XIII is reacted with a suitably substituted hydrazine to form a phthalazinone (VIII). In the case where it is desired that $R^1$ be other than hydrogen, the phthalazinone is reacted with a slight excess of a suitable alkyl or aryllithium compound in an inert solvent, preferably THF, at -78° to 0°C, preferably about -65°C, and the resulting adduct is reduced as described below without isolation. In the case where $R^1$ is hydrogen, the phthalazinone (VIII) is reduced directly to the diamine (VI) with 3.5 to 9.0 equivalents of diborane in an inert solvent, preferably THF, at 20° to 100°, preferably 67°C. A catalytic amount of sodium borohydride and some diglyme may be added.

The diamine (VI) may be condensed in one of three ways to produce the benzodiazepine (I,$R_4$=H): (1) the free base of the diamine in acetic acid is treated with five to seven equivalents of the appropriate orthoester $R^3C(OR^{12})_3$

at 0-50°C, preferably 25°C, or the diacid salt of the diamine, preferably the dihydrochloride salt, in an inert solvent is treated with five to seven equivalents of an appropriate orthoester plus one to two equivalents of a weak base, preferably sodium or potassium acetate; (2) a diacid salt of the diamine (VI), preferably the dihydrochloride salt, in an inert solvent, preferably methanol, is treated with two to three equivalents of the appropriate iminoether hydrochloride and about two equivalents of a weak base, preferably sodium acetate, at 0 to 60°C, preferably 25°C, or the free base of the diamine (VI) in an inert solvent, preferably methanol, is treated with two to three equivalents of the appropriate iminoether hydrochloride and two to three equivalents of a weak acid, preferably acetic acid, at 0° to 60°C, preferably 25°C, or (3) a diamine or a diacid salt of the diamine, preferably the dihydrochloride salt, in an inert solvent, preferably toluene, is treated with slightly more than two equivalents of trimethylaluminum at -30°to +110°C, followed by treatment with 1 to 1.5 equivalents of a lower-alkyl ester of the appropriate acid ($R^3COOR^{12}$).

In the case where it is desired that $R^4$ be other than hydrogen, the diazepine (I, $R_4$=H)may be reacted with a strong base such as butyllithium and the resulting anion reacted with an appropriate electrophile.

The reactions described for the compounds of formula XXXVI wherein A is a phenyl ring are equally applicable to the compounds wherein A is other than phenyl. The starting materials are likewise commercially available or known.

Compounds of formula V, a subset of benzodiazepines of formula I, may be prepared by the ring closure of aminoamides. A monosalt of the aminoamide XXVII or XXVIII, preferably the monohydrochloride in an inert solvent, preferably toluene, is treated with a slight excess, preferably about 1.1 equivalents, of trimethylaluminum at 0° to 150°, preferably about 110° to produce a benzodiazepine of formula V:

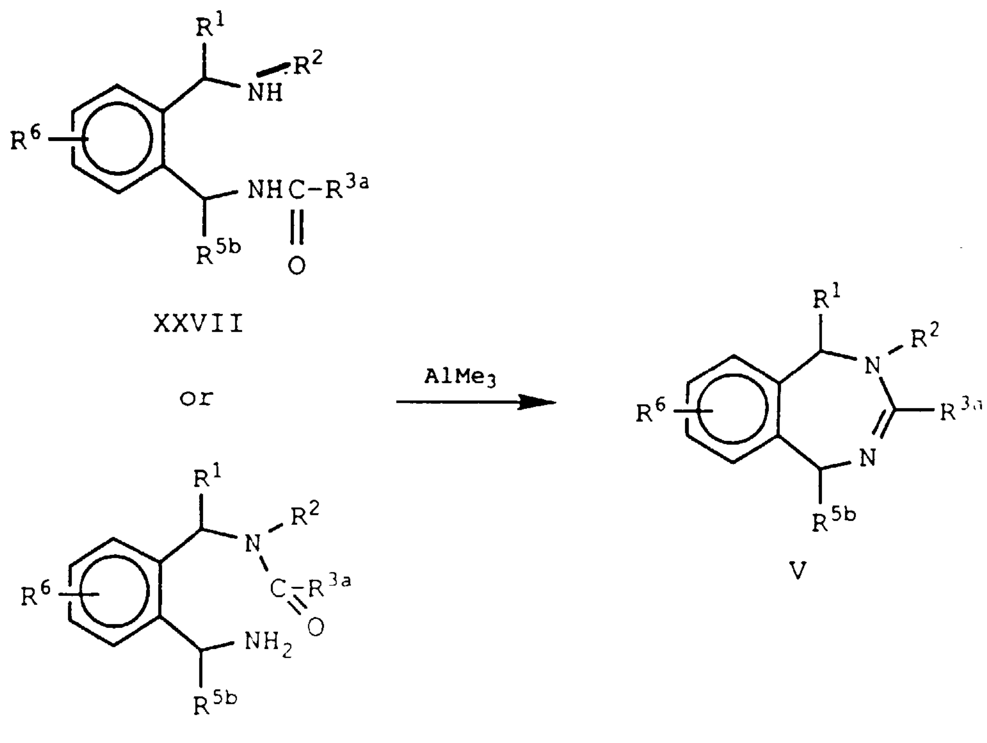


XXVII

or

XXVIII

V

Aminoamides of formula XXVII may be obtained as described in Examples 177-184 by incomplete cyclization or, as described in General Method U, by hydrolytic cleavage of benzodiazepines. Aminoamides XXVII or XXVIII or mixtures of the two may also be obtained by procedures well known in the art for condensing acids of formula $R^{3a}COOH$ with amines of formula VId

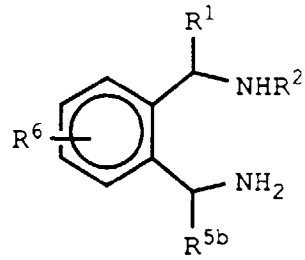

VId

In the case of compounds of formula I where p (in $R^3$) is one and $Y^b$ is -NH-, -S-, or -O-, the compounds may be made by an alternate route from the diamine VI shown in Scheme C:

Scheme C

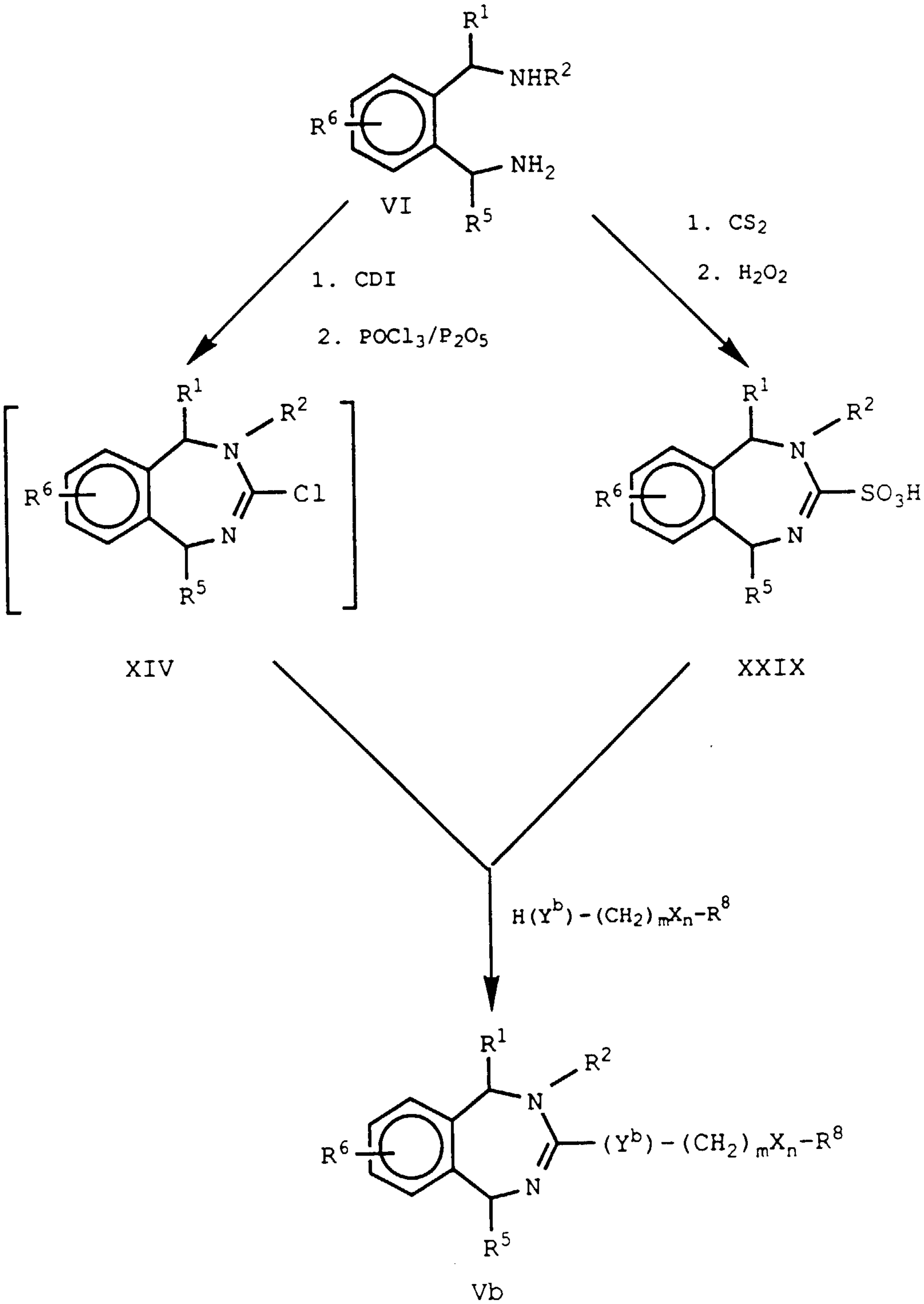


The diamine (VI) is reacted with carbonyl diimidazole (CDI) in an inert solvent, preferably chloroform, at ambient temperature to produce a benzodiazepin-3-one, which is treated with a large excess, preferably about 13 equivalents, of phosphorus oxychloride and preferably about 0.25 equivalents of phosphorus pentoxide to produce a 3-chlorobenzodiazepine (XIV). The 3-chlorobenzodiazepine is then reacted, usually without isolation, with the appropriate nucle-

ophile, $R^8X_n(CH_2)_m(Y^b)H$, to produce the benzodiazepines of structure Vb.

Alternatively the diamine VI is reacted with preferably about one equivalent of carbon disulfide in an inert solvent, preferably 2-propanol, at 0°to 100°C, preferably at about 20° to 85°C and the resulting carbamodithioic acid is treated with a catalytic amount of an acid, preferably hydrochloric acid, in an inert solvent, preferably ethanol, at 0 to 100°C, preferably at about 78°C, to produce a tetrahydrobenzodiazepine-3-thione. The thione is oxidized with slightly more than three equivalents of 30% hydrogen peroxide according to the procedure of Maryanoff et al. [J. Org. Chem. 51, 1882 (1986)] to produce the sulfonic acid XXIX. The sulfonate may then be displaced by an appropriate nucleophile, as before, optionally in an inert solvent at 0°-100°C. Example 153 illustrates an alternative, lower-yield conversion of the thione to the compounds of formula Vb.

In the case where all of $R^2$, $R^4$ and $R^5$ are other than hydrogen, the subgenus of compounds of formula VII wherein $R^3$ is attached to the benzodiazepine ring through a carbon (i.e. p in $R^3$ is zero and $R^3$ is $-CH_2(CH_2)_{m-1}X_nR^8$ or p is one and Y is

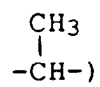

may be made alternately

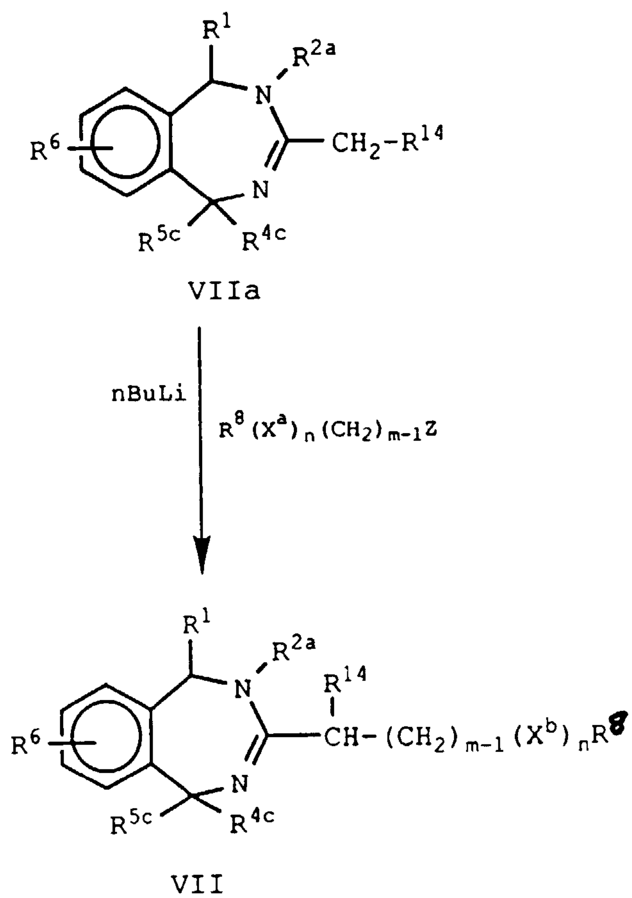


by treatment of a compound of formula VIIa wherein $R^{14}$ is hydrogen or methyl in an inert solvent, preferably THF, at -78° to +25°, with a slight excess, preferably about 10 to 20%, of a strong base, preferably n-butyllithium followed by a slight excess, preferably 10-50%, of an appropriate electrophile. The electrophile can be of the form $R^8-(X^a)_n-(CH_2)_{m-1}-Z$ wherein Z is a group that is readily displaced by an anion, such as a halogen, sulfide, sulfonate, ester, etc. or in the case where m minus one is zero it can take the form of an aldehyde, ketone or imine so that addition of the anion to the electrophile followed by quenching with a proton source results in the overall addition of the elements of VIIa to the electrophile.

In cases where $R^3$ is attached to the benzodiazepine via a heteroatom-methylene link (XV), that is $R^3$ is of the form $-CH_2(X^c)_nR^8$ wherein $X^c$ is -S-, -O- or $-SO_2-$ (i.e. in formula XXXVI, X is -S-, -O- or $-SO_2-$, p is zero, m is one and n is one when $R^8$ is lower-alkyl, phenyl or substituted phenyl; n is zero when $R^8$ is amino or an N- attached heterocycle) the compounds may conveniently be synthesized from the corresponding chloromethyl species (XVI).

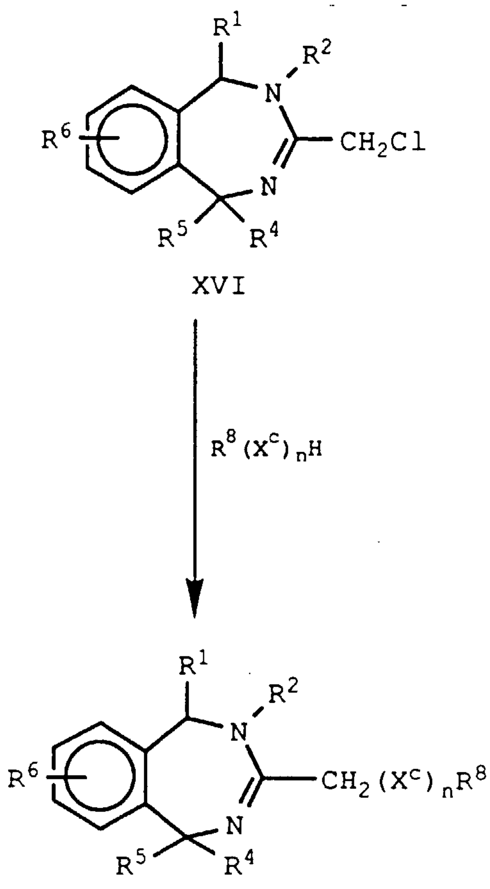


XVI

XV

The 3-chloromethyl-2,4-benzodiazepine (XVI) in an inert solvent, preferably chloroform when the heteroatom is nitrogen and methanol or acetonitrile when the heteroatom is sulfur, is treated with one to three equivalents of the $R^8(X^c)_nH$ species at 0° to 100°C, preferably at 25° to 65°C. The chloromethyl benzodiazepine XVI can be synthesized directly from the diamine VI by condensation with ethyl 2-chloroethanimidate or in the case where all of $R^2$, $R^4$ and $R^5$ are other than hydrogen, the chloromethyl benzodiazepine (XVI) may be synthesized from the corresponding 3-methylbenzodiazepine by anion formation as described in the foregoing paragraph and quenching with about 1.1 equivalents of hexachloroethane.

Compounds of formula IV may be prepared by the reduction and cleavage of phthalazinones and phthalazines as shown in Scheme F.

Scheme F

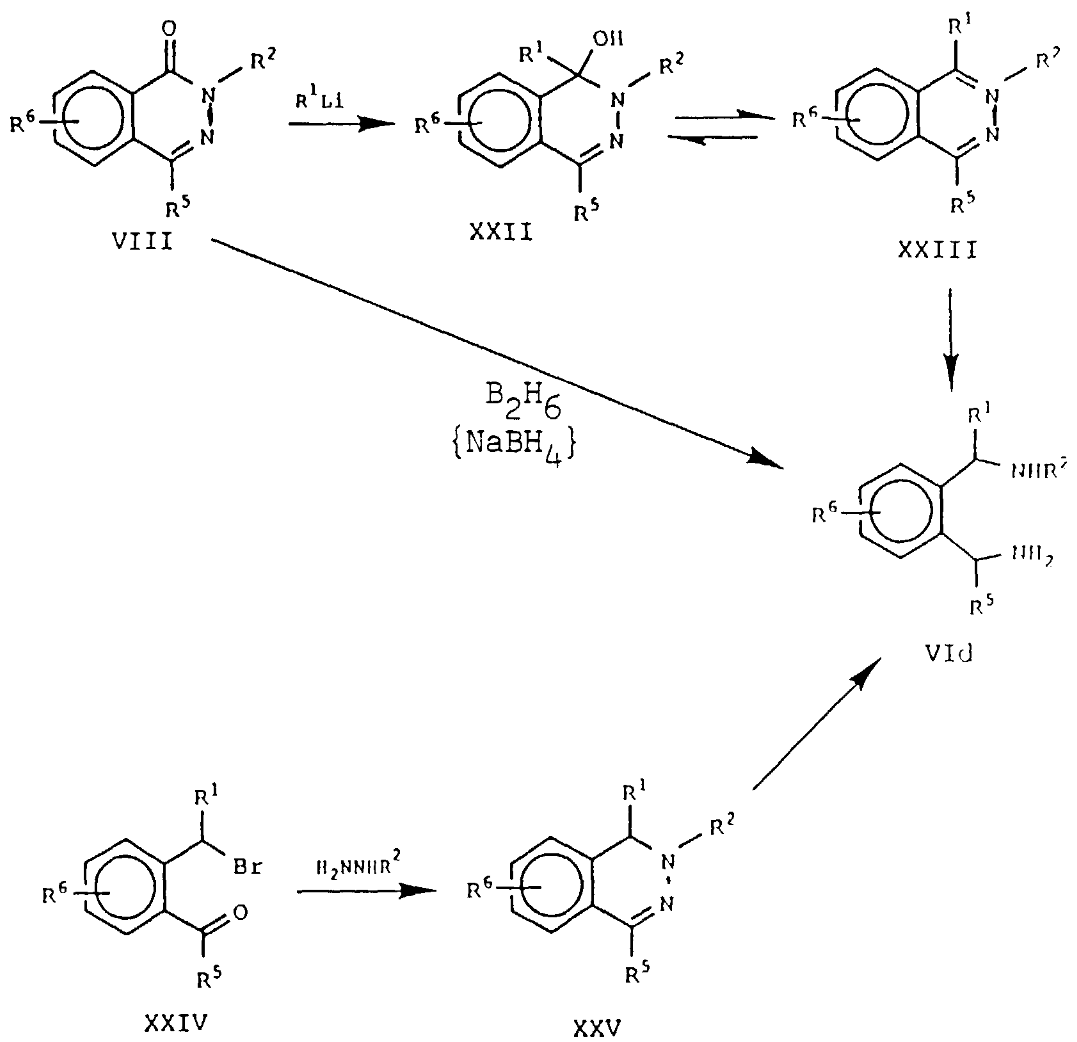


VIII XXII XXIII VId XXIV XXV

A phthalazinone of formula VIII is reacted with 3.5 to 9.0 equivalents of diborane in an inert solvent, preferably THF, at 20° to 100°, preferably about 67°, to produce diamines of the formula VId wherein $R^1$ is hydrogen. A catalytic amount of sodium borohydride in diglyme may be added. If it is desired that $R^1$ be other than hydrogen, phthalazines of formulas XXII, XXIII and XXV may be reduced in like fashion. The phthalazines XXII and XXIII may be obtained from the corresponding phthalzinones by reaction with a suitable alkyllithium or aryllithium in an inert solvent, preferably THF, at -78° to 0°, preferably about -65°. The resulting phthalazine may exist as the hydroxyphthalazine XXII or may spontaneously eliminate the elements of water to form the phthalazinium species XXIII. The phthalazines of formula XXV may be synthesized by the condensation of the appropriate hydrazine with a γ-haloketone, preferably a γ-bromoketone.

In the case wherein in formula VIII $R^2$ is hydrogen, the simple reduction with diborane described above is so slow as to be of less practical use than a three-step reduction:

Scheme G

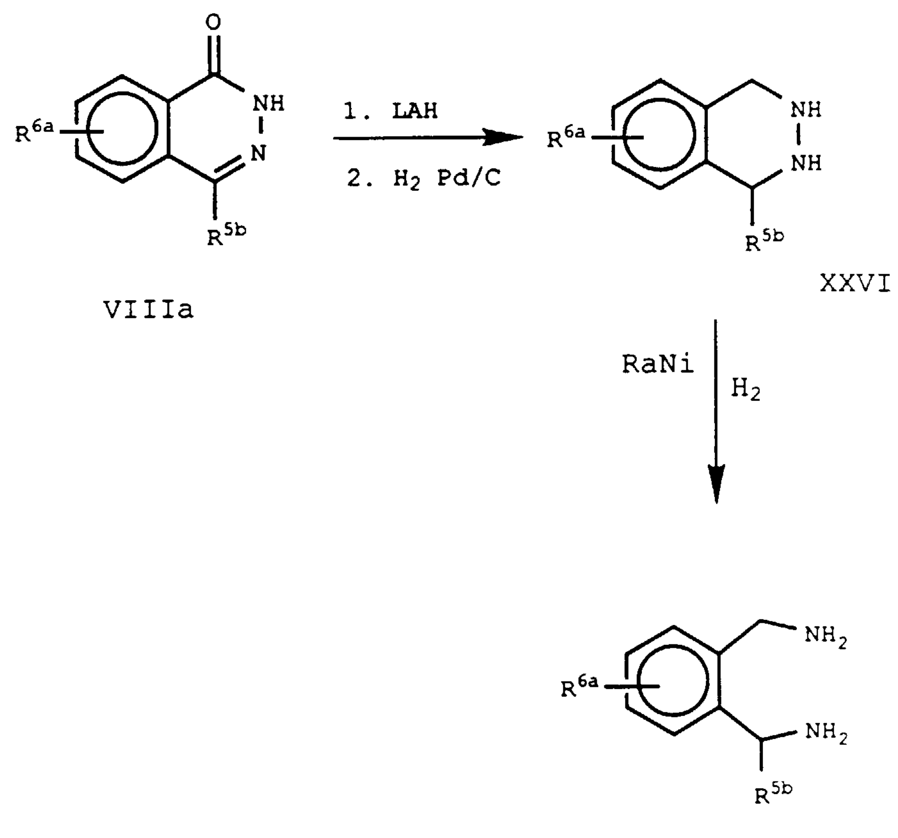


VIIIa

XXVI

IVa

The phthalazinone VIIIa is treated with about two equivalents of an aluminum hydride reducing agent, preferably lithium aluminum hydride, in an inert solvent, preferably THF, at 20° to 120°, preferably about 65°. The resulting dihydrophthalazine is reacted with hydrogen in an inert solvent, preferably a lower alkanol, most preferably ethanol, in the presence of a palladium catalyst at 20° to 60°, preferably 40°-50°, preferably at about 3 atmospheres pressure. The resulting tetrahydrophthalazine (XXVI) is reacted with hydrogen in an inert solvent, preferably methanol, in the presence of a Raney nickel catalyst at 20° to 80°, preferably about 65°, preferably at about 3 atmospheres pressure.

Many of the compounds of the invention are asymmetric at C-1 of the benzodiazepine. In some cases there may be an advantage to using one or the other enantiomer for the treatment of arrhythmia. Single enantiomers may be synthesized from chiral starting materials or the racemates may be resolved by methods well known in the art, such as chromatography on chiral media or recrystallization of diastereomeric salts.

The compounds of the invention are useful both in the free base form and the form of acid-addition salts, and both forms are within the purview of the invention. The acid-addition salts are in some cases a more convenient form for use, and in practice the use of the salt form inherently amounts to the use of the base form. The acids which can be used to prepare the acid-addition salts include preferably those which produce, when combined with the free base, medicinally acceptable salts, that is, salts whose anions are relatively innocuous to the animal organism in medicinal doses of the salts so that the beneficial properties inherent in the free base are not vitiated by side effects ascribable to the anions. In practicing the present invention, it is convenient to form the hydrochloride, fumarate, toluenesulfonate, hydrogen sulfate, methanesulfonate, or maleate salts. However, other appropriate medicinally acceptable salts within the scope of the invention are those derived from other mineral acids and organic acids. The acid-addition salts of the basic compounds are prepared either by dissolving the free base in aqueous alcohol solution containing the appropriate acid and isolating the salt by evaporating the solution, or by reacting the free base and an acid in an organic solvent, in which case the salt separates directly, is precipitated with a second organic solvent, or can be obtained by concentration of the solution. Although medicinally acceptable salts of the basic compounds are preferred, all acid-addition

salts are within the scope of the present invention. All acid-addition salts are useful as sources of the free base form even if the particular salt per se is desired only as an intermediate product, as, for example, when the salt is formed only for purposes of purification or identification, or when it is used as an intermediate in preparing a medicinally acceptable salt by ion exchange procedures.

The structures of the compounds of the invention were established by the mode of synthesis, by elemental analysis, and by infrared, nuclear magnetic resonance, and mass spectroscopy. The course of the reactions and the identity and homogeneity of the products were assessed by thin layer chromatography (TLC) and high-pressure liquid chromatography (HPLC). The starting materials are either commercially available or may be prepared by procedures well known in the art.

In the following procedures, melting points are given in degrees C and are uncorrected.

In the examples which follow, Me is methyl, Et is ethyl, Ph is phenyl, Bzl is benzyl, iPr is isopropyl, tBu is t-butyl, OAc is acetyl, THF is tetrahydrofuran, hex is hexane, IPA is isopropylamine, DMF is dimethylformamide, and TMS is trimethylsilyl.

The invention will be described by way of illustration only with reference to the following Examples.

TABLE A

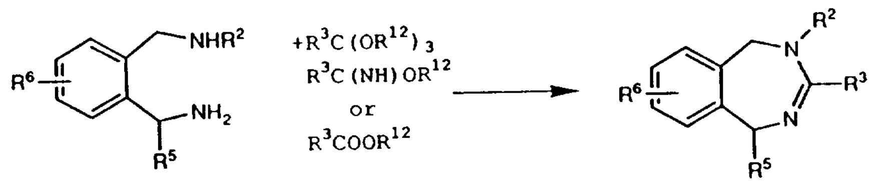


| Example No. | $R^2$ | $R^3$ | $R^5$ | $R^6$ | Method | Yield % | Melting Range | Salt | Recrystallized From |
|---|---|---|---|---|---|---|---|---|---|
| 1 | Me | Ph | Ph | H | A | 63 | 114-115 | free base | hexane |
| 2 | Me | Me | Ph | H | A | 27 | 177-178 | maleate | MeCN |
| 3 | Me | Et | 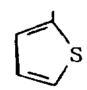 | H | B | 26 | 213-215 | HCl | MeCN |
| 4 | iPr | Et | Ph | H | C | 71 | 232-234 | HCl | EtOAc/ether |
| 5 | Bzl | Et | Ph | H | C | 37 | 222-224 | HCl ¼$H_2O$ | EtOH/MeCN/ether |
| 6 | Me | $-CH_2Cl$ | Ph | H | D | 30-95 | 129-131 | free base | tBuOMe/hexane |
| 7 | 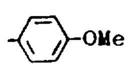 | Et | Me | H | A | 59 | 207-209 | HCl | EtOH/tBuOMe |

TABLE A

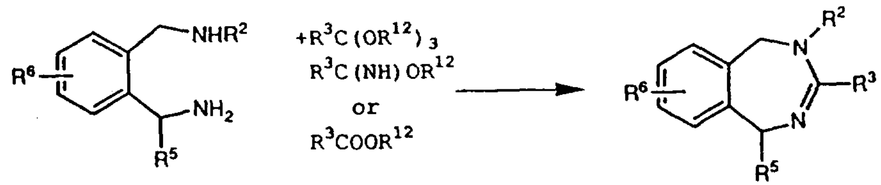


| Example No. | $R^2$ | $R^3$ | $R^5$ | $R^6$ | Method | Yield % | Melting Range | Salt | Recrystallized From |
|---|---|---|---|---|---|---|---|---|---|
| 8 | Me | Et | Ph | H | A | 40 | 198-200 | HCl | MeCN/tBuOMe |
| 8A | | | | | | | 163-165 | maleate | acetone |
| 8B | | | | | | | 98-100 | free base | iPrOAc |
| 8C | | | | | | | 201-202 | ½ fumarate | acetone |
| 8D | | | | | | | 178-179 | toluene-sulfonate | iPrOH |
| 8E | | | | | | | 172-174 | methane-sulfonate | EtOAc |
| 9 | Me | H | Ph | 7,8-diOMe | A | 48 | 148-150 | maleate | EtOAc/MeCN |
| 10 | Me | nPr | Ph | H | A | 49 | 218-221 | HCl | MeCN/tBuOMe |

TABLE A

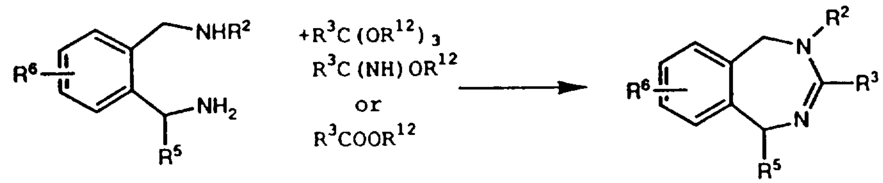


| Example No. | $R^2$ | $R^3$ | $R^5$ | $R^6$ | Method | Yield % | Melting Range | Salt | Recrystallized From |
|---|---|---|---|---|---|---|---|---|---|
| 11 | Me | nBu | Ph | H | A | 46 | 212-214 | HCl | MeCN/tBuOMe |
| 12 | $CH_2CH_2OMe$ | Et | Ph | H | A | 44 | 186-189 | HCl | iPrOH/tBuOMe |
| 13 | Me | $CH_2CH_2$-$cC_6H_{11}$ | Ph | H | D | 80 | 146 | HCl | MeCN/ether |
| 14 | Me | $-(CH_2)_4CH_3$ | Ph | H | D | 27 | 197-198 | HCl | acetone/ether |
| 15 | H | $-CH_2CH_2Ph$ | Ph | H | D | 90 | 148-149 | free base | $CH_2Cl_2$/hexane |
| 16 | Me | Et | Ph | 6-F | A | 50* | 152-154 | maleate | MeOH/ether |
| 17 | Me | Et | Ph | 7-F | A | 53 * | 185-187 | maleate | MeOH/ether |
| 18 | Me | Et | Ph | 8-F | A | 69 | 158-161 | maleate | MeOH/ether |

*over two steps

TABLE A

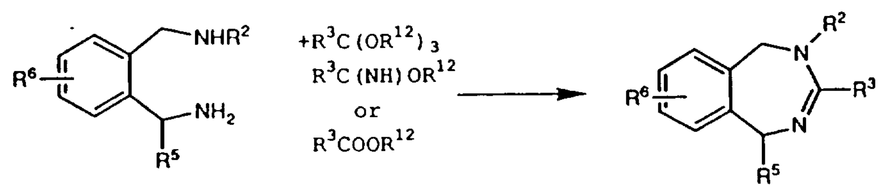


| Example No. | $R^2$ | $R^3$ | $R^5$ | $R^6$ | Method | Yield % | Melting Range | Salt | Recrystallized From |
|---|---|---|---|---|---|---|---|---|---|
| 19 | Me | Et | 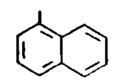 | H | A | 48 | 145-148 | --- | EtOH |
| 20 | Me | iPr | Ph | H | B | 60 | 79-80 | --- | $Et_3N$/pentane |
| 21 | Me | Bzl | Ph | H | B | 79 | 236-237 | HCl | MeCN |
| 22 | iPr | $PhCH_2CH_2$ | Ph | H | E | 73 | 150-153 | maleate | acetone/ether |
| 23 | iPr | Me | Ph | H | E | 56<br>71 | 165-166<br>130-132 | maleate<br>free base | acetone/ether<br>EtOAc/hexane |
| 24 | iPr | nBu | Ph | H | E | 78 | 108-109 | maleate<br>hemihydrate | acetone/ether<br>or<br>MeCN/tBuOMe |
| 25 | Me | $PhCH_2CH_2$ | Ph | H | D<br>F | 50<br>62 | 118-123<br>132-133 | HCl<br>HCl | MeCN/ether<br>MeCN |

## TABLE A

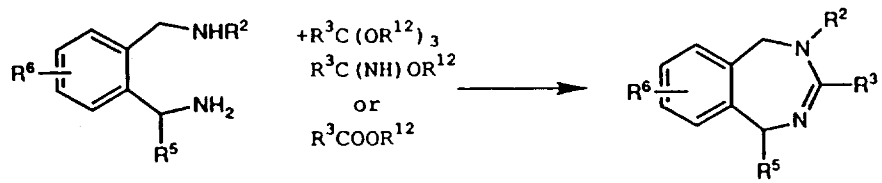


| Example No. | $R^2$ | $R^3$ | $R^5$ | $R^6$ | Method | Yield % | Melting Range | Salt | Recrystallized From |
|---|---|---|---|---|---|---|---|---|---|
| 26 | Ph | Me | Me | H | A | 35 | 89-92 | HCl | EtOH/tBuOMe |
| 27 | Ph | Et | Me | H | A | 2 | 207-208 | HCl | MeCN/tBuOMe |
| 28 | Me | Me | 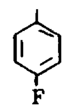 | H | A | 53 | 185-187 | maleate | EtOH/tBuOMe |
| 29 | Me | Ph | 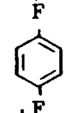 | H | A | 48 | 146-148 | free base | MeCN |
| 30 | Me | Me | 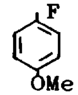 | H | A | 26 | 235-237 | HCl | MeCN/tBuOMe |
| 31 | 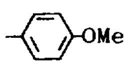 | Me | Me | H | A | 60 | 241-243 | HCl | MeCN/tBuOMe |
| 32 | Me | Et | Ph | 8,9 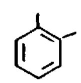 | A | 40 | 149-151 | free base | florisil EtOAc/IPA Chromatog. |

TABLE A

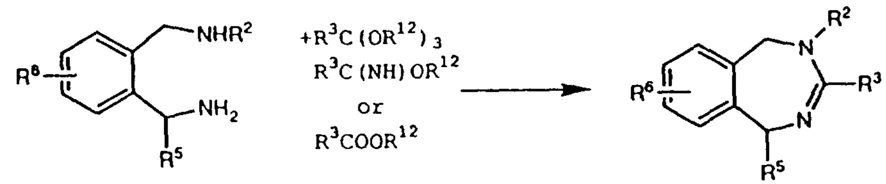


| Example No. | $R^2$ | $R^3$ | $R^5$ | $R^6$ | Method | Yield % | Melting Range | Salt | Recrystallized From |
|---|---|---|---|---|---|---|---|---|---|
| 33 | Me | Et | Ph | 9-Me | A | | 49-53 | free base $\cdot\frac{1}{4}H_2O$ | florisil EtOAc/IPA Chromatog. |
| 34 | Me | $-(CH_2)_7CH_3$ | Ph | H | D | 28 | 159-161 | HCl | MeCN/ether |
| 35 | Me | $CH_2CH_2$ 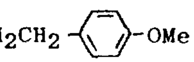 | Ph | H | D | 53 | 131-133 | HCl | MeOH/ether |
| 37 | Me | Et | 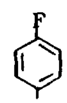 | H | A | 58 | 133-135 | maleate | MeCN/tBuOMe |
| 38 | Me | $-CH_2O$ 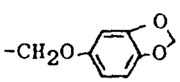 | Ph | H | D | 33 | 196-198 | HCl | EtOH/ether |
| 39 | Me | $-CH_2O$ 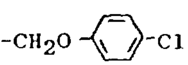 | Ph | H | D | 14 | 166-168 | HCl $\frac{1}{2}H_2O$ | EtOH/ether |
| 40 | Me | $-CH_2O$ 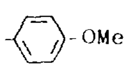 | Ph | H | D | 17 | 225-226 | HCl | EtOH/ether |

TABLE A

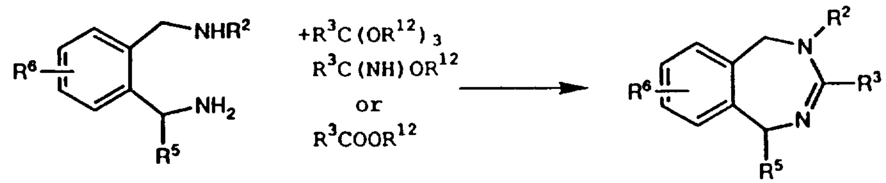


| Example No. | $R^2$ | $R^3$ | $R^5$ | $R^6$ | Method | Yield % | Melting Range | Salt | Recrystallized From |
|---|---|---|---|---|---|---|---|---|---|
| 41 | Me | Et | 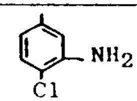 | H | G | 23 | 249-251 | fumarate | EtOH/ether |
| 42 | Me | $-CH_2OPh$ | Ph | H | G | 74 | 153-155 | HCl | EtOH/ether |
| 43 | Me | $-(CH_2)_3Ph$ | Ph | H | E | 79 | 101-106 | fumarate | EtOH/ether |
| 44 | $CH_2CH_2OMe$ | Ph | Ph | H | A | 70 | 170-172 | maleate | |
| 45 | $CH_2CH_2NEt_2$ | Me | Ph | H | A | 24 | 154-156 | 2 maleate | MeCN |
| 46 | $CH_2CH_2NEt_2$ | H | Ph | H | A | 18 | 145-147 | 2 maleate | MeCN acetone tBuOMe |
| 47 | $CH_2CH_2$- 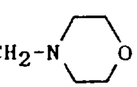 | H | Ph | H | A | 27 | 175-177 | 2 maleate | EtOH |

TABLE A

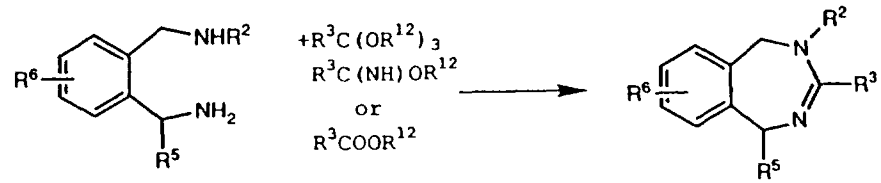


| Example No. | $R^2$ | $R^3$ | $R^5$ | $R^6$ | Method | Yield % | Melting Range | Salt | Recrystallized From |
|---|---|---|---|---|---|---|---|---|---|
| 48 | $CH_2CH_2N$ 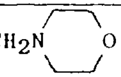 | Me | Ph | H | A | 15 | 167-169 | 2 maleate | MeCN/tBuOMe |
| 49 | Me | H | Ph | H | A | 7 | 149-151 | 2 maleate | EtOH |
| 50 | $CH_2CH_2NEt_2$ | Et | Ph | H | A | 10 | 224-227 | 2 HCl | MeCN/tBuOMe |
| 51 | Me | 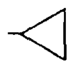 | Ph | H | G | 29 | 113-115 | free base | iPrOH |
| 52 | Me | $CH_2CH_2Ph$ | Bzl | H | D | 47 | 139-141 | fumarate | EtOH |
| 53 | $CH_2$ 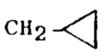 | $CH_2CH_2Ph$ | Ph | H | E | 45 | 173-187 | HCl | acetone/ether |
| 54 | $CH_2$ 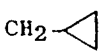 | Et | Ph | H | E | 87 | 189-191 | HCl | acetone/ether |
| 55 | $CH_2$ 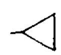 | Me | Ph | H | E | 70 | 226-234 | HCl | acetone/ether |

TABLE A

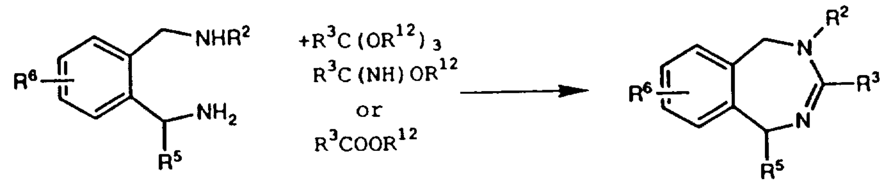


| Example No. | $R^2$ | $R^3$ | $R^5$ | $R^6$ | Method | Yield % | Melting Range | Salt | Recrystallized From |
|---|---|---|---|---|---|---|---|---|---|
| 56 | $CH_2CH_2Ph$ | Et | Ph | H | E | 90 | 225-228 | HCl | acetone |
| 57 | $CH_2CH_2Ph$ | Me | Ph | H | E | 95 | 233-235 | HCl | acetone/ether |
| 58 | Me | $CH_2CH_2$- 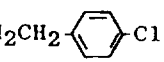 | Ph | H | D | 48 | 185-187 | fumarate | iPrOH/ether |
| 59 | Me | $CH_2SO_2Ph$ | Ph | H | F | 47 | 255-257 | HCl | MeOH/ether |
| 60 | Me | $CH_2CH_2Ph$ | 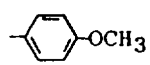 | H | D | 35 | 204-206 | HCl | MeOH/ether |
| 61 | Me | CH=CHPh trans | Ph | H | F | 67 | 245-250 | HCl | $CHCl_3$/ether |
| 62 | Me | Ph | 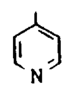 | H | C | 30 | 134(d) | free base | iPrOH/hexane |
| 63 | Me | $CH_2CH_2Ph$ | 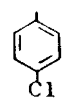 | H | G | 31 | 202-203 | HCl | MeCN/EtOH |

TABLE A

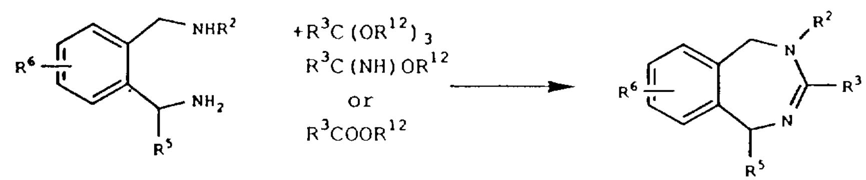


| Example No. | $R^2$ | $R^3$ | $R^5$ | $R^6$ | Method | Yield % | Melting Range | Salt | Recrystallized From |
|---|---|---|---|---|---|---|---|---|---|
| 210 | Bzl | Me | Ph | H | E | 74 | 126-127<br>148-149 | free base<br>HCl | EtOAC/hexane<br>MeOH/MeOtBu |
| 211 | Me | CH=CH 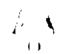 | Ph | H | F | 28 | 237-239 | fumarate | MeOH/EtOH/ether |
| 212 | Me | CH=CH 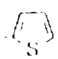 | Ph | H | F | 22 | 234-236 | fumarate | MeOH/EtOH/ether |
| 213 | Me | $CH_2CH_2$ 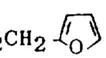 | Ph | H | F | 67 | 184-185 | fumarate | EtOH/ether |
| 214 | Me | $CH_2CH_2$ 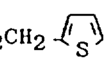 | Ph | H | F | 48 | 176-177 | fumarate | EtOH/ether |
| 215 | Me | CH=CH 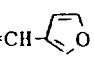 | Ph | H | F | 22 | 212-213 | fumarate | EtOH/ether |
| 216 | Me | CH=CH 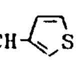 | Ph | H | F | 42 | 246-247 | fumarate | MeOH/EtOH/ether |
| 217 | Me | $CH_2CH_2Ph$ | $(CH_2)_3CH_3$ | H | D | 20 | 152-154 | HCl | MeOH/ether |

TABLE A

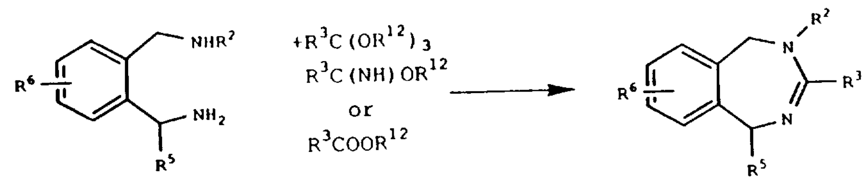


| Example No. | $R^2$ | $R^3$ | $R^5$ | $R^6$ | Method | Yield % | Melting Range | Salt | Recrystallized From |
|---|---|---|---|---|---|---|---|---|---|
| 218 | Me | $CH_2CH_2$- 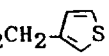 | Ph | H | F | 64 | 179-180 | fumarate | MeOH/EtOH/ether |
| 219 | Me | $CH_2CH_2$- 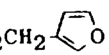 | Ph | H | F | 32 | 169-171 | fumarate | MeOH/EtOH/ether |
| 220 | Me | CH=CH- 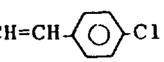 | Ph | H | F | 32 | 219-222 | fumarate | MeOH/EtOH/ether |
| 221 | Bzl | $CH_2CH_2Ph$ | Ph | H | F | 13 | 137-138 | fumarate | EtOH/EtOAC |
| 222 | Me | $CH_2CH_2Ph$ | 1-naphthyl | H | D | 6 | 268-270 | HCl·¼$H_2O$ | MeOH/THF/ether |
| 223 | Bzl | CH=CH-Ph | Ph | H | F | 39 | 197-199 | HCl | THF/ether |
| 224 | Me | CH=CH- 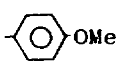 | Ph | H | F | 28 | 207-208 | fumarate | EtOH |
| 225 | Me | CH=CH- 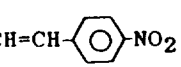 | Ph | H | F | 30 | 230-232 | fumarate ·$H_2O$ | MeOH/EtOH/ether |

TABLE A

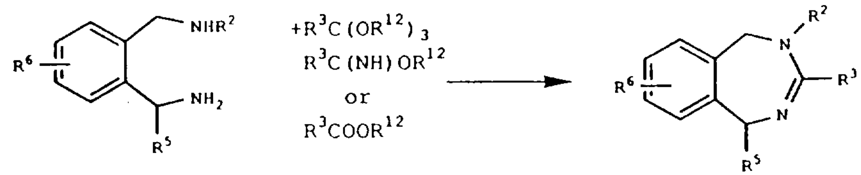


| Example No. | $R^2$ | $R^3$ | $R^5$ | $R^6$ | Method | Yield % | Melting Range | Salt | Recrystallized From |
|---|---|---|---|---|---|---|---|---|---|
| 226 | Me | $CH_3$ C=C($CH_3$)Ph* | Ph | H | F | 14 | 207-209 | fumarate | MeOH/ether |
| 227 | Me | $CH_2CH_2Ph$ | cyclohexyl | H | F | 53 | 215-216 | fumarate | MeOH/ether |
| 228 | Me | $CH_2CH_2$-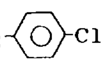-Cl | 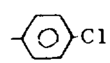-Cl | H | F | 39 | 155-157 | fumarate | MeOH/ether |
| 229 | Me | $CH_2CH_2$-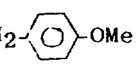-OMe | 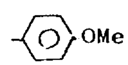-OMe | H | F | 66 | 154-156 | fumarate | MeOH/EtOH/ether |
| 230 | Me | CH=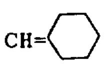 | Ph | H | F | 34 | 217-218 | fumarate | MeOH/ether |
| 231 | Me | $C\equiv C$-Ph | Ph | H | F | 5 | 223-225 | HCl·$H_2O$ ·½EtOH | EtOH/ether |
| 232 | Me | CH=CH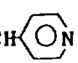 | Ph | H | F | 18 | 243-245 | 2HCl· 5/4 $H_2O$ | MeOH/THF/ether |

*mixture of isomers

TABLE A

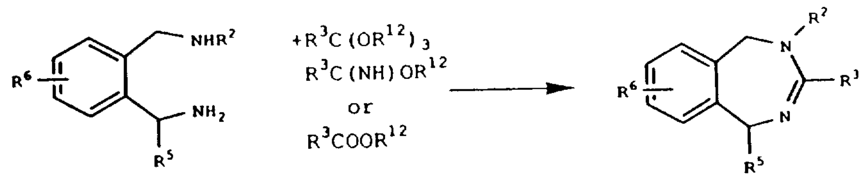


| Example No. | $R^2$ | $R^3$ | $R^5$ | $R^6$ | Method | Yield % | Melting Range | Salt | Recrystallized From |
|---|---|---|---|---|---|---|---|---|---|
| 233 | Me | 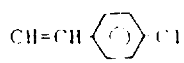 | 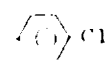 | H | F | 54 | 233-234 | fumarate | MeOH/ether |
| 234 | Me | CH=CH-Ph | 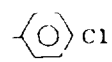 | H | F | 64 | 229-231 | fumarate | MeOH/ether |
| 235 | Ph | $CH_2CH_2PH$ | Me | H | F | 30 | 207-209 | HCl · ¼$H_2O$ | THF/ether |
| 236 | Me | 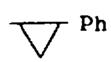 | Ph | H | F | 86 | 205-207 | fumarate ·4/5 $H_2O$ | MeOH/ether |
| 237 | Me | 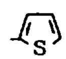 | Ph | H | F | 47 | 137-139 | free base | MeOH |
| 238 | Me | 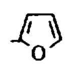 | Ph | H | F | 37 | 110-112 | free base | MeOtBu/hexane |
| 239 | Me | 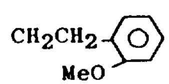 | 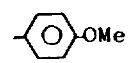 | H | F | 98 | 196-198 | fumarate | MeOH/MeCN/ether |

TABLE A

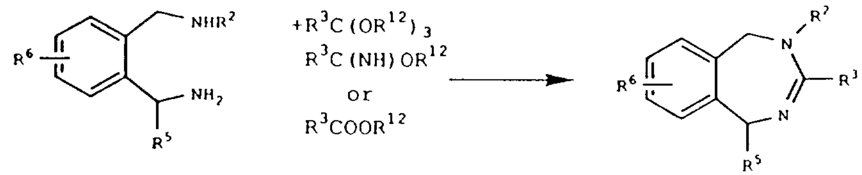


| Example No. | $R^2$ | $R^3$ | $R^5$ | $R^6$ | Method | Yield % | Melting Range | Salt | Recrystallized From |
|---|---|---|---|---|---|---|---|---|---|
| 240 | Me | 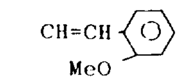 | 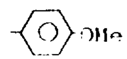 | H | F | 16 | 204-205 | fumarate | MeOH/ether |
| 241 | Me | 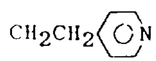 | Ph | H | F | 45 | 241-243 | 2 HCl | MeOH/MeCN/THF |
| 242 | Me | 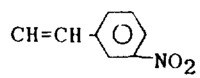 | Ph | H | F | 54 | 209-210 | fumarate | EtOH/ether |
| 243 | Me | 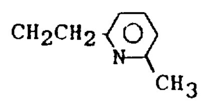 | Ph | H | F | 52 | 235-237 | 2HCl·½H$_2$O | MeOH/ether |
| 244 | Me | 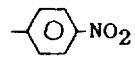 | Ph | H | F | 34 | 145-150 | fumarate | MeOH/ether |
| 245 | Me | 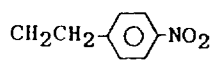 | Ph | H | E | 91 | 123-125 | HCl·½EtOH | EtOH/ether |
| 246 | Me | 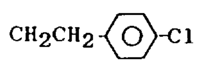 | 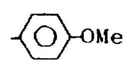 | H | F | 71 | 191-193 | fumarate | MeOH/MeCN/ether |
| 247 | Me | 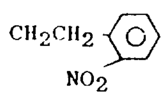 | Ph | H | E | 66 | 234-236 | HCl | MeOH/ether |

TABLE A

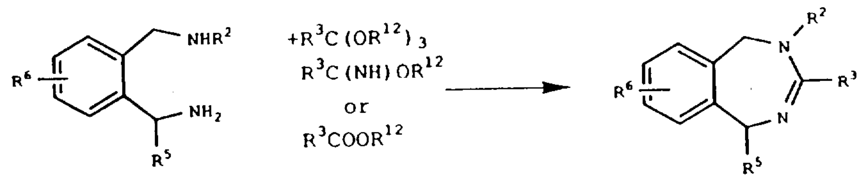


| Example No. | $R^2$ | $R^3$ | $R^5$ | $R^6$ | Method | Yield % | Melting Range | Salt | Recrystallized From |
|---|---|---|---|---|---|---|---|---|---|
| 248 | Me | $CH_2CH_2Ph$ | Ph | 8-$NO_2$ | G | 88 | 241-242 | HCl | MeOH/ether |
| 269 | Me | $CH_2CH_2$-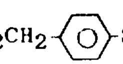-$SO_2NEt_2$ | Ph | H | F | 54 | 171-173 | HCl | EtOH/ether |
| 276 | $CH_2CH_2$-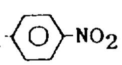-$NO_2$ | Et | Ph | H | E | 64 | 222-224 | HCl | MeCN/THF |

## TABLE J

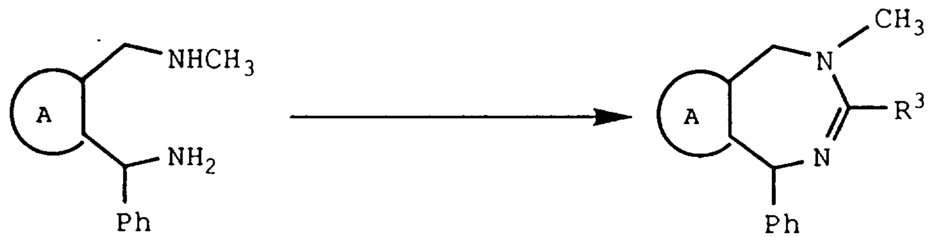

| Example No. | A | $R^3$ | Method | Yield % | Melting Range | Salt | Recrystallized from |
|---|---|---|---|---|---|---|---|
| 200 | 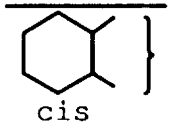 cis | $CH_2CH_2Ph$ | E | 32 | 190-192 | fumarate | THF/MeCN |
| 201 | 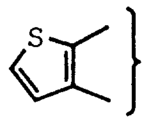 | $CH_2CH_2Ph$ | E | 67 | 180-181 | fumarate | EtOH/ether |
| 202 | 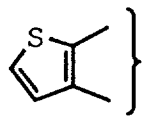 | $CH_2CH_3$ | E | 61 | 191-192 | fumarate | EtOH/ether |
| 203 | 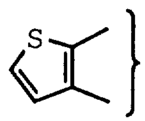 | CH=CH 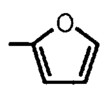 | F | 20 | 184-185 | fumarate | MeOH/MeCN (after chromatography on $S_iO_2$ with 4:49:3 hexane/tBuOMe/IPA) |
| 204 | 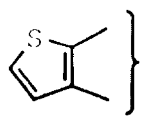 | $CH_2SO_2Ph$ | F | 55 | 132-135 | fumarate | EtOH/ether |

General Method A

The appropriate diamine and five to seven equivalents of the corresponding triethylorthoester were stirred at room

temperature while 0.4-0.5 mL of acetic acid per mmol of diamine were added in one portion. The mixture was stirred at reflux for 2-12 hours or stirred at room temperature for 2-72 hours. The reaction was diluted with ethyl acetate, washed with 2N sodium hydroxide and extracted into three portions of 2N HCl. The HCl extracts were combined, washed twice with ether, made basic with excess 35% sodium hydroxide and extracted into three portions of ether. The ether extracts were combined, dried over magnesium sulfate and the solvent removed in vacuo. The free base or the salt was recrystallized as shown in Table A.

General Method B

The diamine was added to two equivalents of potassium acetate or a catalytic amount of potassium acetate in 0.8-1.2 mL of acetic acid per mmol of diamine. The mixture was stirred at room temperature and from two to five equivalents of the appropriate triethylorthoester were added. The reaction was stirred at room temperature for 18-72 hours and stripped in vacuo. The product was worked up as described for General Method A.

General Method C

The dihydrochloride of the diamine was dissolved in 1-3 mL of acetic acid per mmol of diamine and about 2.0 to 2.5 equivalents of sodium acetate were added. The mixture was stirred for about ten minutes at room temperature, and three to five equivalents of the appropriate triethylorthoester were added. The mixture was stirred at room temperature for 2-48 hours and stripped in vacuo. The reaction was worked up as described in General Method A.

General Method D

The diamine dihydrochloride and two to three equivalents of the appropriate methoxyimine hydrochloride were dissolved in 2-6 mL of methanol per mmol of diamine. The mixture was stirred, and two equivalents of sodium acetate were added. After 2-18 hours the solvent was removed in vacuo and the product worked up as described in General Method E.

General Method E

The diamine dihydrochloride, 1.3-3.0 equivalents of the appropriate trimethyl- or triethylorthoester and 1.0-1.8 equivalents of sodium acetate were combined in about 3 to 6 mL of isopropyl acetate per mmol of of the diamine. The mixture was refluxed for 3-18 hours. The reaction was cooled, washed with two portions of 2N sodium hydroxide and dried over sodium sulfate. The solvent was removed in vacuo and either the salt or the free base was purified from the residue as shown in Table A.

General Method F

To the diamine dihydrochloride, slurried in about 3 mL of toluene per mmol of diamine, was added dropwise at 0° under nitrogen 2.1 equivalents of 2M trimethylaluminum in toluene. The reaction was allowed to come to room temperature and stirred for 2 hours, then 1.25 to 1.50 equivalents of the appropriate methyl or ethyl ester were added. The reaction was refluxed 2 hours, cooled and quenched by the sequential addition of ice, methanol, dichloromethane and 2N NaOH. The aluminum salts were filtered off, the layers separated, washed with more dichloromethane, dried over sodium sulfate, stripped and crystallized as shown in Table A. Occasionally flash chromatography on silica gel with MeOtBu, optionally containing up to 2% isopropylamine, was necessary before crystallization.

General Method G

The free base of the diamine, three equivalents of acetic acid and three equivalents of either the appropriate triethylorthoester or the hydrochloride of the appropriate ethoxyimine in 2-4 mL of methanol per mmol of diamine were stirred at room temperature for 18 hours. The solvent was removed in vacuo and the product treated as described in General Method A.

TABLE B

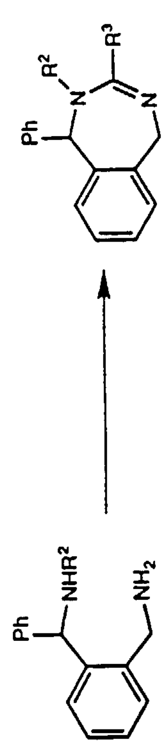

| Example No. | $R^2$ | $R^3$ | Method | Yield % | Melting Range | Salt | Recrystallized From |
|---|---|---|---|---|---|---|---|
| 64 | Me | Et | C | 92 | 205-207 | HCl | MeOH/ether |
| 65 | Me | Me | C | 27 | 261-264 | HCl | MeOH/ether |
| 66 | Me | H | C | 32 | 244-245 | HCl | MeOH/ether |
| 67 | Me | nPr | C | 62 | 223-226 | HCl | MeOH/ether |
| 68 | Bzl | Et | C | 49 | 209-210 | HCl | EtOAc/HCl in ether |
| 69 | Bzl | H | H | 52 | 149-151 | $H_2O$ | |
| 70 | Bzl | nPr | I | 20 | 211-212 | HCl | EtOAc/ether |
| 249 | Me | $CH_2CH_2Ph$ | E | 78 | amorphous | HCl·3/5 $H_2O$ | ether |

General Method H

The diamine dihydrochloride, 2.2 equivalents of sodium acetate and 1.5 equivalents of triethylorthoester were combined in 1.2 mL of isopropyl acetate per mmol of diamine and refluxed for four days. The solvent was removed in vacuo, the residue taken up in dichloromethane, the dichloromethane was washed two times with 2N sodium hydroxide and dried over magnesium sulfate. The solvent was removed in vacuo and the product chromatographed on silica gel eluting with 49:49:2 ethyl acetate/dichloromethane/ diethylamine. The hydrochloride of the purified free base was formed by dissolving the free base in ethyl acetate and adding HCl in ether.

General Method I

The diamine dihydrochloride, 2.1 equivalents of sodium acetate, 3 equivalents of trimethylorthoester, and 5 equivalents of acetic acid were stirred together for seven days at room temperature. The workup was the same as that described for General Method H.

TABLE C

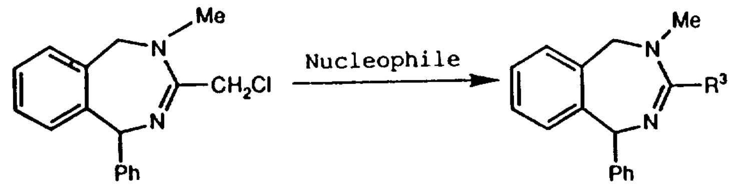


| Example No. | Nucleophile | $R^3$ | Method | Yield* % | Melting Range | Salt | Recrystallized From |
|---|---|---|---|---|---|---|---|
| 71 | EtSH | $-CH_2SEt$ | J | 35 | 267-269 | HCl | EtOH/ether |
| 59 | $PhSO_2Na$ | 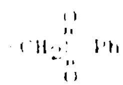 | K | 31 | 246-248 | HCl | MeOH/ether |
| 72 | $Me_2NH$ | $-CH_2NMe_2$ | K | 41 | 184-186 | fumarate | EtOH/ether |
| 73 | PhSH | $-CH_2SPh$ | J | 12 | 196-197 | fumarate | MeOH/ether |
| 74 | 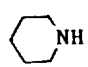 | 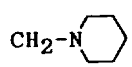 | K | 43 | 194-196 | fumarate | EtOH/ether |
| 75 | $BzlNHCH_3$ | 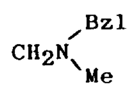 | K | 23 | 102-104 | fumarate | EtOH/ether |

*yield calculated over two steps from the diamine.

TABLE C

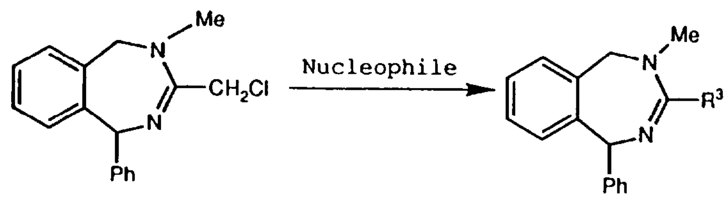


| Example No. | Nucleophile | $R^3$ | Method | Yield* % | Melting Range | Salt | Recrystallized From |
|---|---|---|---|---|---|---|---|
| 76 | 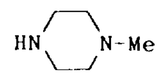 | 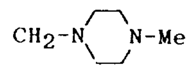 | K | 52 | 233-235 | 2 fumarate | MeOH/ether |
| 77 | 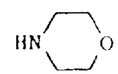 | 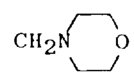 | K | 28 | 206-208 | fumarate | MeOH/ether |
| 78 | $Et_2NH$ | $CH_2NEt_2$ | K | 24 | 133-135 | 1.5 fumarate 0.25 EtOH | EtOH/ether |
| 79 | 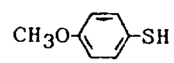 | 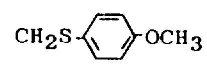 | J | 16 | 208-210 | HCl | MeCN/ether |
| 80 | 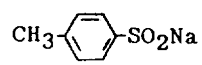 | 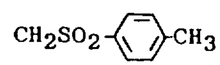 | K | 30 | 247-249 | HCl | EtOH/MeCN |
| 81 | 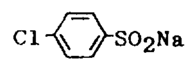 | 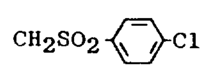 | K | 10 | 214-217 | HCl | MeOH/MeCN |

General Method J

The 3-chloromethylbenzodiazepine was dissolved in three mL of acetonitrile per mmol of benzodiazepine and added to 1.0-1.7 equivalents of the thiol plus 2.3 equivalents of milled potassium carbonate in three mL of acetonitrile per mmol of benzodiazepine. The reaction was stirred at room temperature for 18 hours and filtered. The acetonitrile

was removed in vacuo and the product worked up as described in General Method A.

General Method K

The 3-chloromethylbenzodiazepine and three equivalents of the appropriate amine or sodium sulfinate were combined in 1-5 mL of solvent per mmol of benzodiazepine and refluxed for 3-5 hours. The reactions with amines were run in chloroform; the reactions with sulfinate were run in methanol. The product was worked up as described in General Method A.

TABLE D

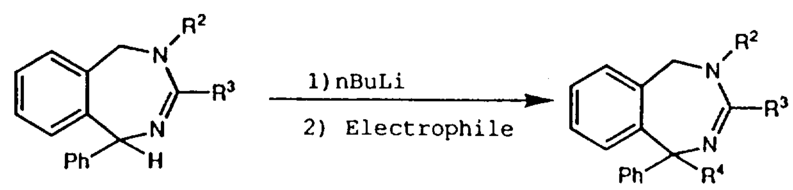


| Example No. | $R^2$ | $R^3$ | $R^4$ | Electrophile | Method | Yield % | Melting Range | Salt | Recrystallized From |
|---|---|---|---|---|---|---|---|---|---|
| 82 | Me | Et | $-CH_2COOEt$ | $BrCH_2COOEt$ | L | 22 | 166-167 | fumarate | EtOH/ether |
| 83 | Me | Et | $-CH(OH)CH_2CH_3$ | $CH_3CH_2CHO$ | M | 21 | 155(d) | fumarate | EtOH/ether |
| 84 | Me | Et | -COOEt | ClCOOEt | L | 57 | 162(d) | fumarate | EtOH |
| 85 | Me | Et | $-C(=O)Me$ | $CH_3COCl$ | L | 22 | 184-185 | fumarate | EtOH |
| 86 | Me | Et | $-CH_2CH=CH_2$ | $BrCH_2CH=CH_2$ | L | 25 | 180-187 | fumarate | EtOH/ether |
| 87 | Me | Et | iPr | iPrI | L | 25 | 208-210 | fumarate | iPrOH |
| 88 | Me | Et | Et | EtI | L | 45 | 234-235 | fumarate | EtOH |
| 89 | $CH_2$ 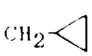 | Me | Me | MeI | L | 56 | 107-108 | free base | ether/hexane |
| 90 | Me | Ph | Et | EtI | L | 80 | 126-127 | free base | tBuOMe/hexane |

TABLE D

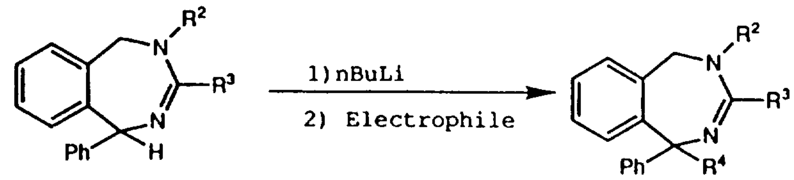


| Example No. | $R^2$ | $R^3$ | $R^4$ | Electrophile | Method | Yield % | Melting Range | Salt | Recrystallized From |
|---|---|---|---|---|---|---|---|---|---|
| 91 | Me | Et | n-Pr | nPrI | L | 50 | 199-202 | fumarate | EtOH/ether |
| 92 | iPr | Me | Me | MeI | L | 93 | 150-151<br>103-104 | maleate<br>free base | acetone/ether<br>hexane |
| 93 | iPr | Me | Et | EtI | L | 79 | 158-159<br>unrecorded | maleate<br>free base | acetone/ether<br>hexane |
| 94 | Me | Ph | Me | MeI | L | 40 | 130-132 | free base | hexane/$CH_2Cl_2$ |
| 95 | Me | Et | Me | MeI | L | 66 | 244-246 | fumarate | EtOH |
| 96 | Me | Me | Me | MeI | L | 61 | 228-230<br>115-116 | fumarate<br>free base | trit. iPrOH<br>hexane |
| 97 | Me | Me | $CH_2OMe$ | $ClCH_2OMe$ | L | 17 | 155-156 | free base | EtOAc/hexane |

TABLE E

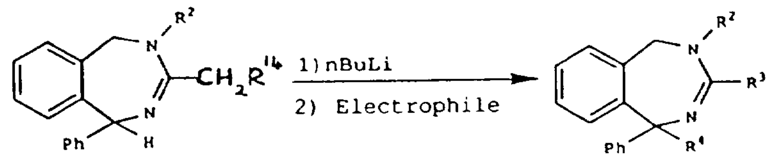


| Example No. | $R^2$ | $R^3$ | $R^4$ | Electrophile | $R^{14}$ | Method | Yield % | Melting Range | Salt | Recrystallized From |
|---|---|---|---|---|---|---|---|---|---|---|
| 98 | Me | $-CH_2Cl$ | Me | $C_2Cl_6$ | H | L | 18 | 118-120 | free base | $CH_2Cl_2$/hex |
| 99 | Me | $CH_2NMe_2$ | Me | $C_2Cl_6/Me_2NH$ | H | N | 51* | 89-90 | free base | Chromatog 9:1 hex/$Et_2NH$ |
| 100 | Me | $CH_2-N$ 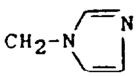 | Me | $C_2Cl_6/HN$ 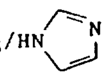 | H | N | 17* | 144-145 | free base | $CH_2Cl_2$/hex |
| 101 | Me | $-CH_2COOEt$ | Me | ClCOOEt | H | O | 12* | 100-102 | free base | $CH_2Cl_2$/hex |
| 102 | Me | $-CH(COOEt)_2$ | Me | ClCOOEt | H | L | 17* | 146-147 | free base | $CH_2Cl_2$/hex |
| 103 | Me | $-CH_2CH_2-$ 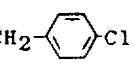 -Cl | Me | $ClCH_2-$ 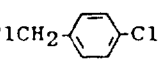 -Cl | H | L | 48* | 149-151 | maleate hemihydrate | acetone |
| 103A | | | | | | L | 78 | 143-147 | HCl (+)isomer | $H_2O$ |
| 103B | | | | | | L | 66 | 143-146 | HCL (-)isomer | $H_2O$ |

*Over two steps

TABLE E

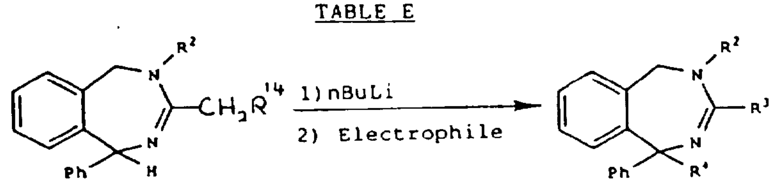


| Example No. | $R^2$ | $R^3$ | $R^4$ | Electrophile | $R^{14}$ | Method | Yield % | Melting Range | Salt | Recrystallized From |
|---|---|---|---|---|---|---|---|---|---|---|
| 104 | iPr | $-CH_2CH_2Ph$ | Me | BzlBr | H | L | 86 | 173-175 | maleate | acetone/ether |
| 105 | $CH_2$ 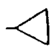 | $CH_2CH_2$- 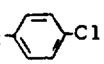 -Cl | Me | $ClCH_2$- 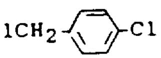 -Cl | H | L | 70 | 189-191 | HCl | MeCN/acetone/ ether |
| 106 | iPr | $CH_2CH_2CH_2Ph$ | Et | BzlBr | H | L | 88 | 160-162 | maleate | acetone/ether |
| 107 | Me | iPr | Me | MeI | Me | P | 40 | 223-225 | fumarate | EtOH |
| 108 | Me | $CH_2NEt_2$ | Me | $C_2Cl_6/Et_2NH$ | H | N | 65 | 200-206 | 2HCl | MeCN/ether |
| 109 | Me | $CH_2N$ 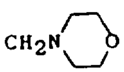 | Me | $C_2Cl_6/HN$ 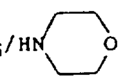 | H | N | 15 | 137-140 | $2H_2SO_4$ | MeOH/acetone |
| 110 | Me | $-CH_2COPh$ | Me | PhCOOMe | H | L | 18 | 160-161 | free base | EtOAc/hex |
| 111• | Me | $-CH(CH_3)CH_2Ph$ | Me | BzlBr | Me | L | 6 | 168-169 | maleate | MeCN/ether |
| 112• | Me | $-CH(CH_3)CH_2Ph$ | Me | BzlBr | Me | L | 30 | 177-180 | maleate | MeCN |

• diastereomeric pair

TABLE E

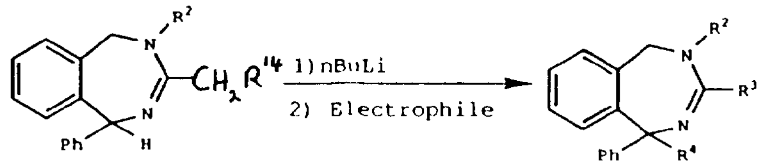


| Example No. | $R^2$ | $R^3$ | $R^4$ | Electrophile | R14 | Method | Yield % | Melting Range | Salt | Recrystallized From |
|---|---|---|---|---|---|---|---|---|---|---|
| 113 | Me | $CH_2CH(NH_2)Ph$ | Me | PhCH NTMS | H | L | 80 | 222-256 | 2HCl | MeOH/ether |
| 114 | Me | $-CH_2CH(OH)-$ 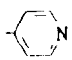 | Me | 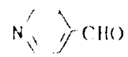 CHO | H | M | 45* | 189-190 | fumarate | EtOH/ether |
| 115 | Me | $CH_2CH(OH)-Ph$ | Me | PhCHO | H | M | 44* | 169-171 | maleate | EtOH/ether |
| 116 | Me | $CH_2CH_2$ 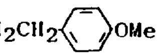 OMe | Me | $ClCH_2$ 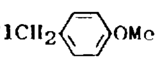 OMe | H | L | 84* | 171-172 | maleate | acetone/ether |
| 117 | Me | $CH_2SEt$ | Me | EtSSEt | H | L | 69* | 235-236 | HCl | $Al_2O_3$ Chromatog then ether |
| 118 | Me | $CH_2SPh$ | Me | PhSSPh | H | L | 27* | 240-250 | HCl | $SiO_2$ EtOAc/MeOH/IPA then ether |

*over two steps

TABLE E

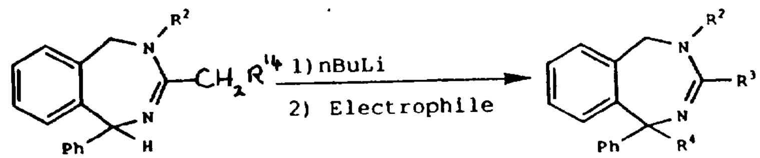


| Example No. | $R^2$ | $R^3$ | $R^4$ | Electrophile | $R^{14}$ | Method | Yield % | Melting Range | Salt | Recrystallized From |
|---|---|---|---|---|---|---|---|---|---|---|
| 119 | Me | $CH_2CH_2Ph$ | Me | BzlBr | H | L | 82 | 168-169 | maleate | MeCN/ether |
| 120 | Me | 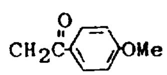 | Me | 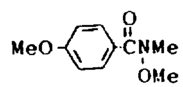 | H | Q | 50 | 144-146 | maleate | acetone/ether |

General Method L

The benzodiazepine in 3-7 mL of THF per mmol of benzodiazepine was stirred at -78°C to -42°C while 1.1 equivalent of N-butyllithium was added under nitrogen. The solution was stirred for one hour at -78°C, 1.1-1.3 equivalents of the appropriate electrophile were added, and the reaction was allowed to come to room temperature. The reaction was poured into 1N HCl, washed with ether, made basic, and extracted into ether. The combined ether layers were dried over potassium carbonate, filtered and the solvent removed in vacuo. The free base was recrystallized or a salt was prepared as shown in Table D.

Example 265

1-(4-Chlorophenyl)-3-[2-(4-chlorophenyl)ethyl]-4,5-dihydro-1,4-dimethyl-1H-2,4-benzodiazepine

(Formula Ia: $R^1$, R6 = H; $R^{2a}$, $R^{4b}$ = Me;
$R^{3a}$ = $CH_2CH_2$-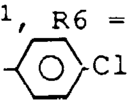-Cl; $R^{5c}$ = -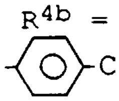-Cl

According to General Method L, 14.5 g of 1-(4-chlorophenyl)-3-[2-(4-chlorophenyl)ethyl]-4,5-dihydro-1,4-dimethyl-1H-2,4-benzodiazepine as its fumarate salt was prepared from 17.3 g of the compound of Example 228 and 7.6 g of methyl iodide. The salt was recrystallized from EtOH/ether, mp 173-175.

General Method M

The procedure described under General Method L was followed except that two equivalents of N-butyllithium and two equivalents of aldehyde were used.

General Method N

The benzodiazepine in 3-7 mL of THF per mmol of benzodiazepine was stirred at -78°C while 1.1 equivalents of butyllithium was added under nitrogen. The solution was stirred for one hour at -78°C, 1.1-1.3 equivalents of hexachloroethane were added, and the reaction was stirred for one-half hour at -78°C. The reaction was poured into 1N HCl, washed three times with ether, made basic with 35% sodium hydroxide, extracted into ether, dried over potassium carbonate, filtered and stripped. The resulting brown oil was filtered through silica with ethyl acetate, stripped and taken directly to the next step. The 3-chloromethyl benzodiazepine was either dissolved in chloroform and treated with 3-5 equivalents of the appropriate amine or it was dissolved directly in a large excess of the amine. The solution was refluxed from 1-20 hours. The solvent was removed and the product was crystallized as shown in Table E.

General Method O

The procedure was substantially similar to General Method L except that inverse addition of the lithium salt of the benzodiazepine was made to 1.5 equivalents of the chloroester.

General Method P

The procedure was substantially similar to General Method L except that lithium diisopropylamide, generated from butyllithium and diisopropylamine, was used as the base and the reaction was run at 0°.

General Method Q

The procedure was substantially similar to General Method L except that the reaction was quenched after stirring one hour at -55° by adding a slight excess of acetic acid in THF.

General Method R

The benzodiazepine-3-one was dissolved in 13-14 equivalents of phosphorus oxychloride and one-quarter equivalent of phosphorus pentoxide was added. The mixture was stirred at room temperature under nitrogen briefly, then heated at 90°C for 18 hours. The solution was stripped in vacuo, and the residue treated with 4-9 equivalents of the

appropriate amine and stirred for two hours at room temperature. The excess amine was stripped in vacuo, and the residue was crystallized as shown in Table F.

TABLE F

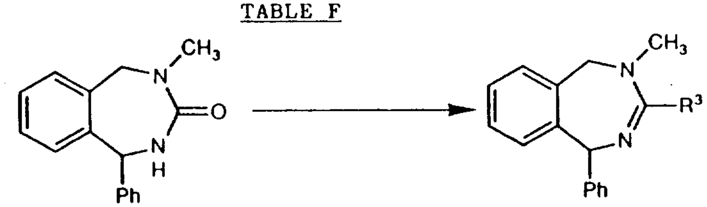

| Example No. | $R^3$ | Method | Yield % | Melting Range | Salt | Recrystallized From |
|---|---|---|---|---|---|---|
| 121 | 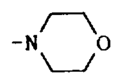 | R | 20 | 245-246 | HCl | $CH_2Cl_2$/hexane |
| 122 | $N(Me)_2$ | R | 33 | 124-125 | free base | not rex; from column 9:1 EtOAc $Et_2N$ |
| 123 | 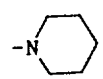 | R | 53 | 159-161 | 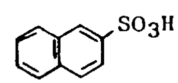 | 2-butanone |

TABLE G

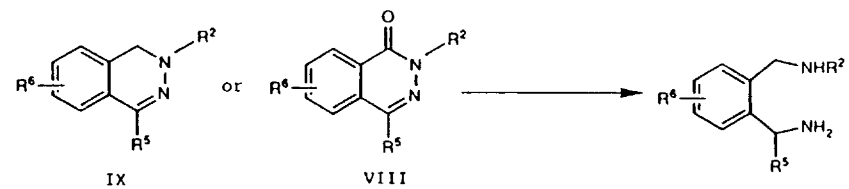

IX or VIII

| Example No. | Starting Material | $R^2$ | $R^5$ | $R^6$ | Method | Yield % | Melting Range | Salt | Recrystallized From |
|---|---|---|---|---|---|---|---|---|---|
| 124 | VIII | 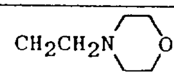 | Ph | H | S | 100 | oil | --- | --- |
| 125 | VIII | Ph | Me | H | T | 88 | 71-72 | free base | ether/hexane |
| 126A<br>126B | IX<br>VIII | Me | 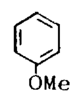 | H | S | 48<br>70 | 245-247 | 2HCl | MeOH/ether |
| 127 | VIII | 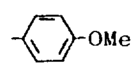 | Me | H | S | 100 | oil | | |
| 128 | VIII | Me | Ph | 7,8-diOMe | S | | oil | --- | --- |
| 129 | VIII | $CH_2CH_2OMe$ | Ph | H | S | 42 | 252-254 | 2 HCl | |
| 130 | VIII | Me | 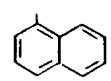 | H | T | 72 | 244-246 | $2HCl \cdot \frac{1}{2}H_2O$ | EtOH/ether |

TABLE G

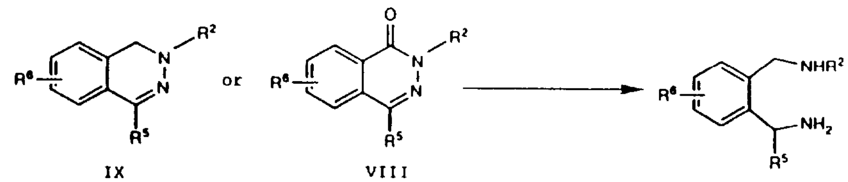

IX or VIII

| Example No. | Starting Material | $R^2$ | $R^5$ | $R^6$ | Method | Yield % | Melting Range | Salt | Recrystallized From |
|---|---|---|---|---|---|---|---|---|---|
| 131 | VIII | iPr | Ph | H | S | 94 | 264-266 | 2HCl | MeOH/ether |
| 132 | VIII | Me | Ph | H | S | 94 | 163-168 | HCl | EtOH/ether |
| 133 | VIII | Me | 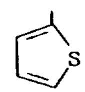 | H | T | 48 | 201-203 | 2HCl | MeOH/EtOH |
| 134 | VIII | Bzl | Ph | H | S<br>T | 70<br>83 | >260<br>275-276 | 2HCl<br>2HCl | MeOH/ether<br>MeOH/ether |
| 135 | VIII | Me | 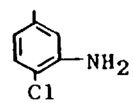 | H | T | 57 | --- | free base | --- |
| 136 | VIII | Me | 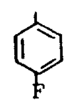 | H | S | 100 | --- | free base | --- |
| 137 | IX | Me | Ph | 6-F | S | 89 | oil | free base | --- |

TABLE G

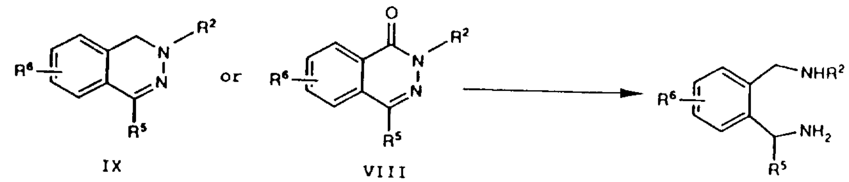

IX or VIII

| Example No. | Starting Material | $R^2$ | $R^5$ | $R^6$ | Method | Yield % | Melting Range | Salt | Recrystallized From |
|---|---|---|---|---|---|---|---|---|---|
| 138 | IX | Me | Ph | 7-F | S | 92 | oil | free base | --- |
| 139 | IX | Me | Ph | 8-F | S | 61 | oil | free base | --- |
| 140 | IX | Me | Ph | 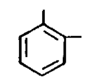 8,9 fused | T | 56 | oil | free base | --- |
| 141 | IX | Me | Ph | 9-Me | S | 29-61 | oil | free base | --- |
| 142 | VIII | $CH_2CH_2NEt_2$ | Ph | H | S | 89 | oil | free base | --- |
| 143 | VIII | Et | Ph | H | S | 91 | 241-243 | 2HCl·¼$H_2O$ | MeOH/THF |
| 144 | VIII | Me | 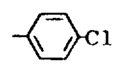 | H | T | 61 | 259-261 | 2HCl | EtOH/ether |
| 145 | VIII | $CH_2$ 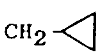 | Ph | H | T | 90 | amorphous | 2HCl | ether |
| 146 | VIII | Me | 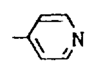 | H | S | 72 | | | |

TABLE G

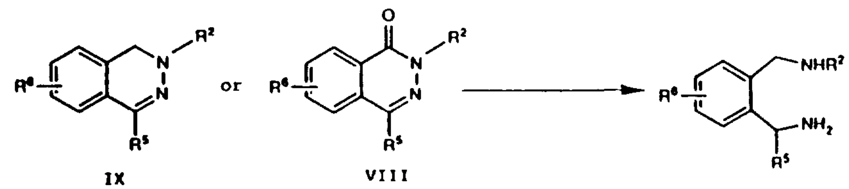

IX or VIII

| Example No. | Starting Material | $R^2$ | $R^5$ | $R^6$ | Method | Yield % | Melting Range | Salt | Recrystallized From |
|---|---|---|---|---|---|---|---|---|---|
| 147 | VIII | $CH_2CH_2Ph$ | Ph | H | T | 96 | 156-160 | 2HCl | MeOH/THF/ether |
| 148 | VIII | Me | Bzl | H | T | 87 | 267 | 2HCl | MeOH/ether |
| 207 | VIII | Me | cyclohexyl | H | S | 98 | 275-277 | 2HCl | MeOH/ether |
| 287 | VIII | Me | Ph | $NO_2$ | S | 24 | 80-81 | free base | MeOtBu/hexane |
| 277 | VIII | $CH_2CH_2$-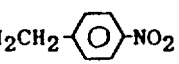-$NO_2$ | Ph | H | S | 82 | amorphous | 2HCl | MeOH/ether |

General Method S

The appropriate phthalazine or phthalazinone was treated with 4-8 equivalents of diborane in THF and the mixture was refluxed for 2-5 days under nitrogen. Usually the 4-8 equivalents of diborane were added in two or three portions over the course of the reaction. The reaction was allowed to cool to room temperature and excess aqueous or alcoholic hydrochloric acid was added carefully under nitrogen. The reaction was refluxed, the THF was removed in vacuo and the residue was made basic with 35% aqueous sodium hydroxide. The product was extracted into ethyl acetate, dried over sodium sulfate, concentrated in vacuo, and either purified as the hydrochloride salt as shown in Table G or, more commonly, used without further purification as the free base. In Table G, the Roman numeral IX indicates that the starting material was the corresponding phthalazine; the Roman numeral VIII indicates that the starting material was the corresponding phthalazinone.

General Method T

The procedure was substantially similar to General Method S except that 0.1-0.5 equivalent of sodium borohydride and 0.7 to 1.5 mL of diglyme per mmol of phthalazinone were added.

Example 149

4,5-Dihydro-3-ethyl-4-methyl-1-phenylmethyl-1H-2,4-benzodiazepine

(Formula I: $R^1$, $R^4$, $R^6$ = H; $R^2$ = Me; $R^3$ = Et; $R^5$ = Bzl)

A solution of 12.5 g (50 mmol) of 2-[(1-amino-2-phenyl)-ethyl]-N-methylbenzenemethanamine in 150 mL of isopropyl acetate was treated with 4.1 g (50 mmol) of sodium acetate and 30 mL (150 mmol) of triethylorthopropionate and 5 mL (87 mmol) of acetic acid. The mixture was refluxed for three hours and poured into 1.5 L of ice water containing 200 mL of 2N sodium hydroxide. The product was extracted into ethyl acetate, dried over sodium sulfate and stripped. The residue was recrystallized from isopropyl alcohol/ether to yield 7.5 g of the free base. The free base in ethanol was treated with 4.6 g of cyclohexane sulfamic acid and the solvent removed in vacuo. The residue was recrystallized from isopropyl alcohol/ether to provide 5.8 g of product as the cyclohexane sulfamic acid salt, mp 137-138.

Example 150

4,5-Dihydro 3-ethyl-1-phenyl-1H-2,4-benzodiazepine

(Formula I: $R^1$, $R^2$, $R^4$, $R^6$ = H; $R^3$ = Et; $R^5$ = Ph)

A mixture of 1.36 g (3.6 mmol) of 4-benzyl-4,5-dihydro-3-ethyl-1-phenyl-1H-2,4-benzodiazepine, 136 mg of 10% palladium on carbon, and 257 mg (4.0 mmol) of ammonium formate in 50 mL of methanol was refluxed under nitrogen for three hours. Four more 230-mg portions of ammonium acetate were added every two hours during reflux until TLC on silica gel with 5% diethylamine in ethyl acetate showed a complete conversion. The reaction was cooled, filtered and stripped. The residue was distributed between aqueous sodium hydroxide and ether. The ether extract was dried over sodium sulfate, treated with decolorizing carbon, filtered and stripped. The residue was taken up in 60:40 ethyl acetate/ether and acidified with dilute ethereal HCl. The resulting precipitate was filtered off and recrystallized from isopropanol/ether to yield 0.61 g (61%) of the hydrochloride salt of the product, mp 203-204.

Example 151

4,5-Dihydro-4-methyl-1-phenyl-1H-2,4-benzodiazepin-3-amine monohydrochloride

(Formula I: $R^1$, $R^4$, $R^6$ = H; $R^2$ = Me; $R^3$ = $NH_2$; $R^5$ = Ph)

A solution of 15 g (66 mmol) of 2-[(methylamino)-methyl]-α-phenylbenzenemethanamine in 85 mL of methanol was treated with 7.2 g (68 mmol) of cyanogen bromide at room temperature. The solution was stirred at room temperature for 18 hours and stripped. The residue was dissolved in ethanol and the ethanol stripped off. The residue was

recrystallized from methanol/isopropyl acetate to yield 4.55 g of the free base, mp 156-159. The mother liquors were dissolved in ethanol, treated with a slight excess of ethanolic HCl and recrystallized from ethanol to yield 1.3 g of the hydrochloride salt, mp 259-261.

Example 152

1,2,4,5-Tetrahydro-4-methyl-1-phenyl-3H-2,4-benzodiazepin-3-thione

To a suspension of 15 g (50 mmol) of 2-[(methylamino)-methyl]-$\alpha$-phenylbenzenemethanamine dihydrochoride in 100 mL of isopropyl alcohol was added 10 g (100 mmol) of potassium acetate followed by 3.3 mL (55 mmol) of carbon disulfide in 35 mL of isopropyl alcohol. The suspension was stirred at room temperature for one and one-half hours and then refluxed for 30 minutes. The reaction was chilled in ice and the internal salt of the carbamodithioic acid, contaminated with two equivalents of of potassium chloride, was filtered off. The carbamodithioic acid was suspended in 125 mL of 95% ethanol, and 1.3 mL of of 12N hydrochloric acid was added. The suspension was refluxed for three days, cooled, and 15.3 g (114%) of the crude benzodiazepin-3-thione was filtered off. A 6-g portion of the crude product was recrystallized from 2-ethoxy ethanol to yield 2.0 g (38%) of product, mp 208-209.

Example 153

3-[[2-(Diethylamino)ethyl]amino]-4,5-dihydro-4-methyl-1-phenyl-1H-2,4-benzodiazepine

[Formula I: $R^1$, $R^4$, $R^6$ = H; $R^2$ = Me; $R^3$ = $NH(CH_2)_2N(C_2H_5)_2$]

A slurry of 11.7 g (44 mmol) of 4-methyl 1-phenyl-1,2,4,5-tetrahydro-3H-2,4-benzodiazepin-3-thione of Example 152 in 146 mL ethanol was treated with 4.2 mL (67 mmol) of iodomethane in 30 mL ethanol added dropwise at 50°. The reaction was stirred at ambient temperature for 18 hours and 13.48 g (75%) of 4-methyl-1-phenyl-3-methylthio-4,5-dihydro-1H-2,4-benzo-diazepine was collected, mp 201-205, as the hydriodide salt.

A solution of 22.7 g (55 mmol) of the 3-methylthio-benzodiazepine in 285 mL of methanol was refluxed with 7.8 mL (55 mmol) of N,N-diethylethylenediamine for 18 hours. The reaction was filtered hot to remove a small amount of insoluble impurity, cooled, stripped, and distributed between methylene chloride and aqueous sodium hydroxide. The organic extracts were dried over magnesium sulfate and stripped. The residue was recrystallized with great difficulty as the fumarate salt from isopropanol. After multiple recrystallizations 1.5 g of the product was obtained as the difumarate hemihydrate, mp 160-162.

Example 154

4,5-Dihydro-4-methyl-1-phenyl-1H-2,4-benzodiazepin-3-sulfonic acid

(Formula I: $R^1$, $R^4$, $R^6$ = H; $R^2$ = Me; $R^3$ = $SO_3H$; $R^5$ = Ph)

Twenty-nine grams (108 mmol) of 1,2,4,5-tetrahydro-4-methyl-1-phenyl-3H-2,4-benzodiazepin-3-thione of Example 152 was treated with 2.4 g of sodium chloride, 420 mg of sodium molybdate dihydrate and 35 mL of 30% hydrogen peroxide in 50 mL of water and 10 mL of t-butanol according to the procedure of Maryanoff et al., J.O.C. 51, 1882 (1986). The reaction remained a suspension at all times, and after heating at 70-80° for two hours, the product was filtered off from the chilled suspension to yield 30.6 g (90%) of the sulfonic acid requiring no further purification, mp 188-190.

Example 155

4,5-Dihydro-4-methyl-1-phenyl-3(1-pyrrolidino)-1H-2,4-benzodiazepine

(Formula I: $R^1$, $R^4$, $R^6$ = H; $R^2$ = Me; $R^3$ = $C_4H_8N$; $R^5$ = Ph)

A mixture of 4.75 g (15 mmol) of the sulfonic acid of Example 154 and 20 mL of pyrrolidine was refluxed for 18 hours. The pyrrolidine was stripped off and the residue was chromatographed on 340 g of silica gel, eluting with 95:5 ethyl acetate/diethylamine to yield 3.12 g of residue which was recrystallized from 40 mL of hexane to yield 2.14 g (47%) of product, mp 118-119.

Example 156

3-[(4,5-Dihydro-4-methyl-l-phenyl-1H-2,4-benzodiazepin-3-yl)thio]-N,N-diethylpropaneamine

(Formula I: $R^1$, $R^4$, $R^6$ = H; $R^2$ = Me; $R^3$ = $S(CH_2)_3N(C_2H_5)_2$; $R^5$ = Ph)

A solution of of 12 g (45 mmol) of 1,2,4,5-tetrahydro-4-methyl-1-phenyl-3H-2,4-benzodiazepin-3-thione of Example 152 in 100 mL of DMF was treated with 1.24 g (50 mmol) of sodium hydride at 70° and 7.5 g (50 mmol) of 3-diethylaminopropyl chloride was added dropwise at 70°. The reaction was stirred at 70° for five hours and then at room temperature for two days. The reaction was poured into 250 mL of ice water and extracted twice into ethyl acetate. The product was extracted into 150 mL of 2N HCl, washed with ethyl acetate, made basic, and extracted back into ethyl acetate. The ethyl acetate solution was dried over magnesium sulfate, stripped, and the residue was dissolved in acetone. Two equivalents of maleic acid in 40 mL of acetone was added, followed by a small amount of ether. The resulting precipitate was recrystallized from acetone/ether to provide 13.2 g of product as the dimaleate salt, mp 95-97.

Example 157

4,5-Dihydro-4-methyl-3-methylthio-1-phenyl-1H-2,4-benzodiazepine

(Formula I: $R^1$, $R^4$, $R^6$ = H; $R^2$ = Me; $R^3$ = SMe; $R^5$ = Ph)

A solution of 8 g (30 mmol) of the thione of Example 152 and 2.7 mL (44 mmol) of methyl iodide in 100 mL of ethanol was refluxed two hours, cooled, and the hydriodide of the product filtered off. The salt was partitioned between methylene chloride and aqueous sodium bicarbonate, the organic layer dried over magnesium sulfate, and stripped. The residue was dissolved in ethanol and 2.7 g of methanesulfonic acid was added followed by ether. The resulting precipitate was filtered off and recrystallized from ethanol to yield 5.2 g of product as the methanesulfonate salt, mp 195-196.

Example 158

1,2,4,5-Tetrahydro-4-methyl-1-phenyl-3H-2,4-benzodiazepin-3-one

A solution of 32.8 g (145 mmol) of 2-[(methylamino)methyl]-α-phenylbenzenemethanamine in 215 mL of chloroform was treated with 25.9 g (159 mmol) of carbonyldiimidazole. The reaction was stirred at room temperature for 19 hours, washed four times with water, dried over sodium sulfate and stripped in vacuo. The gummy residue was triturated in and recrystallized from ethyl acetate to yield 26 g (71%) of product, mp 198-199.

Example 159

5-Butyl-4,5-dihydro-3-ethyl-4-methyl-1-phenyl-1H-2,4-benzodiazepine

(Formula I: $R^1$ = nBu; $R^2$ = Me; $R^3$ = Et; $R^4$ and $R^6$ = H, $R^5$ =Ph)

A suspension of 14.16 g (60 mmol) of 2-methyl-4-phenyl-1(2H)-phthalazinone in 340 mL of THF was cooled to -65° under nitrogen and treated with 24.8 mL (62 mmol) of 2.5N n-butyllithium in hexane. The mixture was stirred for 20 minutes at -65°, and 240 mL (240 mmol) of 1N borane-THF complex was added. The solution was allowed come

to room temperature and 340 mg (9 mmol) of sodium borohydride were added. The reaction was refluxed for 20 hours, another 340 mg of sodium borohydride was added, the reaction refluxed another 24 hours. The reaction was cooled and quenched with 100 mL of methanol. Eighty milliliters of 3.5N HCl in methanol was added, the reaction was refluxed two hours and 19.9 g (93%) of the dihydrochloride of 2-[1-(methylamino)pentyl]-α-phenyl-benzenemethanamine was isolated by filtration. The benzene-methanamine was treated with triethylorthopropionate and sodium acetate in isopropyl acetate according to General Method E to yield 9.48 g of the free base of the product, mp 102-114 after recrystallization from methyl t-butyl ether/hexane. Seven grams of the free base was coverted to the hydrochoride salt and recrystallized from acetone ether to yield 5.08 g of product as the monohydrochloride salt, mp 209-211.

Example 160

4,5-Dihydro-1,5-diphenyl-3-ethyl-4-methyl-1H-2,4-benzodiazepine

(Formula I: $R^1$, $R^5$ = Ph; $R^2$ = Me; $R^3$ = Et; $R^4$, $R^6$ = H)

The procedure of Example 159 was used, substituting phenyllithium for butyllithium. The intermediate 2-[(methylamino)phenylmethyl]-α-phenylbenzenemethanamine was crystallized as the dihydrochloride salt containing 0.6 moles water of hydration, mp 202-216. It was cycylized with triethyl-orthopropionate as in Example 158 to yield 32% of the product as the hydrochloride salt, mp 275-276, from acetone/ether.

Example 266

4-5-Dihydro-1-(4-hydroxyphenyl)-4-methyl-3-(2-phenylethyl)-1H-2,4-benzodiazepine

(Formula I: $R^1$, $R^4$, R6 = H; $R^2$ = Me;
$R^3$ = $CH_2CH_2Ph$ $R^5$ = 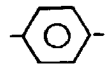-OH)

A solution of 6.78 g (17 mmol) of the methoxy compound of example 60 in 70 mL of methylene chloride was treated with 32 mL of 1M boron tribromide in methylene chloride (32 mmol) at 0° under nitrogen for 2 hours. The reaction was poured into 2N aqueous HCl, stirred 1 hour, filtered free of boron salts and extracted into methylene chloride with a trace of methanol after making basic with $Na_2CO_3$. The organic layer was dried, stripped and the residue taken up in methanol. Methanolic HCl was added and the salt crystallized by the addtion of ether. The hydrochloride was recrystallized from methanol, mp 245-247, yield 90%.

Example 267

4,5-Dihydro-3-[2-(4-hydroxyphenyl)ethyl]-4-methyl-1-phenyl-1H-2,4-benzodiazepine

(Formula I: $R^1$, $R^4$, $R^6$ = H; $R^2$ = Me,
$R^3$ = $CH_2CH_2$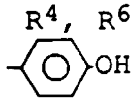OH; $R^5$ = Ph)

By a process analogous to that of Example 266, 1.14 g of 4,5-dihydro-3-[2-(4-hydroxyphenyl)ethyl]-4-methyl-1-phenyl-1H-2,4-benzodiazepine was obtained as the hydrochloride salt from 2.47g (6.1 mmol) of the methoxy compound of Example 35, mp 160-162 from methanol/ether.

Example 268

4,5-Dihydro-1-(4-hydroxyphenyl)-3-[2-(4-hydroxyphenyl)ethyl]-4-methyl-1H-2,4-benzodiazepine

(Formula I: $R^1$, $R^4$, $R^6$ = H; $R^2$ = Me;
$R^3$ = $CH_2CH_2$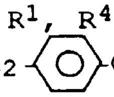OH; $R^5$ = 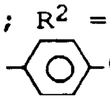-OH

By a process analogous to that of Example 266, 1.80 g of 4,5-dihydro-1-(4-hydroxyphenyl)-3-[2-(4-hydroxyphenyl) ethyl]-4-methyl-1H-2,4-benzodiazepine was obtained from 4.5 g (8.7 mmol) of the dimethoxy compound of example 229 using 3.5 equivalents of boron tribromide. The free base was insoluble in methylene chloride. The hydrochloride hemihydrate was obtained by recrystallization from MeCN/MeOH, mp 266-228.

Resolution of enantiomers

Example 167

(R)-(+)-4,5-Dihydro-4-methyl-1-phenyl-3-(2-phenylethyl)1H-2,4-benzodiazepine

To a 1 L Erlenmeyer flask was added 100 mL of methanol, 200 mL of water and 49.8 g (0.133 mol) of the racemic hydrochloride salt of Example 25. The solution was stirred for ten minutes, then 200 mL of t-butylmethyl ether (TBME) added to the homogeneous solution followed by 220 mL (0.66 mol, 5.0 eq) of 3N sodium hydroxide. The mixture was stirred for 10 minutes. The layers were separated and the aqueous layer extracted with 100 mL of saturated sodium chloride. The organic layer was dried over magnesium sulfate, filtered and the solvent removed under reduced pressure at 30°C to give a quantitative yield of free base. The viscous golden-brown oil was placed on a vacuum pump at 0.5 mmHg for 1 hour.

The free base was dissolved into 40 mL of methanol with slight warming. The solution was transferred to a 500 mL 3 neck flask equipped with mechanical stirrer and condenser. The transfer was completed by rinsing with an additional 20 mL of methanol. The methanol solution was warmed to 45°C with an external temperature-controlled water bath.

To a 250 mL beaker was added 40.4 g (0.113 mol, 0.85 eq) of D-O,O'-dibenzoyltartaric acid and 40 mL of methanol. (Slight warming may be necessary to get chiral acid into solution.) The methanol solution of the chiral acid was added slowly with constant stirring to the solution of free base. The resulting mixture became a very light green color. An additional 20 mL of methanol was used for rinsing to complete the transfer. After stirring 5 minutes, the solution was seeded. The product began to precipitate immediately. The solution was stirred overnight at 45°C. The granular, white precipitate was collected on a Buchner funnel, washed 3 x 25 mL with cold methanol (5°C) and dried overnight at 60°C under reduced pressure.

The dried dibenzoyltartrate salt weighed 40.9 g (88%) after correcting for the quantity of seed crystal; $[\alpha]_D^{25}$ = +192. (c=1, methanol); mp 143-145°C dec.

The hydrochloride salt may be made by the following procedure:

The free base from 100 g (0.143 mol) of dibenzoyltartrate salt was prepared as above. The free base was dissolved into 300 mL of ethyl acetate. The solution was transferred to a 2-liter 3-neck flask equipped with mechanical stirrer, condenser and additional funnel. The transfer was completed by rinsing with an additional 300 mL of ethyl acetate. The ethyl acetate solution was warmed to 45°C with a temperature-controlled water bath.

To the warmed ethyl acetate solution of the free base was slowly added 69 mL of a 2.3N hydrogen chloride/ethyl acetate solution. The addition of the acid was completed over a 0.5 h period, followed by stirring at 45°C for 1 hour. The ethyl acetate suspension of the resulting hydrochloride salt was refluxed for 1 hour to eliminate the excess hydrogen chloride present in the solvent and cooled to ambient temperature. The flocculent white precipitate was collected on a Buchner funnel and washed 3 x 150 mL with ethyl acetate. The product was dried overnight at 80°C under reduced pressure.

The dried hydrochloride salt weighed 50.9 g, $[\alpha]_D^{25}$ = +234° (c=1, methanol); mp 197-199°C.

Example 168

(S)-(-)-4,5-Dihydro-4-methyl-1-phenyl-3-(2-phenylethyl)-1H-2,4-benzodiazepine

The mother liquors from crystallization in Example 167 were stripped and the free base liberated as before using tBuOMe and aqueous NaOH. The free base was dissolved in 100 mL of methanol, treated with 34.3 g of dibenzoyl-L-tartaric acid and seeded. There was obtained 37.2 g of the diastereomeric salt of the (-) isomer, mp 160-170, $[\alpha]_D^{25}$ -198° (C=1, MeOH). The free base was generated as above, and the HCl salt was formed and recrystallized from acetonitrile/ether, mp 198-199, $[\alpha]_D^{25}$ = -249° (C=1, $CHCl_3$).

Example 169

(+)-4,5-Dihydro-1-phenyl-1,3,4-trimethyl-1H-2,4-benzodiazepine

By a process analogous to that of Example 167 involving multiple recrystallizations, 1.4 g of (+)-4,5-dihydro-1-phenyl-1,3,4-trimethyl-1H-2,4-benzodiazepine was obtained from 8.9 g (33.7 mmol) of the racemic product of Example 96 and 12.7 g (33.7 mmol) of dibenzoyl-L-tartaric acid hydrate. The free base was obtained, without recrystallization, by stripping the tBuOMe, mp 115-116, $[\alpha]_D^{25}$ = +101° (C=1, MeOH).

Example 170

(-)-4,5-Dihydro-1-phenyl-1,3,4-trimethyl-1H-2,4-benzodiazepine

By a process analogous to that of Example 168, 1.9 g of (-)-4,5-dihydro-1-phenyl-1,3,4-trimethyl-1H-2,4-benzodiazep-ine was obtained from the mother liquors of Example 169, mp 116-117, $[\alpha]_D^{25}$ = -93° (C=1, MeOH).

Example 171

(R)-(+)-4,5-Dihydro-3-ethyl-4-methyl-1-phenyl-1H-2,4-benzodiazepine

By a process analogous to that of Example 167, 7.5 g of R-(+)-4,5-dihydro-3-ethyl-4-methyl-1-phenyl-1H-2,4-benzodiazepine was obtained from 92 g of the free base of the racemic product of Example 8, after repeated crystallization. The hydrochloride salt was obtained from ethanol/ether, mp 244-247, $[\alpha]_D^{25}$ = +347° (C=1, $CHCl_3$).

The d-10-camphorsulfonic acid salt was obtained from acetonitrile, mp 215-218, $[\alpha]_D^{25}$ = + 203° (C=1, MeOH), $[\alpha]_D^{25}$ = +242° (C=1, $CHCl_3$).

Example 172

(S-) (-)-4,5-Dihydro-3-ethyl-4-methyl-l-phenyl-1H-2,4-benzodiazepine

By a process analogous to that of Example 168, 15 g of the levo enantiomer was obtained from the mother liquors of Example 171. The product was crystallized as the hydrochloride from ethanol/ether, mp 247-249, $[\alpha]_D^{25}$ = -343° (C=1, $CHCl_3$).

Example 173

(S)-(-)4,5-Dihydro-3-ethyl-4-methyl-1-phenyl-1H-2,4-benzodiazepine

The following procedure describes an alternate synthesis of the compound of Example 172:

Two grams (6.3 mmol) of the monohydrochloride of (S)-N-[[(2-(methylamino)methyl]phenyl]phenylmethyl]propanamide (Example 181) was stirred in 15 mL of toluene under nitrogen and 3.45 mL of 2M trimethyl aluminum in toluene was added at 0°. The mixture was stirred two hours at room temperature, then 1.5 hours at reflux. The reaction was cooled and quenched with 0.31 mL of water followed by 0.93 mL of 30% aqueous NaOH. Methylene chloride, a small amount of methanol and some sodium sulfate were added, the mixture was filtered, stripped and the residue recrystallized from MeOH/ether as the hydrochloride, mp 247-248.

Example 174

2-[[(1,1-Dimethylethyl)amino]methyl]-α-phenylbenzene-methanamine

(Formula IV: $R^{1a}$, $R^6$ = H, $R^{2a}$ = tBu; $R^{5a}$ = Ph)

A solution of 15.9 g (90 mmol) of N-t-butylbenzamide in 390 mL of THF was cooled to -15° under nitrogen and 77 mL (193 mmol) of 2.5N n-butyllithium in hexane was added. The mixture was stirred at -5 ± 3° for one hour and 17.5 g (99 mmol) of the trimethylsilylimine of benzaldehyde [prepared according to the procedure of Hart et al., J. Org. Chem. 48, 289-294 (1983)] was added over ten minutes at -10°. The reaction was stirred at 0° for one hour, then 5° for 45 minutes. It was poured into 400 mL of ice water containing 225 mL of 2N HCl and washed twice with ether. The

aqueous layer was made basic with sodium hydroxide and extracted into ether. The ether extracts were dried over sodium sulfate and stripped to yield 25.4 g of 2-[(amino)(phenyl)methyl]-N-(1,1-dimethyl-ethyl)benzamide.

The entire portion of aminoamide in 50 mL of THF was combined with 450 mL (450 mmol) of 1N borane-THF complex and the mixture stirred at reflux for 18 hours. The reaction was cooled, 225 mL of methanol was added, and the solution was refluxed for one hour. It was recooled and 200 mL of half-saturated methanolic HCl was added. The solution was again refluxed for one hour, evaporated in vacuo and the residue recrystallized from chloroform/ether to yield 21.3 g (70%) of product as the dihydrochloride, mp 222-231.

Example 175

2-(Aminomethyl)-N-methyl-α-phenylbenzenemethanamine

(Formula IV: $R^{1a}$ = Ph; $R^{2a}$ = Me; $R^{5a}$, $R^{6}$ = H)

Seventy-five grams (0.36 mole) of 2-benzoylbenzaldehyde was dissolved in 70 mL of THF and 18.5 g (0.39 moles) of methylhydrazine was added over 30 minutes at 0°. The suspension became a homogeneous solution which was allowed to stand for four days. A first portion comprising 8.5 g of product was obtained by addition of hexane and filtration. A second portion of 27.5 g of product was obtained by chromatography on silica gel with 85:15 methylene chloride/ethyl acetate. The mp of the hydrazone after recrystallization from methylene chloride-hexane was 164-165.

A solution of 44.5 g of the methylhydrazone in 80 mL of THF was treated with 374 mL of 1 M borane-THF and stirred at reflux. After 24 hours and 72 hours, additional 187 mL portions of 1 M borane were added. After six days the reaction was worked up as described in Example 164 and the dihydrochloride recrystallized from methanol ether, mp 224-226.

Example 176

2-(Aminomethyl)-α-phenyl-N-(phenylmethyl)benzenemethanamine

(Formula IV: $R^{1a}$ = Ph; $R^{2a}$ = Bzl; $R^{5a}$, $R^{6}$ = H)

Sixty-two grams (0.30 mole) of 2-benzoylbenzaldehyde in 140 mL of THF was treated with 81.5 g (0.59 mole) of potassium carbonate and 64 g (0.32 mole) of benzylhydrazine dihydrochloride. The mixture was stirred 30 minutes at 0° and 40 mL of methylene dichloride was added. The mixture was stirred at room temperature for one day, filtered and stripped in vacuo to provide 131 g of 2-benzyl-1-phenyl phthalazinium chloride.

The crude phthalazinium chloride in 175 mL of THF was treated with 1.325 L of 1 M borane-THF at reflux under nitrogen. After 24 hours the reaction was worked up as described in Example 175 and the dihydrochloride salt was formed by precipitation from ether with ethereal HCl to provide 36.8 g of dihydrochloride of the product, mp 175-178.

Example 270

N-Methyl-α'-phenyl-2,3-thiophenedimethanamine

(Formula XXXVII: A = thiophene; R1a = H;

$R^{2a}$ = Me; $R^{5a}$ = Ph

Following the procedure of General Method T, 19 g of N-methyl-α'-phenyl-2,3-thiophenedimethanamine was prepared from 32.6 g (0.135 mol) of 6,7-dihydro-6-methyl-7-oxo-4-phenylthieno[2,3-d]pyridazine. The product was recrystallized as its dihydrochloride from ethanol-water, mp 290-292.

TABLE H

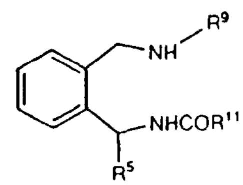

| Example No. | $R^9$ | $R^{11}$ * | $R^{5a}$ | Source | Yield % | Melting Range | Salt/Solvate | Recrystallization From |
|---|---|---|---|---|---|---|---|---|
| 177 | Me | $CH_2CH_2Ph$ | Ph | General Method U benzodiazepine from Ex. 25 | 96 | 110-123 | HCl (amorphous) | $CH_2CH_2$/ether |
| 178 | Me | $CH_2CH_2Ph$ | 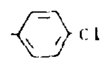 | alumina chromatography of Ex. 63 hexane/$CH_2CH_2$ 80:20 | 15 | 274-275 | HCl | EtOH/ether |
| 179 | Me | 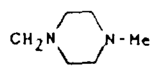 | Ph | silica gel chromatography of Ex. 76 MeOtBu/hexane/iPrNH$_2$ 85:10:5 | 12 | 134-136 | 1.5 fumarate | MeOH/ether |
| 180 | Me | 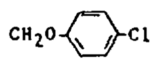 | Ph | silica gel chromatography of Ex. 39 toluene/hexane/iPrNH$_2$ 50:49:1 | 26 | 178-179 | HCl | EtOH/ether |
| 181 | Me | Et | Ph | General Method U benzodiazepine of Ex. 8 | 89 | 206-208 | HCl | MeOH/ether |
| 182 | Me | Ph | Ph | silica gel chromatography of Ex. 1 0.75% iPrNH$_2$ in tBuOMe | 8 | 132-133 | free base | $CH_2Cl_2$/tBuOMe/ hexane |
| 183 | iPr | Me | Ph | from recrystallization in EtOAc/hexane of Ex. 23 | 4 | 116-117 | free base | EtOAc/hexane |
| 184 | $CH_2CH_2NEt_2$ | Et | Ph | silica gel chromatography of Ex. 50 $CHCl_3/Et_3N$ 96:4 | 2 | viscous liquid | free base | --- |

$*R^{11} = (CH_2)_nR^{10}$

**TABLE H**

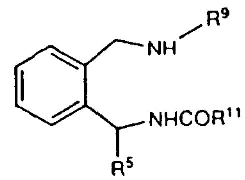

| Example No. | $R^9$ | $R^{11}$ * | $R^{5a}$ | Source | Yield % | Melting Range | Salt/Solvate | Recrystallization From |
|---|---|---|---|---|---|---|---|---|
| 208 | Ph | $Ch_2CH_2Ph$ | Me | silica gel chromatography of Ex. 235 MeOtBu/hexane 1:1 | 34 | 137-138 | free base | MeOtBu/hexane |
| 209 | Me | 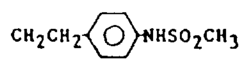 | Phe | crystallized from free base of Ex. in toluene | 34 | 224-228 | HCl | EtOH/ether |
| 273 | 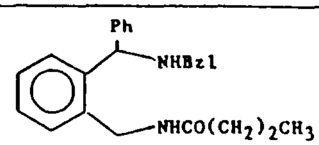 | | | silica gel chromatography of Ex. 70 $CH_2Cl_2$/EtOAc/diethylamine (49:50:1) | | 116-119 | HCl | |

$$* \ R^{11} = (CH_2)_n R^{10}$$

<u>Examples 177-184,208,209, 273</u>

Aminoamides of formula III

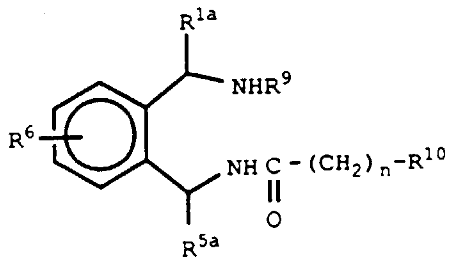

III

were obtained as by-products of the synthesis of the corresponding benzodiazepines and were usually isolated via chromatography on silica gel using basic elution solvents. They were also obtained by hydrolysis of the corresponding benzodiazepines as described in General Method U. Examples are given in Table H

General Method U

A solution of the appropriate benzodiazepine in 3 to 5 mL of methanol per millimole of diazepine was stirred at room temperature for 1 to 4 days with 3 to 5 equivalents of potassium hydroxide in 1 to 2 mL of water per millimole of diazepine. Aqueous sodium chloride was added and the aminoamide was extracted into ether. The ether layer was dried over $MgSO_4$, filtered, stripped and the residue was treated as shown in Table H. It is anticipated that any species described in Tables A through E could be converted to the corresponding aminoamide by this procedure.

## TABLE L

Reduction of $NO_2$ to $NH_2$ in compound of formula:

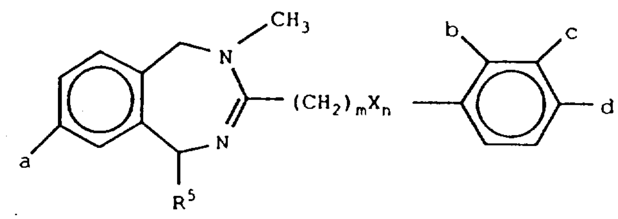

| Example No. | $R^5$ | X | n | Position of NO2 -->NH2 | m | Method | Yield % | Melting Range | Salt | Recrystal-lized from |
|---|---|---|---|---|---|---|---|---|---|---|
| 250 | Ph | CH=CH | 1 | c | 0 | V | 58 | 234-237 | 2HCl | MeOH/ether |
| 251 | Ph | --- | 0 | d | 0 | V | 81 | 314-315 | 2HCl | MeOH/ether |
| 252 | Ph | --- | 0 | a | 2 | V | 77 | 209-211 | 2HCl | MeOH/ether |
| 253 | Ph | --- | 0 | b | 2 | W | 92 | 215-228 | 2HCl | MeOH/ether |
| 254 | 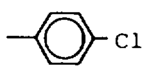 | --- | 0 | d | 2 | W | 80 | 290-291 | 2HCl | MeOH/ether |
| 255 | Ph | --- | 0 | d | 2 | W | 88 | 173-175 | HCl.1/4 EtOH | EtOH/ether |
| 256 | Ph | CH=CH | 1 | d | 0 | V | | 212-215* | 2HCl | MeCn/ether |

*4.1 trans/cis

General Method V

The appropriate nitro compound, as its salt, usually the fumarate salt, was dissolved in about 10 mL of dry methanol

per millimole of nitro compound and 0.1 to 0.15 g of 10% Pd on carbon was added per millimole of nitro compound. The reaction was stirred at 18-24° and 7.5 to 8.0 equivalents of ammonium formate was added. After 1-2 hours the reaction was filtered, stripped, distributed between methylene chloride and 2N NaOH, separated, dried and stripped. The residue was crystallized as shown in Table L.

General Method W

The appropriate nitro compound, as its hydrochloride salt or the free base plus one equivalent of methanolic HCl, was dissolved in about 20 mL of methanol or ethanol per millimole of nitro compound and about 0.05 to 0.1 g of 10% Pd on carbon was added per millimole of nitro compound. The mixture was hydrogenated at 3.5 to 1.4 atm on a Parr shaker. When the calculated amount of hydrogen had been consumed, the reaction was filtered, excess ethereal HCl was added, and the solution was stripped. The residue was crystallized as shown in Table L.

Example 274

4-[2-(4,5-Dihydro-3-ethyl-1H-2,4-benzodiazepin-4-yl) ethyl]benzeneamine

(Formula I: $R^1$, $R^4$, $R^6$ = H; $R^2$ = $CH_2CH_2$ - 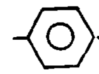 -$NH_2$;
$R^3$ = Et; $R^5$ = Ph

By General Method W, 4.50 g (10.3 mmol) of 4,5-dihydro-3-ethyl-4-[2-(4-nitrophenyl)ethyl]-1H-2,4-benzodiazepine hydrochloride of Example 276 was reduced to 3.33 g of of 4[2-(4,5-dihydro-3-ethyl-1H-2,4-benzodiazepin-4-yl)ethyl] benzeneamine as its monohydrochloride, mp 149-151 from EtOH-ether.

## TABLE M

Acylation and Sulfonylation of Amines of Formula:

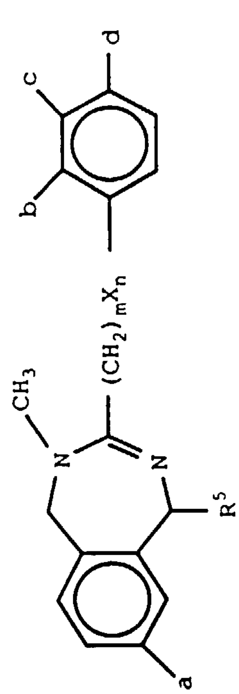

| Example No. | $R^5$ | m | X | n | Position of NH2 | Product | Method | Yield % | Melting Range | Salt | Recrystallized from |
|---|---|---|---|---|---|---|---|---|---|---|---|
| 257 | Ph | 0 | CH=CH | 1 | d | $NHSO_2CH_3$ | X | 82 | 274-276 | HCl | EtOH/ether |
| 258 | Ph | 0 | CH=CH | 1 | c | $NHSO_2CH_3$ | X | 60 | 297-298 | HCl | MeOH/ether |
| 259 | Ph | 0 | CH=CH | 1 | d | $NHCOCH_3$ | X | 62 | 174-190 | HCl.2H2O | $EtOH/H_2O$ |
| 260 | Ph | 0 | --- | 0 | d | $NHSO_2CH_3$ | X | 53 | 283-284 | HCl | MeOH/ether |
| 261 | Ph | 0 | --- | 0 | d | $NHCOCH_3$ | X | 76 | >300 | HCl | MeOH/ether |
| 262 | Ph | 2 | --- | 0 | d | $NHSO_2CH_3$ | X | 87 | 153-159 | HCl.EtOH | EtOH/ether |
| 263 | Ph | 2 | --- | 0 | a | $NHSO_2CH_3$ | X | 75 | 170-182 | HCl | MeCN/ether |
| 264 | 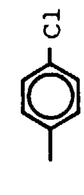 | 2 | --- | 0 | d | $NHSO_2CH_3$ | X | 93 | 168-172 | HCl | MeOH/ether |

General Method X

A solution of the appropriate amine as its dihydrochloride and from 3 equivalents of pyridine to 30 equivalents of pyridine were stirred at 0° in about 10 mL of methylene chloride per millimole of amine under a nitrogen atmosphere while 1.1 to 1.5 equivalents of methanesulfonyl chloride or acetyl chloride was added dropwise. The reaction was stirred at 0° for 1-2 hours and one volume of saturated aqueous $Na_2CO_3$ was added. In a few cases where TLC showed incomplete reaction, an additional 1 to 3 equivalents of chloride was added before the $Na_2CO_3$ solution. The layers were separated and the organic layer was stripped. The residue was flash chromatographed, if necessary, on silica

gel eluting with MeOH/MeOtBu/isopropylamine 49:49:2. The product was recrystallized as shown in Table M.

Example 275

N-[4-[2-(4,5-Dihydro-3-ethyl-1H-2,4-benzodiazepin-4-yl) ethyl]phenyl]methanesulfonamide

(Formula I : $R^1$, $R^4$, $R^6$ = H; $R^2$ = $CH_2CH_2$ 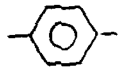 $-NHSO_2CH_3$;
$R^3$ = Et; $R^5$ = Ph)

By General Method X, 3.25 g of 4-[2-(4,5-dihydro-3-ethyl-1H-2,4-benzodiazepin-4-yl)ethyl]benzeneamine of Example 274 was converted to 3.49 g of N-[4-[2-(4,5-dihydro-3-ethyl-1H-2,4-benzodiazepin-4-yl)ethyl]phenyl]-methanesulfonamide, mp 129-142 as the free base from EtOH-ether-methylene chloride.

Starting Materials

The phthalazinones, which are the starting materials for the synthesis of diamines described in Table G, are generally available by methods known in the literature. They are most commonly synthesized by condensation of the corresponding γ-ketoacids with the appropriate hydrazine. For example,

Example 185

2-Methyl-4-phenyl-1(2H)-phthalazinone

A 100 gallon stainless steel unit was charged with 40.0 kg of 2-benzoylbenzoic acid and 87.5 kg of toluene. Methylhydrazine was added over about 45 minutes with the internal temperature rising to 34°.

The resulting thin slurry was warmed at reflux (95-118°) for 4 1/2 hours while collecting about 7.5 L of water.

The reaction mixture was cooled slowly with initial precipitation evident at 88°. The resulting slurry was cooled to 0 to -5° before collecting the beige colored crystals. The cake was washed with 2 x 20 L of cold toluene and dried in vacuo at 45-50° overnight to afford 38.0 kg (91.0% yield) 2-methyl-4-phenyl-1(2H)-phthalazinone, mp 166-168.

Example 271

6,7-Dihydro-6-methyl-7-oxo-4-phenylthieno[2,3-d] pyridazine

A solution of 31.2 g (0.134 mol) of 3-benzoyl-2-thiophenecarboxylic acid in 400 mL of ethanol was treated with 9.3 (0.2 mol) of methylhydrazine at room temperature for 18 hours, refluxed 3 hours, cooled and 30.7 g of the product filtered off, mp 174-175.

The 3-benzoyl-2-thiophene carboxylic acid was obtained from 3-bromothiophene by the method of MacDowell and Ballas [J. Org. Chem. 42, 3717 (1977).]

In the cases where the appropriate alkylhydrazine for the condensation to the phthalazinone is not readily available, the γ-ketoacid is condensed with hydrazine and the resulting 2-unsubstituted 1-phthalazinone is alkylated. For example,

Example 186

4-Phenyl-2-(2-phenylethyl)-1 (2H)-phthalazinone

Eighty-five grams (0.38 moles) of 4-phenyl-1(2H)-phthalazinone was added to 18.4 g (0.47 moles) of sodium hydride in 1L of DMSO in four portions. The mixture was stirred for two hours at room temperature until evolution of hydrogen had ceased, and 95.5 g (0.52 moles) of 2-bromoethylbenzene was added. The mixture was stirred 1.5 hours at room temperature, 1 L of 2N NaOH was added and the slurry was poured into 1 L of water. The product was filtered off and dried to yield 118 g (95%) of 4-phenyl-2-(2-phenylethyl)-1(2H)-phthalazinone, mp 135-138.

Example 278

2-[2-(4-Nitrophenyl)ethyl]-4-phenyl-1(2H)-phthalazinone

(Formula VIII: $R^2$ = $CH_2CH_2$ 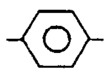 $-NO_2$,

$R^5$ = Ph, $R^6$ = H)

By a procedure analogous to that of Example 186, 11.8 g of 2-[2-(4-nitrophenyl)ethyl]-4-phenyl-1(2H)-phthalazinone was prepared from 10.0 g (45 mmol) of 4-phenyl-1 (2H)-phthalazinone and 11.6 g (50 mmol) of 4-nitrophenethyl bromide. The product was recrystallized from EtOAc-ether-hexane, mp 152-155°.

Synthesis of Esters of the formula

$$R^8CH{=}CH\text{-}COOEt$$

and

$$R^8CH_2CH_2COOEt$$

wherein $R^8$ is heteroaryl

In those cases where the appropriate propanoate and propenoate esters were not commercially available, the propenoate was synthesized by condensation of ethyl acetate with the appropriate aldehyde in the presence of one equivalent of sodium metal. The unsaturated esters were reduced with a large excess of magnesium metal in methanol to provide the propanoates.

Miscellaneous syntheses of diamines, phthalazines, and precursors are shown below:

Example 187

4-Benzyl-2-methyl-1(2H)-phthalazinone

A solution of 30 g of potassium hydroxide and 31.3 g (140 mmol) of benzylidenephthalide in 100 mL of water was heated to homogeneity and poured into a solution of 40 mL of $H_2SO_4$ in 250 mL of water. After cooling, the resulting solid was collected and dissolved in aqueous sodium bicarbonate. 2N HCl was added until the first signs of precipitation, the aqueous solution was washed four times with chloroform and then acidified with excess 2N HCl. A white precipitate of 20.3 g of 2-(1-oxo-2-phenylethyl)benzoic acid was filtered off and dried, mp 74-75, after recrystallization from ethanol water. This $\gamma$-ketoacid was treated with methylhydrazine according to the method of Example 185 to yield 17.3 g of the phthalazinone product, mp 144-146.

Example 188

2-Methyl-4-thienyl-1(2H)-phthalazinone

A solution of 74.1 g (0.5 mole) of phthalic anhydride in 300 mL of nitrobenzene was treated with 147 g (1.1 mole) of aluminum chloride. The solution was stirred for two hours and 42.1 g (0.5 mole) of thiophene was added dropwise over 80 minutes at 40-45°. The reaction was stirred at 50-55° for two hours and then let sit at room temperature overnight. The reaction was poured into 2.8 L of cold water, stirred, separated, and the nitrobenzene was removed from the nitrobenzene layer by steam distillation. The residue was recrystallized from toluene to provide 31.1 g (27%) of 2-thienoylbenzoic acid, mp 141-143. The thienoylbenzoic acid was treated with methylhydrazine as described in Example 185 to provide 24.4 g (75%) of the phthalazinone product, mp 143-144, after recrystallization from ethyl acetate.

Example 189

5-Fluoro-3,4-dihydro-3-methyl-1-phenylphthalazine

To a solution of 20 g (0.14 mole) of 2-fluoro-6-chloro-toluene in 125 mL of THF was added 6.9 g (0.28 mole) of magnesium turnings. The mixture was refluxed while 12 mL of 1,2-dibromoethane in 50 mL of benzene was added dropwise over three hours. The reaction was refluxed a further hour and 15 mL of benzonitrile was added. The reaction was refluxed a further two hours, cooled and quenched with 50 mL of water added dropwise. The mixture was extracted into ethyl acetate, dried over sodium sulfate and stripped. The residue was dissolved in 50 mL of ethanol, 25 mL of 1N HCl was added, and the mixture was refluxed for three hours. The ethanol was stripped, the product was extracted into ethyl acetate, the ethyl acetate dried over sodium sulfate and stripped. The residue of 3-fluoro-2-methyl-benzophenone was chromatographed on silica gel with 20% ether in hexane.

A solution of 1.69 g (7.89 mmol) of the benzophenone in 40 mL of carbon tetrachloride was treated with 100 mg of benzoyl peroxide and 1.5 g (8.43 mmol) of N-bromosuccinimide. The reaction was stirred at room temperature for two hours and then refluxed an additional four hours during which a second portion of 50 mg of benzoyl peroxide and 400 mg of N-bromosuccinimide were added. The reaction was cooled, a small amount of impurity filtered off, and the filtrate concentrated in vacuo to provide 2.5 g of 2-bromomethyl-3-fluorobenzophenone, presumably containing trapped carbon tetrachloride.

The residue of α-bromomethylketone was dissolved in 40 mL of chloroform and a mixture of 1.5 mL of triethylamine and one equivalent of methylhydrazine was added dropwise. The reaction was stirred one hour, washed with 20 mL of aqueous sodium bicarbonate and filtered directly through silica gel eluting with 25% ethyl acetate in hexane. Concentration in vacuo gave 1.86 g (98%) of product which was reduced immediately as shown in Table G.

Example 190

2-Benzoyl-5-fluorobenzoic acid

Following the procedure of Example 189, 4.0 g (21.2 mmol) of 2-bromo-5-fluoro toluene was treated with 2.4 mL (23.5 mmol) of benzonitrile. The imine resulting from the condensation was not hydrolyzed. Instead 28.6 g (0.13 mole) of 4-fluoro-2-methyl-benzophenoneimine in 200 mL of water and 100 mL of pyridine was refluxed for eight hours and treated with four portions of potassium permanganate at roughly two-hour intervals. The portions were 53 g, 28 g, 20 g, and 10 g. The reaction was cooled, filtered through diatomaceous earth and concentrated in vacuo. The residue was distributed between aqueous acetic acid and ethyl acetate, the ethyl acetate was dried over magnesium sulfate, and the ethyl acetate was removed in vacuo to provide 16.2 g (51%) of a yellow gum which was used as is.

Example 191

2-Benzoyl-4-fluorobenzoic acid

The procedure of Example 190 was used to provide 14.2 g (53%) of product from 21.3 g of 2-bromo-4-fluorotoluene.

Example 192

3,4-dihydro-3-methyl-1-phenylbenzo[f]phthalazine

A solution of 10 g (45 mmol) of 1-bromo-2-methylnaphthalene in 75 mL of THF was refluxed with 1.2 g (50 mmol) of magnesium turnings for three hours. The reaction was cooled on ice and 4.8 mL (43 mmol) of benzaldehyde was added. The ice was removed, the reaction was stirred 45 minutes and quenched with 5 mL of 1N HCl followed by 50 mL of water. The reaction was extracted into ethyl acetate, dried over sodium sulfate and flash chromatographed through silica gel with 5-10% ethyl acetate in hexane to provide 8.4 g (75%) of 2-methyl-a-phenyl-1-naphthalene-methanol as a pale yellow gum.

A solution of 10.0 g (40 mmol) of the secondary alcohol in dichloromethane was treated with 12.4 g (58 mmol) of pyridine chlorochromate, refluxed briefly, and stirred at room temperature for one hour. The reaction was diluted with 150 mL of ether and filtered through florisil. Concentration of the filtrate in vacuo afforded 7.92 g (80%) of 1-benzoyl-2-methylnaphthalene as a bright orange gum which slowly crystallized on standing.

By the procedure described in Example 189, 4.4 g (18 mmol) of the benzoylnaphthalene was converted to 1.73 g of the phthalazine product, which was reduced immediately as shown in Table G.

Example 193

3,4-Dihydro-3,8-dimethyl-1-phenylphthalazine

By a procedure exactly analagous to that of Example 192, the phthalazine was synthesized from 2-bromo-m-xylene.

Example 194

2-Aminomethyl-α-phenylbenzenemethanamine

To a slurry of 3.8 g (100 mmol) of lithium aluminium hydride in 120 mL of THF was added 11.1 g (50 mmol) of 4-phenyl-1(2H)phthalazinone. The mixture was refluxed one hour, cooled, diluted with 100 mL of ether and sequentially treated with 3.8 mL of water, 3.8 mL of 15% aqueous sodium hydroxide and 11.4 mL of water. The mixture was stirred for 30 minutes and the granular precipitate filtered off. The filtrate was diluted with a little toluene, dried over sodium sulfate and stripped to provide 14.1 g of an oil which was dissolved in 180 mL of ethanol and hydrogenated at 50 psi in the presence of 20 mL of ethanolic HCl and 1.5g of 10% palladium on carbon. After 24 hours a precipitate had formed. The reaction was filtered, the precipitate was slurried in 250 mL of hot methanol and filtered again. The combined filtrates were stripped to about 100 mL and diluted with ether. On cooling, 7.3 g of 1,2,3,4-tetrahydro-1-phenylphthalazine as the monohydrochloride salt was filtered off. It was recrystallized from methanol/ether to provide 6.93 g (56%) of product, mp 251-253.

The tetrahydrophthalazine was redissolved in 200 mL of methanol by warming and hydrogenated at 50 psi at 66° for 20 hours in the presence of 3.5 g of Raney nickel catalyst. The catalyst was filtered off and the filtrate stripped. The residue was recrystallized from methanol/ether to provide 99% yield of the diamine dihydrochloride salt, mp 270-273.

Example 195

2-(4-Methoxybenzoyl)benzoic acid

A mixture of 57 g (0.4 mole) of phthalic anhydride and 43 mL (0.4 mole) of anisole in 400 mL of benzene was treated with 105 g (0.8 mole) of aluminum chloride at 5°. The reaction was kept for five days at 5°, poured into 600 mL of 2N aqueous HCl and ice and filtered. The residue was triturated in aqueous sodium carbonate and filtered repeatedly until the solid no longer contained product. The sodium carbonate extracts were combined, washed with ether, and acidified with 2N aqueous HCl. The product was extracted into ether, dried over sodium sulfate and stripped. It was recrystallized from toluene to provide 80% yield of product, mp 145-147.

Example 196

2-(2-Methoxyethyl)-4-phenyl-1(2H)-phthalazinone

Eighty-five grams (0.38 mole) of 2-benzoylbenzoic acid and 28.6 g (0.38 mole) of hydroxyethylhydrazine were reacted according to the procedure of Example 185. The resulting hydroxyethyl-phthalazinone was suspended in 300 mL of DMF and 200 mL THF and 12.3 g of 60% sodium hydride in oil was added in portions over 40 minutes under nitrogen. The reaction was stirred an additional 45 minutes at room temperature and the evolution of hydrogen ceased. Thirty-one milliliters of methyl iodide was added over 1.5 hours and the reaction was stirred at gentle reflux for 16 hours. It was poured into water and extracted into ether. The ether layers were dried over sodium sulfate and stripped. The residue was chromatographed on silica with 5% ethylamine in ethyl acetate to provide 39 g (47%) of product, mp 115-118 after recrystallization from cyclohexane.

Example 197

2-Benzoyl-4,5-dimethoxybenzoic acid

Five hundred milliliters of 37% formalin solution was saturated with hydrogen chloride gas at 15-20°C and 70 g (0.38 mole) of veratric acid was added in one portion. The mixture was heated at 60-70° for seven hours and allowed to sit at room temperature for 14 hours. The solution was concentrated in vacuo, dissolved in about 300 mL of water, cooled and made basic with ammonium hydroxide. The resulting solid was collected by filtration and dried to provide a 65% yield of dimethoxyphthalide.

One hundred eight grams (0.56 mole) of the phthalide was oxidized with 258 grams (1.64 moles) of potassium permanganate according to the procedure of Example 190.

Eighty-one grams of the dimethoxyphthalic acid was converted to 72 g of the corresponding dimethoxyphthalic anhydride by heating briefly in 200 mL of acetic anhydride.

Thirty grams (0.14 mole) of 4,5-dimethoxyphthalic anhydride was suspended in 300 mL of THF and 87 mL (0.17 mole) of phenylmagnesium chloride in THF was added over two hours. The reaction was stirred at room temperature for 14 hours, refluxed for two hours, cooled and poured into saturated ammonium chloride. The mixture was made acidic with 6N HCl, extracted into chloroform, dried over magnesium sulfate, concentrated to provide 30 g of 2-benzoyl-4,5-dimethoxybenzoic acid.

Example 272

Methyl 4-(Diethylaminosulfonyl)benzenepropanoate

To 14.5 g of N,N-diethyl-4-bromobenzenesulfonamide (50 mmol) (prepared by reaction of 4-bromobenzenesulfonyl chloride with diethylamine) was added 13.8 g of tetrabutylammonium chloride (50 mmol), 10.3 g of $NaHCO_3$ (124 mmol), 266 mg of palladium (II) acetate (1.07 mmol) and 100 mL of DMF in the order given. To this suspension was added 8.8 ml of methyl acrylate (98 mmol) and the reaction was stirred 1 hour at 80°. The reaction was cooled, 500 mL of water and 900 mL of ether were added, the layers were separated and the ether layer was filtered to remove Pd (0) and combined with 2 further ether washes of the aqueous phase. The combined ether solutions were dried over $MgSO_4$, filtered, stripped and recrystallized from methanol/ether to yield 9.4 g of methyl 4-(diethylaminosulfonyl) benzene-2-propenoate. Six grams of the propenoate was reduced in ethanol at 3.5 atm over 10% Pd on carbon in a Parr Shaker to produce 5.9 g of product as a yellow oil. It was used in that form in Example 269.

Example 198

1[4-(Diethylamino)phenyl]-3-ethyl-4-methyl-1H-2,4-benzodiazepine

(Formula I: $R^1$, $R^4$, $R^6$ = H; $R^2$ = Me; $R^3$ = Et;

$R^5$ = 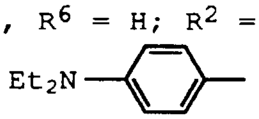

By a procedure analogous to that of Example 41, it is contemplated that 1-[4-(diethylamino)phenyl]-3-ethyl-4-methyl-1H-2,4-benzodiazepine can be synthesized from 2-[4-(diethylamino)benzoyl]benzoic acid (see U.S. Pat. 4,106,174), methylhydrazine, and triethylorthopropionate.

Example 199

3-Methyl-1-[2-[(1-oxopropyl)amino]phenyl]-4-(3-phenylpropyl)-1H-2,4-benzodiazepine

(Formula I: $R^1$, $R^4$, $R^6$ = H; $R^2$ = $(CH_2)_3Ph$; $R^3$ = Me;

$R^5$ = 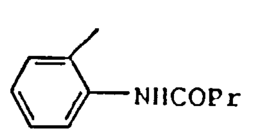

By a procedure analogous to that of Example 41, it is contemplated that 1-(2-aminophenyl)-4-(3-phenylpropyl)-3-methyl-1H-2,4-benzodiazepine can be synthesized from 2-(2-aminobenzoyl)-benzoic acid, hydrazine, bromobenzenepropane and triethyl-orthoacetate. It is further contemplated that this product may be acylated by treatment with propionic anhydride at room temperature to produce 3-methyl-1-[2-[(1-oxopropyl)amino]-phenyl]-4-(3-phenylpropyl)-1H-2,4-benzodiazepine.

Iminoethers

(alkoxyimines)

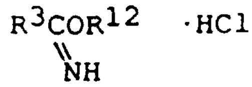

The ethoxy and methoxy imines used for condensation with the diamines were obtained from the corresponding nitriles by methods well known in the art. In general, the nitrile was dissolved in ether, 1.1 equivalents of alkanol was added, 1.1 equivalents of dry HCl gas was bubbled in, and the mixture was held at 5° for 24-48 hours; the hydrochloride salt of the iminoether was recovered by simple filtration.

The trialkylorthoesters were obtained by treating the corresponding iminoether with the appropriate alkanol under conditions known in the art.

The compounds having formulas XXXVI, III, I and XXXVII have antiarrhythmic activity as shown by the results of standard pharmacological tests carried out on representative examples as described below.

Antiarrhythmic activity was demonstrated by a procedure, which is a modification of standard programmed electrophysiological techniques utilized in large animals and in clinical studies in humans. Male Duncan-Hartley guinea pigs (600-800 grams) were anesthetized with sodium pentobarbital (30 mg/kg, i.p.) and artificially ventilated with a Harvard small-animal respirator. A left thoracotomy was performed and a fluid-filled catheter and transducer (Millar Micro-tip, Model 4F, Millar Inst. Inc., Houston, Texas) were inserted through the anterior wall of the left ventricle to monitor left ventricular pressure (LVP). The first derivative of the LVP (dP/dt) was obtained from a Grass differentiator (Model 7P20B) and used as an index of contractile function. A lead II EKG along with LVP and dP/dt were continuously recorded on a Grass polygraph (Model 7B). Rate pressure product (RPP), an index of cardiac work, was calculated using peak systolic LVP and heart rate (HR).

Effective refractory periods (ERP) were evaluated during left ventricular pacing. Grass subcutaneous electrodes were implanted as bipolar ventricular electrodes to deliver stimuli from a Bloom DTU-2 stimulator (Bloom Electronics, Inc., Reading, Pennsylvania) and stimulus isolation unit. Hearts were stimulated at the slowest frequency allowing consistent pacing (S1, 240-300 bpm) using 2 ms pulses at twice diastolic threshold. Threshold was determined by increasing the stimulation voltage until a 1:1 capture of the ventricular response with the stimulus was observed. A train of 8 normal pulses was delivered followed by a premature (S2) pulse. The interval between the last S1 and the premature S2 pulse was reduced in 10-ms increments until a ventricular response was not initiated. The longest S1-S2 interval that failed to produce a ventricular response was defined as the ERP. Pacing stimuli and the EKG were displayed at a sampling frequency of 92 Hz on an Apple IIe microcomputer using a two-channel 8-bit A/D converter (R.C. Electronics, Compu-Scope APL-D2, Santa Barbara, California).

Baseline hemodynamic function was evaluated followed by ventricular pacing to determine ERP. Pacing was discontinued prior to drug administration and resumed at set intervals during the protocol to evaluate ERP. Test compounds were administered (1 mL/kg) via the left ventricular catheter over a 15-second interval for doses less than 10 mg/kg. Higher doses (>10 mg/kg) were slowly infused over a 1-minute interval. Doses were cumulatively increased every 15 minutes until a maximally tolerated dose which reduced dP/dt by 50% was noted. Ten minutes after each dose, hemodynamics and ERP were reevaluated.

Data were analyzed using an analysis of variance for repeated measures of raw data and are expressed as means. An effective dose to increase ERP by a minimum of 20 msecs ($ED_{20}$), which was consistently a statistically significant increase, was derived for each animal from a linear regression of the data and expressed as a mean for the treated population. Biological significance was established at a probability of error less than 0.05. The results are presented in Table N.

TABLE N

| EXAMPLE | $ED_{20}$ (mg/kg) | EXAMPLE | $ED_{20}$ (mg/kg) |
|---|---|---|---|
| 1 | 0.31 | 48 | 2.20 |
| 2 | 1.17 | 49 | 1.36 |
| 4 | 0.20 | 50 | 0.50 |
| 5 | 1.0-1.2 | 51 | 0.52 |
| 7 | 0.72-0.91 | 52 | 0.18 |
| 8 | 0.4-2.5 | 53 | 0.15 |

TABLE N (continued)

| EXAMPLE | $ED_{20}$ (mg/kg) | EXAMPLE | $ED_{20}$ (mg/kg) |
|---|---|---|---|
| 8B | 0.32-0.42 | 54 | 0.23 |
| 8C | 0.31 | 55 | 0.25 |
| 8D | 0.16 | 56 | 0.09 |
| 8E | 0.93 | 57 | 0.14 |
| 9 | 0.50 | 58 | 0.10 |
| 10 | 0.44 | 59 | 0.02-0.1 |
| 11 | 0.14-0.39 | 60 | 0.13 |
| 12 | 0.08 | 61 | 0.05 |
| 13 | 0.14 | 62 | 1.4-1.74 |
| 14 | 0.13 | 63 | 0.20 |
| 16 | 0.53 | 64 | 0.39-1.2 |
| 17 | 0.52-1.29 | 65 | 0.48-0.95 |
| 18 | 0.25-0.44 | 66 | 0.40 |
| 19 | 0.57 | 67 | 0.2-0.4 |
| 20 | 0.40 | 68 | NE* |
| 21 | 0.35 | 69 | 0.54 |
| 22 | 0.23 | 70 | 0.71 |
| 23 | 0.21 | 72 | 0.57 |
| 24 | 0.15 | 74 | 0.43 |
| 25 | 0.04-0.2 | 75 | 0.41 |
| 26 | 1.97 | 76 | 0.42 |
| 27 | 0.99 | 77 | 0.25 |
| 28 | 0.65 | 78 | 0.15 |
| 29 | 0.43 | 80 | 0.04 |
| 30 | 0.28 | 81 | 0.41 |
| 31 | 4.40 | 82 | 0.29 |
| 32 | 0.28 | 84 | 0.39 |
| 34 | 0.17 | 85 | 0.93 |
| 35 | 0.11 | 86 | 0.28 |
| 37 | 1.27 | 87 | NE* |
| 39 | 0.22 | 88 | 0.08 |
| 40 | 0.10 | 89 | 0.08 |
| 41 | 0.35 | 90 | 0.47 |
| 42 | 0.34 | 91 | 0.35 |
| 43 | 0.17 | 92 | 0.22 |
| 44 | 0.24 | 93 | 0.28 |
| 45 | 0.60 | 94 | 0.56 |
| 46 | 0.60 | 95 | 0.16-0.66 |
| 47 | 0.41 | 96 | 0.76-0.83 |
| 97 | 2.49 | 165 | 0.19 |
| 98 | 0.42 | 166 | 0.36 |
| 99 | 0.80 | 167 | 0.01-0.05 |
| 100 | 0.57 | 168 | 0.02-0.15 |
| 101 | 0.32 | 169 | 0.17 |
| 102 | 0.04-0.18 | 170 | NE* |
| 103 | 0.18 | 171 | 0.03-1.0 |
| 104 | 0.27-1.87 | 176 | 0.81 |
| 105 | 0.26 | 177 | 2.63 |
| 106 | 0.30 | 179 | 1.66 |
| 107 | 0.4-0.81 | 180 | 0.18 |

TABLE N (continued)

| EXAMPLE | $ED_{20}$ (mg/kg) | EXAMPLE | $ED_{20}$ (mg/kg) |
|---|---|---|---|
| 108 | 0.16 | 181 | 0.84-5.7 |
| 109 | 2.40 | 182 | 1.14 |
| 112 | 0.38-2.32 | 183 | 5.50 |
| 113 | 0.27 | 184 | 1.14 |
| 114 | 0.53 | 200 | 0.20 |
| 115 | 0.14 | 201 | 0.06 |
| 116 | 0.10 | 202 | 0.70 |
| 117 | 0.30 | 203 | 0.08 |
| 118 | 0.15 | 204 | 0.003 |
| 119 | 0.34 | 205 | 0.10 |
| 120 | 0.88 | 206 | 0.07 |
| 121 | 0.29-0.60 | 209 | 0.16 |
| 122 | 0.08 | 210 | 0.80 |
| 123 | 0.34 | 211 | 0.04 |
| 125 | 29.9 | 212 | 0.05 |
| 129 | 2.12 | 213 | 0.20 |
| 130 | 0.66 | 214 | 0.10 |
| 131 | 0.50 | 215 | 0.03 |
| 133 | NE* | 216 | 0.06 |
| 134 | 1.00 | 217 | 0.10 |
| 143 | 1.40 | 218 | 170.00 |
| 149 | 0.23 | 219 | 0.40 |
| 150 | 1.10-1.18 | 220 | 0.40 |
| 151 | 1.70 | 223 | 0.20 |
| 153 | 0.70 | 224 | 0.40 |
| 155 | 0.18 | 225 | 0.60 |
| 156 | 2.00 | 226 | 0.10 |
| 157 | 1.7-3.2 | 227 | 0.20 |
| 159 | 4.39 | 228 | 0.50 |
| 160 | NE* | 229 | 0.07 |
| 237 | 0.20 | 262 | 0.07 |
| 238 | 0.30 | 263 | 0.16 |
| 239 | 0.08 | 266 | 0.20 |
| 240 | 0.20 | 267 | 0.10 |
| 241 | 0.04 | 268 | 0.50 |
| 243 | 0.07 | 273 | 434.00 |
| 244 | 0.28 | 280 | 0.65 |
| 249 | 0.05 | 281 | 0.20 |
| 250 | 0.10 | 282 | 15.00 |
| 251 | 0.11 | 283 | 0.10 |
| 257 | 1.00 | 284 | 1.10 |
| 259 | 0.17 | 285 | 0.10 |
| 260 | 0.19 | 286 | 0.55 |
| 261 | 0.17 | | |

The compounds of the invention can be prepared for use by conventional pharmaceutical procedures: that is, by dissolving or suspending them or their pharmaceutically acceptable salts in a pharmaceutically acceptable vehicle, e. g., water, aqueous alcohol, glycol, oil solution or oil-water emulsion, for parenteral or oral administration; or by incorporating them in unit dosage form as capsules or tablets for oral administration either alone or in combination with conventional adjuvants or excipients, e.g., calcium carbonate, starch, lactose, talc, magnesium stearate, gum acacia,

and the like.

The percentage of active component in the composition and method for treating or preventing arrhythmia can be varied so that a suitable dosage is obtained. The dosage administered to a particular patient is variable depending upon the clinician's judgement using as the criteria: the route of administration, the duration of treatment, the size and condition of the patient, the potency of the active component, and the patient's response thereto. An effective dosage amount of active component can thus be determined by the clinician considering all criteria and utilizing his best judgement on the patient's behalf.

## Claims

**Claims for the following Contracting States : AT, BE, CH, DE, DK, FR, GB, GR, IT, LI, LU, NL, SE**

1. Compounds having the formula XXXVI or acid-addition salts thereof:

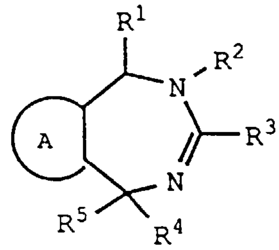

XXXVI

wherein

A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; benzyl; di(lower-alkyl)amino, pyrrolidino; piperidino; morpholino; phenyl; or phenyl substituted with amino, nitro or lower-alkyl sulfonamido;

$R^3$ is $Y_p-(CH_2)_m-X_n-R^8$ wherein

Y is -NH-, -O-, -S-, or

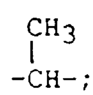

p is zero or one;
m is an integer from zero to seven;
X is -S-, -O-, $-SO_2-$,

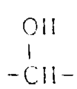 , 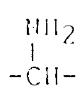 , 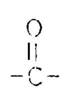 ,

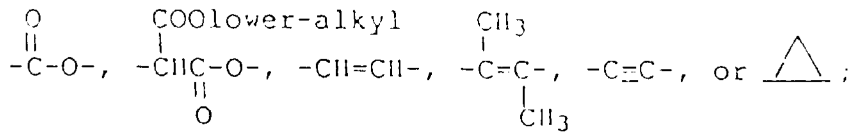

n is zero or one; and

$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heLerocycle containing one or more oxygen atoms; or when n is zero and m is other than zero, $R^8$ is additionally halogen; benzyl(lower-alkyl)- amino; di-(lower-alkyl)amino; or a 5- or 6- membered heterocycle containing one or two nitrogens, said heterocycle being unsubstituted or substituted with one lower-alkyl group; or X and $R^8$ taken together are cyclohexylidine;

$R^1$ is hydrogen; lower-alkyl; allyl; lower-alkoxylower-alkyl; acetyl; lower-alkoxy-carbonyl, lower-alkoxy-carbonyl-alkyl; or α-hydroxy-lower .-alkyl; and

$R^5$ is hydrogen; lower-alkyl; naphthyl; thienyl; pyridinyl; benzyl; phenyl; or phenyl having one or two substituents chosen independently from the group consisting of lower-alkyl,lower-alkoxy, halogen, hydroxyl, amino, di (lower-alkyl)amino, lower-alkylsulfonamido and lower-acylamino;

with the proviso that the total number of carbon atoms in $R^1$ plus $R^2$ plus $R^4$ plus $R^5$ must be five or greater, it being understood that lower-alkyl designates a linear, branched or cyclic saturated carbon chain of eight to fewer carbons and lower-alkoxy designates linear or branched alkyloxy substituents containing eight to fewer carbon atoms.

**2.** Compounds according to claim 1 having the formula

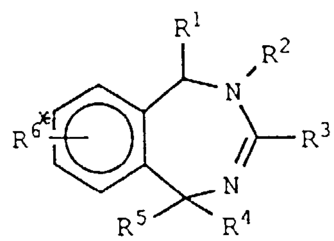

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; benzyl; di(lower-alkyl)amino, pyrrolidino; piperidino; morpholino; phenyl; phenyl substituted with amino, nitro or lower-alkyl sulfonamido;

$R^3$ is $Y_p-(CH_2)_m-X_n-R^8$ wherein

Y is -NH-, -O-, -S-, or

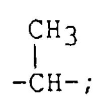

p is zero or one;

m is an integer from zero to seven;

X is -S-, -O-, , $-SO_2-$,

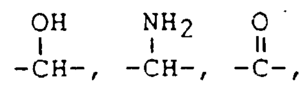

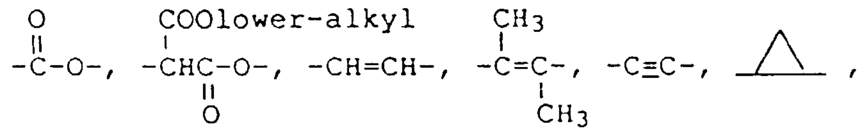

n is zero or one; and

$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)- amino; di-(lower-alkyl) amino; or a 5- or 6- membered heterocycle containing one or two nitrogens, said heterocycle being unsubstituted or substituted with one lower-alkyl group; or X and $R^8$ taken together are cyclohexylidine;

$R^4$ is hydrogen; lower-alkyl; allyl; lower-alkoxy-lower-alkyl; acetyl; lower-alkoxy-carbonyl lower-alkoxy-carbonyl-alkyl ; or α-hydroxy-lower .-alkyl; and

$R^5$ is hydrogen; lower-alkyl; naphthyl; thienyl; pyridinyl; benzyl; phenyl; or phenyl having one or two substituents chosen from the group consisting of lower-alkyl,lower-alkoxy, halogen, hydroxyl, amino, di-(lower-alkyl)amino,lower-alkylsulfonamido and lower acylamino; and

$R^{6*}$ is one or two substituents chosen from the group consisting of hydrogen, lower-alkyl, lower-alkoxy, amino, halogen, nitro and lower-alkylsulfonamido;

with the proviso that the total number of carbon atoms in $R^1$ plus $R^2$ plus $R^4$ plus $R^5$ must be five or greater.

**3.** 4,5-Dihydro-4-methyl-1-phenyl-3-(2-phenylethyl)-1H-2,4-benzodiazepine,

R-(+)-4,5-Dihydro-4-methyl-1-phenyl-3-(2-phenyléthyl)-1H-2,4-benzodiazepine,
N-[4-[2-(4,5-Dihydro-4-methyl-1-phenyl-1H-2,4-benzodiazepin-3-yl)ethyl]phenyl]methanesulfonamide,
N-[4-[2-(4,5-Dihydro-3-ethyl-1-phenyl-1H-2,4-benzodiazepin-4-yl)ethyl]phenyl]methanesulfonamide,
N-[4,5-Dihydro-4-methyl-1-phenyl-3-(2-phenylethyl)-1H-2,4-benzodiazepin-8-yl]methanesulfonamide,
1-(4-Chlorophenyl)-4,5-dihydro-4-methyl-3-(2-phenylethyl)-1H-2,4-benzodiazepine,
3-[2-(4-Chlorophenyl)ethyl]-4,5-dihydro-4-methyl-1-phenyl-1H-2,4-benzodiazepine,
3-[2-(4-Chlorophenyl)ethyl]-4,5-dihydro-1,4-dimethyl-1-phenyl-1H-2,4-benzodiazepine,
N,N-Diethyl-4-[4,5-dihydro-5-methyl-1-phenyl-1H-2,4-benodiazepin-4-yl]benzenesulfonamide
4,5-Dihydro-1-(4-hydroxyphenyl)-4-methyl-3- (2-phenylethyl)-1H-2,4-benzodiazepine,
4,5-Dihydro-3-[2-(4-hydroxyphenyl)ethyl]-4-methyl-1-phenyl-1H-2,4-benzodiazepine or acid-additional salts thereof according to claim 2.

**4.** A compound according to claim 1 of the formula :

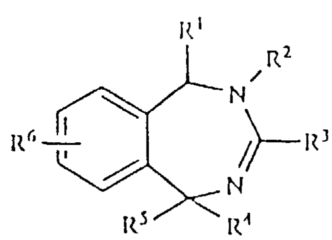

I

wherein

$R^1$ is hydrogen, lower-alkyl, phenyl, phenyl having one or two substituents chosen from the group consisting of lower-alkyl and lower-alkoxy, naphthyl, thienyl, pyridinyl, or benzyl;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; phenyl, benzyl, di-(lower-alkyl)amino, pyrrolidino, piperidino or morpholino;

$R^3$ is $Y_p-(CH_2)_m-X_n-R^8$ wherein

Y is -NH-, -O-, -S-, or

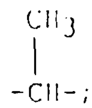;

p is zero or one;

m is an integer from zero to seven;

X is -S-, -O-, $-SO_2-$,

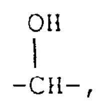, 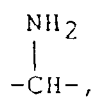, 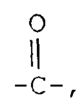,

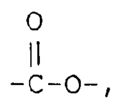, 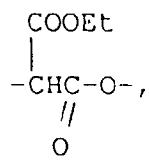,

or -CH=CH-

n is zero or one; and

$R^8$ is lower-alkyl; phenyl; phenyl having one or two substituents chosen from the group consisting of halogen, lower-alkyl, and lower-alkoxy; or when n is zero and m is other than zero, $R^8$ is additionally hydrogen; halogen; benzyl(lower-alkyl)-amino; di-(lower-alkyl)amino; or a 5- or 6-membered heterocycle containing one or two nitrogens, said heterocycle being unsubstituted or substituted with one lower-alkyl group;

$R^4$ is hydrogen; lower-alkyl; allyl; lower-alkoxylower-alkyl; acetyl; lower-alkoxycarbonyl-alkyl; lower alkyl carboxyl; or α-hydroxy-lower-alkyl;

$R^5$ is hydrogen; lower-alkyl; phenyl; phenyl having one or two substituents chosen from the group consisting of lower-alkyl,lower-alkoxy, halogen, amino, di-(loweralkyl)amino, and lower acylamino; naphthyl; thienyl; pyridinyl; or benzyl; and

$R^6$ is one or two substituents chosen from the group consisting of hydrogen, lower-alkyl, lower-alkoxy, and halogen;

with the proviso that the total number of carbon atoms in $R^1$ plus $R^2$ plus $R^4$ plus $R^5$ must be five or greater.

**5.** A process for producing a compound according to claim 1 of formula (V)

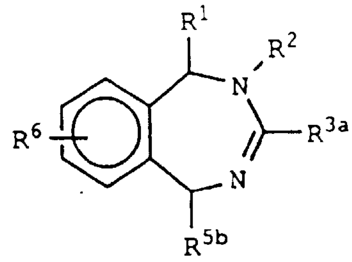

V

which comprises a) reacting a compound of formula VI

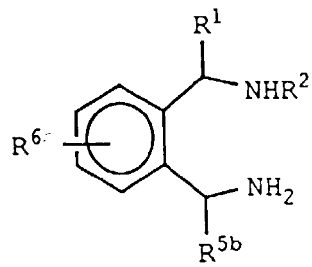

VI d

wherein

$R^1$ is hydrogen, lower-alkyl, benzyl, naphthyl, thienyl pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy, benzyl, di-(lower-alkyl)amino, pyrrolidino, piperidino morpholino, phenyl, or phenyl substituted with amino, nitro or lower-alkylsulfonamido;

$R^{3a}$ is $(Y^a)_p-(CH_2)_m-(X^a)_n-R^8$ wherein

$Y^a$ is -O-, -S- or

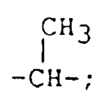

p is zero or one;

m is an integer from zero to seven;

$X^a$ is -S-, $-SO_2-$, -O-, or -CH=CH-;

n is zero or one; and

$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)- amino; di-(lower-alkyl)amino; or a 5- or 6- membered heterocycle containing one or two nitrogens, said heterocycle being unsubstituted or substituted with one lower-alkyl group; or X and $R^8$ taken together are cyclohexylidine;

$R^{5b}$ is hydrogen; lower-alkyl; phenyl; phenyl having one or two substituents chosen from the group consisting of lower-alkyl, lower-alkoxy, and halogen; naphthyl; thienyl; pyridinyl; or benzyl;

$R^6$ is one or two substituents chosen independently from the group consisting of hydrogen, lower-alkyl, lower-alkoxy, halogen, nitro, and loweralkylsulfonamido;

1) with an iminoether, or a salt thereof, of formula

$$R^{3a}C(OR^{12})NH$$

wherein $R^{12}$ is methyl or ethyl;

2) with an orthoester of formula $R^{3a}C(OR^{12})_3$

3) with an ester of formula $R^{3a}COOR^{12}$ in the presence of trialkylaluminum or

b) reacting a compound of formula XXVII or XXVIII

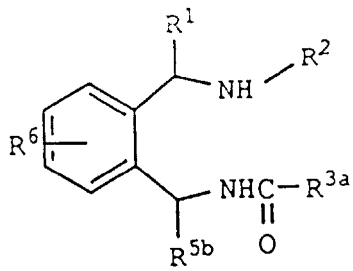

XXVII

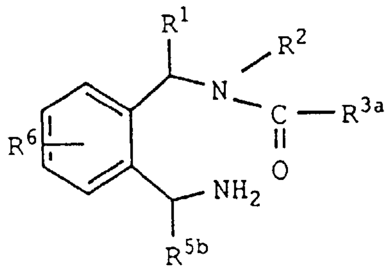

XXVIII

with a trialkylaluminum.

**6.** A process for preparing a compound according to claim 1 of formula Ia

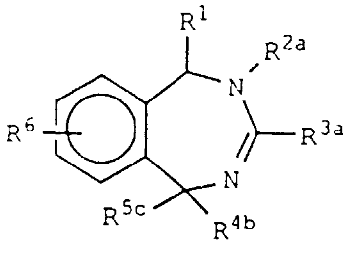

Ia

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^{2a}$ is lower-alkyl; benzyl; phenyl; phenyl substituted with halogen, lower-alkyl or lower-alkoxy; or

$R^{2a}$ is $-CH_2CH_2R^7$ where $R^7$ is lower-alkoxy, phenyl, benzyl, di-(lower-alkyl)amino, pyrrolidino, piperidino, or morpholino;

$R^{3a}$ is $(Y^a)_p-(CH_2)_m-(X^a)_n-R^8$ wherein

$Y^a$ is -O-, -S- or

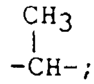

p is zero or one;

m is an integer from zero to seven;

$X^a$ is -S-, $-SO_2-$, -O- or -CH=CH-;

n is zero or one; and

$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)amino; di(lower-alkyl)amino; or

$R^8$ is a 5- or 6-membered heterocycle containing one or two nitrogens, said heterocycle being unsubstituted or substituted with one lower-alkyl group; or X and R8 taken together are cyclohexylidine;

$R^{4b}$ is lower-alkyl; allyl; lower-alkoxy-lower-alkyl; acetyl; lower-alkoxy-carbonyl; lower-alkyl-carboxy-alkyl; or α-hydroxy-lower-alkyl;

$R^{5c}$ is phenyl; phenyl having one or two substituents chosen from the group consisting of lower-alkyl, lower-alkoxy, and halogen; naphthyl; thienyl; or pyridinyl; and

$R^6$ is one or two substituents chosen independently from the group consisting of hydrogen, lower-alkyl, lower-alkoxy, halogen, nitro and lower-alkylsulfonamido ;

which comprises reacting a compound of formula Va

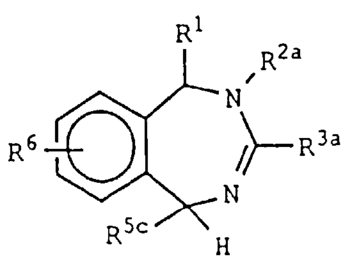

Va

with a strong base and reacting with a) a lower-alkyl aldehyde or b) a compound of formula $R^{4b}$ Z wherein Z is a group subject to nucleophilic displacement.

7. A process for preparing a compound according to claim 1 of formula VII

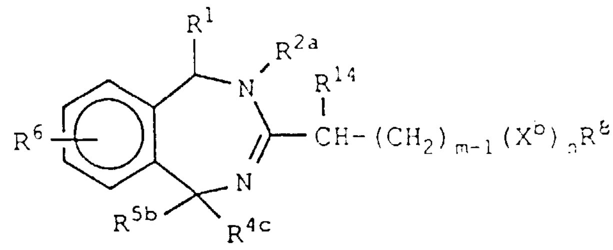

VII

wherein

- $R^1$ is hydrogen, lower-alkyl, benzyl, naphthyl, thienyl, pyridinyl, phenyl, or phenyl having one or two substituents chosen from the group consisting of lower-alkyl and lower-alkoxy;
- $R^{2a}$ is lower-alkyl; benzyl; phenyl; phenyl substituted with halogen, lower-alkyl or lower- alkoxy; or
- $R^{2a}$ is $-CH_2CH_2R^7$ where $R^7$ is lower-alkoxy, phenyl, benzyl, di-(lower-alkyl)amino, pyrrolidino, piperidino, or morpholino;
- m is an integer from zero to seven;
- $X^b$ is -S-, $-SO_2-$, -O-, -CH=CH-,

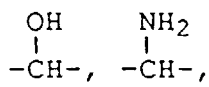

or

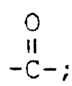

- n is zero or one;
- $R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)amino; di(lower-alkyl)amino; or
  $R^8$ is a 5- or 6-membered heterocycle containing one or two nitrogens, said heterocycle being unsubstituted or substituted with one lower-alkyl group; or X and $R^8$ taken together are cyclohexylidine;
- $R^{4c}$ is lower-alkyl , allyl, or lower-alkoxylower-alkyl;
- $R^{5c}$ is phenyl; naphthyl; thienyl; pyridinyl; or phenyl having one or two substituents chosen from the group consisting of lower-alkyl, lower-alkoxy, and halogen;
- $R^6$ is one or two substituents chosen independently from the group consisting of hydrogen, lower-alkyl, lower-alkoxy, halogen; nitro and lower-alkylsulfonamido ;
  and
- $R^{14}$ is hydrogen or methyl,

which comprises reacting a compound of formula VIIa

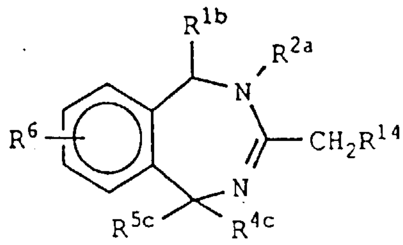

VIIa

with a strong base followed by reaction with an electrophile chosen from the group consisting of $R^8COOR^{12}$, $R^8CHNR^{12}$, $R^8CHO$, $R^8SSR^8$, $R^8(X^b)_n(CH_2)_{m-1}Z$, wherein $R^{12}$ is methyl or ethyl and Z is a group subject to nucleophilic displacement.

**8.** The use of a compound as claimed in claims 1 to 4 for the preparation of a medicament for the treatment of cardiac arrhythmia in a patient in need of such treatment.

**9.** A composition for the treatment of cardiac arrhythmia comprising an antiarrhythmically effective amount of a compound according to any one of claims 1 to 4.

**10.** Compound according to claim 1 in which A is naphthyl.

**Claims for the following Contracting State : ES**

**1.** A process for producing a compound of formula V

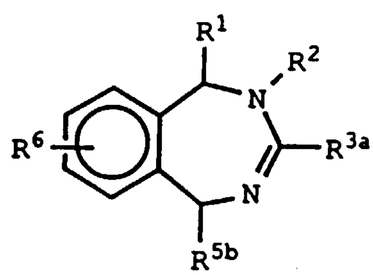

**V**

which comprises a) reacting a compound of formula VId

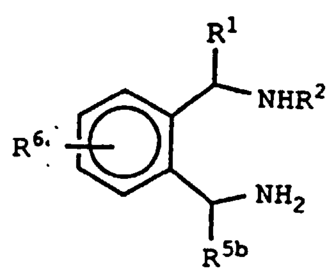

VId

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy, benzyl, di-(lower-alkyl)amino, pyrrolidino, piperidino morpholino, phenyl, or phenyl substituted with amino, nitro or lower-alkylsulfonamido;

$R^{3a}$ is $(Y^a)_p-(CH_2)_m-(X^a)_n-R^8$ wherein

$Y^a$ is -O-, -S- or

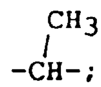

p is zero or one;
m is an integer from zero to seven;
$X^a$ is -S-, $-SO_2-$, -O-, or -CH=CH-;
n is zero or one; and
$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido, dilower-alkylaminosulfonyl, and amino; or with a 5-membered heterocycle containing one or more oxygen atoms; or when n is zero and m is other than zero, $R^8$ is additionally halogen; benzyl(lower-alkyl)- amino; di-(lower-alkyl)amino; or $R^8$ is a 5- or 6- membered heterocycle containing one or two nitrogens, said heterocycle being unsubstituted or substituted with one lower-alkyl group; or X and $R^8$ taken together are cyclohexylidine;

$R^{5b'}$ is hydrogen; lower-alkyl; phenyl; phenyl having one or two substituents chosen from the group consisting of lower-alkyl, lower-alkoxy, and halogen; naphthyl; thienyl; pyridinyl; or benzyl;

$R^6$ is one or two substituents chosen independently from the group consisting of hydrogen, lower-alkyl, lower-alkoxy, halogen, nitro, and lower-alkylsulfonamido;
with the proviso that the total number of carbon atoms in $R^1$ plus
$R^2$ plus $R^{5b}$ must be five or greater, it being understood that lower alkyl designates a linear, branched or cyclic saturated carbon chain of eight to fewer carbon atoms and lower alkoxy designates lower or branched alkyloxy substituents containing eight to fewer carbon atoms;

1) with an iminoether, or a salt thereof, of formula $R^{3a}C(OR^{12})NH$ wherein $R^{12}$ is methyl or ethyl;
2) with an orthoester of formula $R^{3a}C(OR^{12})_3$
3) with an ester of formula $R^{3a}COOR^{12}$ in the presence of trialkylaluminium or
b) reacting a compound of formula XXVII or XXVIII

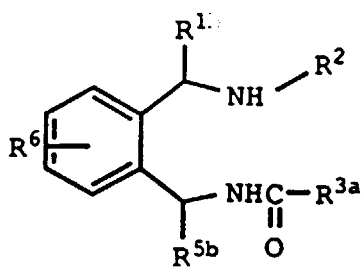

XXVII

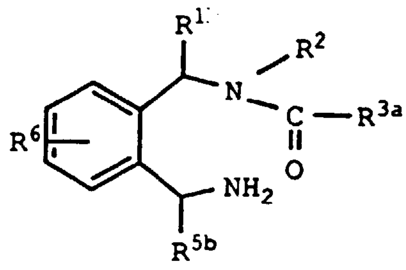

XXVIII

with a trialkylaluminum

if desired, reducing a compound obtained where $R^6$ is nitro or $R^8$ is nitrophenyl to prepare the corresponding compound where $R^6$ is amino or $R^8$ is aminophenyl,
if desired, sulfonylating a compound obtained where $R^6$ is amino or $R^8$ is aminophenyl with a lower-alkylsulfonyl halide to prepare the corresponding compound where $R^6$ is lower-alkylsulfonamido or $R^8$ is lower-alkylsulfonamidophenyl,
if desired, acylating a compound obtained where $R^8$ is aminophenyl to prepare the corresponding compound where $R^8$ is lower-alkylamidophenyl,
if desired, cleaving a compound obtained where $R^8$ is lower-alkoxyphenyl to prepare the corresponding compound where $R^8$ is hydroxyphenyl,
if desired, resolving a mixture of enantiomers obtained where $R^1$ or $R^{5b}$ is other than hydrogen to prepare a corresponding mixture enriched to greater than 90% of a single enantiomer, and
if desired, converting a free base obtained to an acid-addition salt thereof.

2. A process according to claim 1 wherein $R^1$ is hydrogen, $R^2$ is lower-alkyl, $R^{5b}$ is phenyl, naphthyl, thienyl, pyridinyl, benzyl or phenyl having one or two substituents chosen independently from the group consisting of lower-alkyl, lower-alkoxy and halogen, $R^6$ is hydrogen, lower-alkoxy, nitro or lower-alkylsulfonamido, and in $R^{3a}$ p is zero, m is zero, one or two, $R^8$ is phenyl, phenyl having one or two substituents chosen from the group consisting of halogen, lower-alkyl, nitro, hydroxy, lower-alkoxy, lower-alkylsulfonamido and di(lower-alkyl)aminosulfonyl, or $R^8$ is a 5- or 6-membered heterocycle containing one or two nitrogens, said heterocycle being unsubstituted or substituted with one lower-alkyl group.

3. A process according to claim 2 wherein $R^2$ is methyl, $R^{5b}$ is phenyl or substituted phenyl, $R^6$ is hydrogen and in $R^{3a}$ m is zero or two, $X^a$ is -CH=CH- and $R^8$ is phenyl or substituted phenyl.

4. A process according to claim 3 wherein $R^{5b}$ is phenyl and $R^{3a}$ is

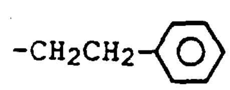

5. A process for preparing a compound of formula Ia

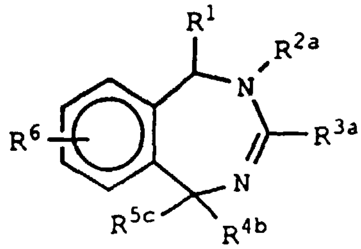

Ia

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^{2a}$ is lower-alkyl; benzyl; phenyl; phenyl substituted with halogen, lower-alkyl or lower-alkoxy; or

$R^{2a}$ is $-CH_2CH_2R^7$ where $R^7$ is lower-alkoxy, phenyl, benzyl, di-(lower-alkyl)amino, pyrrolidino, piperidino, or morpholino;

$R^{3a}$ is $(Y^a)_p-(CH_2)_m-(X^a)_n-R^8$ wherein

$Y^a$ is -O-, -S- or

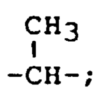

p is zero or one;
m is an integer from zero to seven;
$X^a$ is -S-, $-SO_2-$, -O- or -CH=CH-;
n is zero or one; and
$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)-amino; di-(lower-alkyl) amino; or $R_8$ is a 5- or 6- membered heterocycle containing one or two nitrogens, said heterocycle being unsubstituted or substituted with one lower-alkyl group; or X and $R^8$ taken together are cyclohexylidine;

$R^{4b}$ is lower-alkyl; allyl; lower-alkoxy-lower-alkyl; acetyl ; lower-alkoxy-carbonyl, lower-alkoxy-carbonyl -alkyl; or α-hydroxy-lower .-alkyl; and

$R^{5c}$ is phenyl; phenyl having one or two substituents chosen from the group consisting of lower-alkyl, lower-alkoxy, and halogen; naphthyl; thienyl; or pyridinyl; and

$R^6$ is one or two substituents chosen independently from the group consisting of hydrogen, lower-alkyl, lower-alkoxy, halogen, nitro and lower-alkylsulfonamido with the proviso that the total number of carbon atoms in $R^1$ plus $R^{2a}$ plus $R^{4b}$ plus $R^{5c}$ is five or greater, it being understood that lower alkyl designates a linear, branched or cyclic saturated carbon chain of eight to fewer carbon atoms and lower alkoxy designates lower or branched alkyloxy substituents containing eight to fewer carbon atoms; which comprises reacting a compound of formula Va

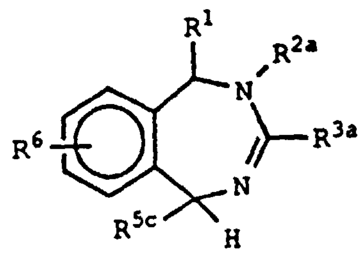

Va

with a strong base and reacting with a) a lower-alkyl aldehyde or b) a compound of formula $R^{4b}$ Z wherein Z is a group subject to nucleophilic displacement

if desired, reducing a compound obtained where $R^6$ is nitro or $R^8$ is nitrophenyl to prepare the corresponding compound where $R^6$ is amino or $R^8$ is aminophenyl,
if desired, sulfonylating a compound obtained where $R^6$ is amino or $R^8$ is aminophenyl with a lower-alkylsulfonyl halide to prepare the corresponding compound where $R^6$ is lower-alkylsulfonamido or $R^8$ is lower-alkylsulfonamidophenyl,
if desired, acylating a compound obtained where $R^8$ is aminophenyl to prepare the corresponding compound where $R^8$ is lower-alkylamidophenyl,
if desired, cleaving a compound obtained where $R^8$ is lower-alkoxyphenyl to prepare the corresponding compound where $R^8$ is hydroxyphenyl,
if desired, resolving a mixture of enantiomers obtained where $R^1$ is other than hydrogen or $R^{5b}$ is different from $R^{4b}$ to prepare a corresponding mixture enriched to greater than 90% of a single enantiomer, and
if desired, converting a free base obtained to an acid-addition salt thereof.

**6.** A process according to claim 5 wherein $R^1$ is hydrogen, $R^{2a}$ is lower-alkyl, $R^{4b}$ is lower-alkyl, $R^{5c}$ is phenyl, naphthyl, thienyl, pyridinyl, benzyl or phenyl having one or two substituents chosen independently from the group consisting of lower-alkyl, lower-alkoxy and halogen, $R^6$ is hydrogen, lower-alkoxy, nitro or lower-alkylsulfonamido, and in $R^{3a}$ p is zero, m is zero, one or two, $R^8$ is phenyl, phenyl having one or two substituents chosen from the group consisting of halogen, lower-alkyl, nitro, hydroxy, lower-alkoxy, lower-alkylsulfonamido and di(lower-alkyl)aminosulfonyl, or $R^8$ is a 5- or 6-membered heterocycle containing one or two nitrogens, said heterocycle being unsubstituted or substituted with one lower-alkyl group.

**7.** A process according to claim 6 wherein $R^{2a}$ is methyl, $R^{4b}$ is methyl, $R^{5c}$ is phenyl or substituted phenyl, $R^6$ is hydrogen and in $R^{3a}$ m is zero or two, $X^a$ is -CH=CH- and $R^8$ is phenyl or substituted phenyl.

**8.** A process for preparing a compound of formula VII

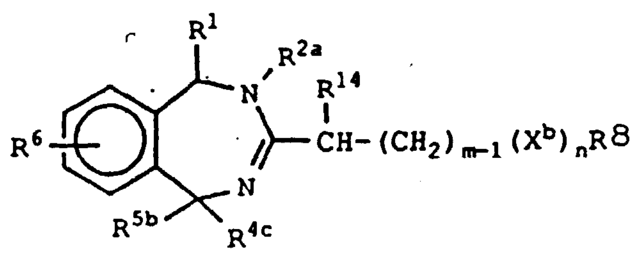

VII

wherein

$R^1$ is hydrogen, lower-alkyl, benzyl, naphthyl, thienyl, pyridinyl, phenyl, or phenyl having one or two substituents chosen from the group consisting of lower-alkyl and lower-alkoxy;
$R^{2a}$ is lower-alkyl; benzyl; phenyl; phenyl substituted with halogen, lower-alkyl or lower-alkoxy; or

$R^{2a}$ is $-CH_2CH_2R^7$ where $R^7$ is lower-alkoxy, phenyl, benzyl, di-(lower-alkyl)amino, pyrrolidino, piperidino, or morpholino;

m is an integer from zero to seven;

$X^b$ is -S-, $-SO_2-$, -O-, -CH=CH-,

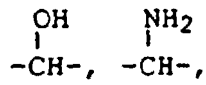

or

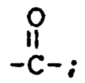

n is zero or one;

$R^8$ is hydrogen;lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)amino; amino; di-(lower-alkyl)amino; or $R^8$ is a 5- or 6-membered heterocycle containing one or two nitrogens, said heterocycle being unsubstituted or substituted with one lower-alkyl group; or X and $R^8$ taken together are cyclohexylidine;

$R^{4c}$ is lower-alkyl, allyl, or lower-alkoxylower-alkyl;

$R^{5c}$ is phenyl; naphthyl; thienyl; pyridinyl; or phenyl having one or two substituents chosen from the group consisting of lower-alkyl, lower-alkoxy, and halogen;

$R^6$ is one or two substituents chosen independently from the group consisting of hydrogen, lower-alkyl, lower-alkoxy, halogen; nitro and lower-alkylsulfonamido; and

$R^{14}$ is hydrogen or methyl,
with the proviso that the total number of carbon atoms of $R^1$ plus $R^{2a}$ plus $4^{4c}$ plus $R^{5b}$ must be five or greater, it being understood that lower alkyl designates a linear, branched or cyclic saturated carbon chain of eight to fewer carbon atoms and lower alkoxy designates lower or branched alkyloxy substituents containing eight to fewer carbon atoms;

which comprises reacting a compound of formula VIIa

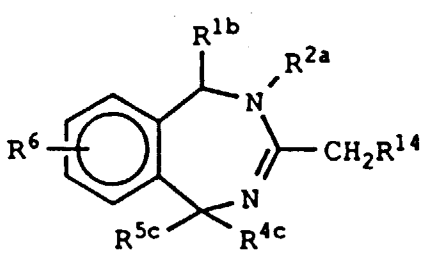

VIIa

with a strong base followed by reaction with an electrophile chosen from the group consisting of $R^8COOR^{12}$, $R^8CHNR^{12}$, $R^8CHO$, $R^8SSR^8$, $R^8(X^b)_n(CH_2)_{m-1}Z$, wherein $R^{12}$ is methyl or ethyl and Z is a group subject to nucleophilic displacement

if desired, reducing a compound obtained where $R^6$ is nitro or $R^8$ is nitrophenyl to prepare the corresponding compound where $R^6$ is amino or $R^8$ is aminophenyl,

if desired, sulfonylating a compound obtained where $R^6$ is amino or $R^8$ is aminophenyl with a lower-alkylsulfonyl

halide to prepare the corresponding compound where $R^6$ is lower-alkylsulfonamido or $R^8$ is lower-alkylsulfonamidophenyl,
if desired, acylating a compound obtained where $R^8$ is aminophenyl to prepare the corresponding compound where $R^8$ is lower-alkylamidophenyl,
if desired, cleaving. a compound obtained where $R^8$ is lower-alkoxyphenyl to prepare the corresponding compound where $R^8$ is hydroxyphenyl,
if desired, resolving a mixture of enantiomers obtained where $R^1$ or $R^{5b}$ is other than hydrogen to prepare a corresponding mixture enriched to greater than 90% of a single enantiomer, and
if desired, converting a free base obtained to an acid-addition salt thereof.

**9.** A process for preparing a compound of formula XXXX

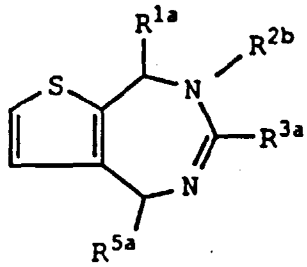

XXXX

wherein

$R^{1a}$ is hydrogen, lower-alkyl or phenyl;
$R^{2b}$ is hydrogen; lower-alkyl; di(lower-alkyl)aminoethyl; benzyl; phenyl; phenethyl or phenyl substituted with halogen, lower-alkyl or lower-alkoxy;
$R^{3a}$ is $(Y^a)_p$-$(CH_2)_m$-$(X^a)_n$-$R^8$ wherein

$Y^a$ is -O-, -S- or

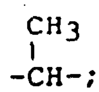

p is zero or one;
m is an integer from zero to seven;
$X^a$ is -S-, -$SO_2$-, -O-, or -CH=CH-;
n is zero or one; and
$R^8$ is hydrogen; lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)- amino; di-(lower-alkyl)amino; or a 5- or 6- membered heterocycle containing one or two nitrogens, said heterocycle being unsubstituted or substituted with one lower-alkyl group; or Xa and $R^8$ taken together are cyclohexylidine;

$R^{5a}$ is hydrogen; phenyl; phenyl having one or two substituents chosen from the group consisting of halogen, lower-alkyl and lower-alkoxy; naphthyl; thienyl; pyridinyl or benzyl;
with the proviso that the total number of carbon atoms in $R^{1a}$ plus $R^{2b}$ plus $R^{5a}$ must be five or greater, it being understood that lower alkyl designates a linear, branched or cyclic saturated carbon chain of eight to fewer carbon atoms and lower alkoxy designates lower or branched alkyloxy substituents containing eight to fewer carbon atoms;

which comprises reacting a compound of formula XXXXI

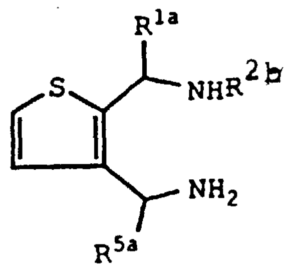

XXXXI

1) with an iminoether, or a salt thereof, of formula $R^{3a}C(OR^{12})NH$, wherein $R^{12}$ is methyl or ethyl,
2) with an orthoester of formula $R^{3a}C(OR^{12})_3$, or
3) with an ester of formula $R^{3a}COOR^{12}$ in the presence of trialkylaluminum

and, if desired, converting a free base obtained to an acid-addition salt thereof.

**10.** A process according to claim 1 for producting a compound of formula V

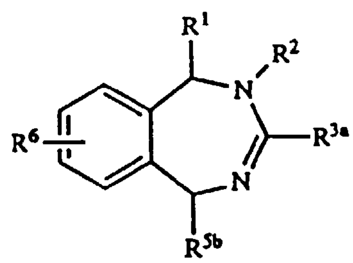

V

which comprises a) reacting a compound of formula VId

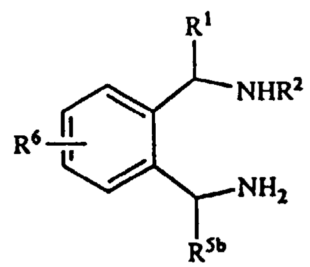

wherein

$R^1$ is hydrogen, lower-alkyl, phenyl; phenyl having one or two substituents chosen from the group consisting of lower-alkyl and lower-alkoxy, naphthyl, thienyl, pyridinyl, or benzyl;
$R^2$ is hydrogen; lower-alkyl; benzyl; phenyl; phenyl substituted with halogen, lower-alkyl or lower-alkoxy; or
$R^2$ is $-CH_2CH_2R^7$ where $R^7$ is lower-alkoxy, phenyl, benzyl, di-(lower-alkyl)amino, pyrrolidino, piperidino or morpholino;
$R^{3a}$ is $(Y^a)_p-(CH_2)_m-(X^a)_n-R^8$ wherein

$Y^a$ is -O-, -S- or

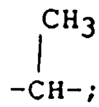

p is zero or one;
m is an integer from zero to seven;
$X^a$ is -S-, $-SO_2-$, -O-, or -CH=CH-;
n is zero or one; and
$R^8$ is lower-alkyl; phenyl; phenyl having one or two substituents chosen from the group consisting of halogen, lower-alkyl, and lower-alkoxy; or when n is zero and m is other than zero, $R^8$ is additionally hydrogen; halogen; benzyl(lower-alkyl)amino; di(lower-alkyl)amino; or
$R^8$ is a 5- or 6-membered heterocycle containing one or two nitrogens, said heterocycle being unsubstituted or substituted with one lower-alkyl group;

$R^{5b}$ is hydrogen; lower-alkyl; phenyl; phenyl having one or two substituents chosen from the group consisting of lower-alkyl, lower-alkoxy, and halogen; naphthyl; thienyl; pyridinyl; or benzyl;
$R^6$ is one or two substituents chosen from the group consisting of hydrogen, lower-alkyl, lower-alkoxy, and halogen;
with the proviso that the total number of carbon atoms in $R^1$ plus $R^2$ plus $R^{5b}$ must be five or greater;

with a compound selected from the group consisting of

1) an iminoether, or a salt thereof, of formula

$$R^{3a}C(OR^{12})NH$$

wherein $R^{12}$ is methyl or ethyl
2) an orthoester of formula $R^{3a}C(OR^{12})_3$,
or 3) an ester of formula $R^{3a}COOR^{12}$ in the presence of trialkylaluminium or

which comprises b) reacting a compound of formula XXVII or XXVIII

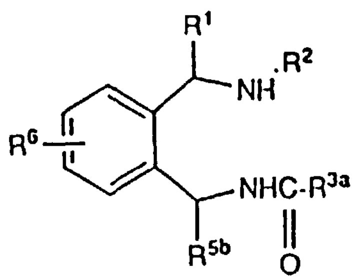

XXVII

or

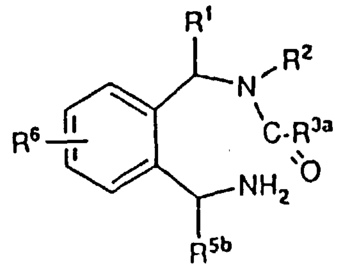

XXVIII

with a trialkylaluminum.

**11.** A process according to claim 5 for preparing a compound of formula Ia

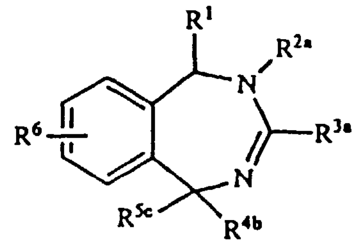

**Ia**

wherein

$R^1$ is hydrogen, lower-alkyl, phenyl; phenyl having one or two substituents chosen from the group consisting of lower-alkyl and lower-alkoxy, naphthyl, thienyl, pyridinyl, or benzyl;

$R^{2a}$ is lower-alkyl; benzyl; phenyl; phenyl substituted with halogen, lower-alkyl or lower-alkoxy; or

$R^{2a}$ is $-CH_2CH_2R^7$ where $R^7$ is lower-alkoxy, phenyl, benzyl, di-(lower-alkyl)amino, pyrrolidino, piperidino, or morpholino;

$R^{3a}$ is $(Y^a)_p-(CH_2)_m-(X^a)_n-R^8$ wherein

$Y^a$ is -O-, -S- or

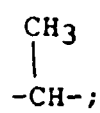

p is zero or one;
m is an integer from zero to seven;
$X^a$ is -S-, $-SO_2-$, -O- or -CH=CH-;
n is zero or one; and
$R^8$ is lower-alkyl; phenyl; phenyl having one or two substituents chosen from the group consisting of halogen, lower-alkyl, and lower-alkoxy; or when n is zero and m is other than zero, $R^8$ is additionally hydrogen; halogen; benzyl(lower-alkyl) amino; di(lower- alkyl)amino; or
$R^8$ is a 5- or 6-membered heterocycle containing one or two nitrogens, said heterocycle being unsubstituted or substituted with one lower-alkyl group;

$R^{4b}$ is lower-alkyl , allyl, lower-alkoxy-lower-alkyl, acetyl, lower-alkoxycarbonyl, lower-alkoxycarbonylalkyl , or α-hydroxylower-alkyl;

$R^{5c}$ is phenyl; phenyl having one or two substituents chosen from the group consisting of lower-alkyl, lower-alkoxy, and halogen; naphthyl; thienyl; or pyridinyl; and

$R^6$ is one or two substituents chosen from the group consisting of hydrogen, lower-alkyl, lower-alkoxy, and halogen;

with the proviso that the total number of carbon atoms in $R^1$ plus $R^{2a}$ plus $R^{4b}$ plus $R^{5c}$ must be five or greater; which comprises reacting a compound of formula Va

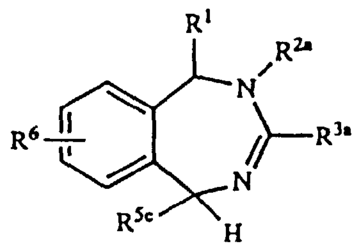

Va

with a strong base and reacting with a suitable electrophile.

**12.** A process according to claim 8 for preparing a compound of formula VII

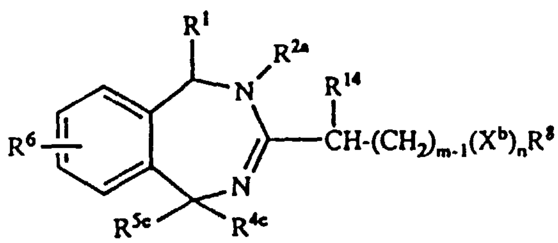

.

VII

wherein

$R^1$ is hydrogen; lower-alkyl; phenyl; phenyl having one or two substituents chosen from the group consisting of lower-alkyl and lower-alkoxy; naphthyl; thienyl; pyridinyl; m or benzyl;

$R^{2a}$ is lower-alkyl; benzyl; phenyl; phenyl substituted with halogen, lower-alkyl or lower-alkyoxy; or

$R^{2a}$ is $-CH_2CH_2R^7$ where $R^7$ is lower-alkoxy, phenyl, benzyl, di-(lower-alkyl)amino, pyrrolidino, piperidino, or morpholino;

m is an integer from zero to seven;

$X^b$ is -S-, $-SO_2-$, -O-, -CH=CH-,

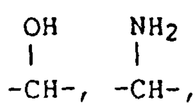

or

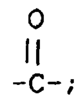

n is zero or one;

$R^8$ is lower-alkyl; phenyl; phenyl having one or two substituents chosen from the group consisting of halogen, lower-alkyl, and lower-alkoxy; or when n is zero and m is other than zero, $R^8$ is additionally hydrogen; halogen; benzyl(lower-alkyl)amino; di(lower-alkyl)amino; or $R^8$ is a 5- or 6-membered heterocycle containing one or two nitrogens, said heterocycle being unsubstituted or substituted with one lower-alkyl group;

$R^{4c}$ is lower-alkyl , allyl, or lower-alkoxylower-alkyl;

$R^{5c}$ is phenyl; phenyl having one or two substituents chosen from the group consisting of lower-alkyl, lower-alkoxy, and halogen; naphthyl; thienyl; or pyridinyl;

$R^6$ is one or two substituents chosen from the group consisting of hydrogen, lower-alkyl, lower-alkoxy, and halogen;
and

$R^{14}$ is hydrogen or methyl,

with the proviso that the total number of carbon atoms in $R^1$ plus $R^{2a}$ plus $R^{5c}$ must be five or greater, which comprises reacting a compound of formula VIIa

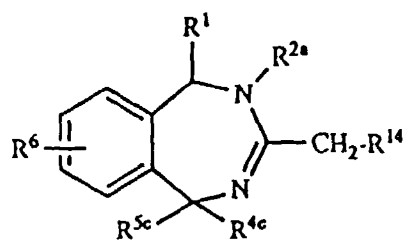

VIIa

with a strong base followed by reaction with an electrophile chosen from the group consisting of $R^8COOR^{12}$, $R^8CHNR^{12}$, $R^8CHO$, $R^8SSR^8$, $R^8(X^b)_n(CH_2)_{m-1}Z$, wherein $R^{12}$ is methyl or ethyl and Z is a group subject to nucleophilic displacement.

**13.** A process for the preparation of a compound of general formula XXXVI

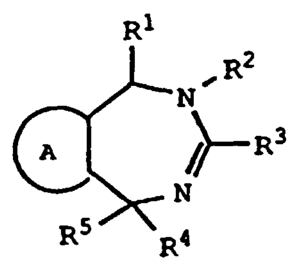

XXXVI

wherein

A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; benzyl; di(lower-alkyl)amino, pyrrolidino; piperidino; morpholino; phenyl; or phenyl substituted with amino, nitro or lower-alkyl sulfonamido;

$R^3$ is $Y_p-(CH_2)_m-X_n-R^8$ wherein

Y is -NH-, -O-, -S-, or

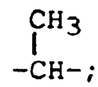

p is zero or one;
m is an integer from zero to seven;
X is -S-, -O-, $-SO_2-$,

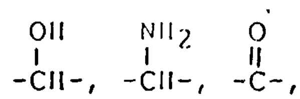

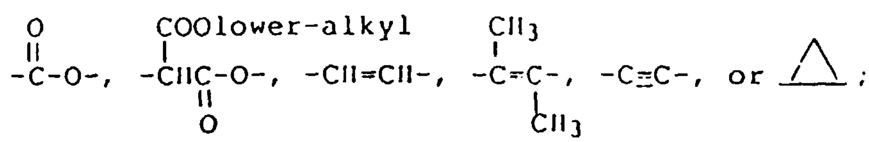

n is zero or one; and
$R^8$ is hydrogen;lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)- amino; di-(lower-alkyl)amino; or a 5- or 6- membered heterocycle containing one or two nitrogens, said heterocycle being unsubstituted or substituted with one lower-alkyl group; or X and $R^8$ taken together are cycloliexylidine;

$R^4$ is hydrogen; lower-alkyl; allyl; lower-alkoxylower-alkyl; acetyl; lower-alkoxy-carbonyl, lower-alkoxy-carbonyl-alkyl; or α-hydroxy-lower .-alkyl; and

$R^5$ is hydrogen; lower-alkyl; naphthyl; thienyl; pyridinyl; benzyl; phenyl; or phenyl having one or two substituents chosen independently from the group consisting of lower-alkyl,lower-alkoxy, halogen, hydroxyl, amino, di (lower-alkyl)amino, lower-alkylsulfonamido and lower-acylamino;

with the proviso that the total number of carbon atoms in $R^1$ plus $R^2$ plus $R^4$ plus $R^5$ must be five or greater,

it being understood that lower-alkyl designates a linear, branched or cyclic saturated carbon chain of eight to fewer carbons and lower-alkoxy designates linear or branched alkyloxy substituents containing eight to fewer carbon atoms,

comprising reacting a compound of formula :

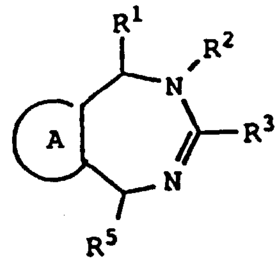

with butyllithium and then reacting the resulting compound with $R^4Z$ wherein Z is a group subjected to nucleophilic displacement.

**14.** Compounds having the formula XXXVI or acid-addition salts thereof:

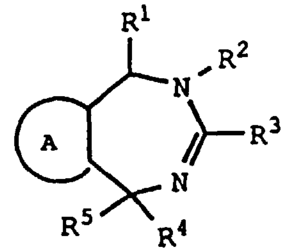

XXXVI

wherein

A is a ring chosen from the group consisting of phenyl, thienyl, furanyl, naphthyl, pyridinyl, cyclohexyl and phenyl having one or two substituents chosen from the group consisting of amino, lower-alkyl, lower-alkoxy, halogen, nitro, and lower-alkylsulfonamido;

$R^1$ is hydrogen, lower-alkyl, benzyl, naphthyl, thienyl, pyridinyl, phenyl, or phenyl having one or two substituents chosen from the group consisting of lower-alkyl and lower-alkoxy;

$R^2$ is hydrogen; lower-alkyl; benzyl; phenyl; phenyl substituted with halogen, lower-alkyl or lower-alkoxy; or

$R^2$ is $-CH_2CH_2R^7$ where $R^7$ is lower-alkoxy; benzyl; di(lower-alkyl)amino, pyrrolidino; piperidino; morpholino; phenyl; or phenyl substituted with amino, nitro or lower-alkyl sulfonamido;

$R^3$ is $Y_p-(CH_2)_m-X_n-R^8$ wherein

Y is -NH-, -O-, -S-, or

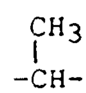;

p is zero or one;
m is an integer from zero to seven;
X is -S-, -O-, $-SO_2-$,

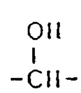, 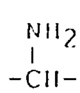, 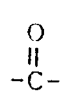,

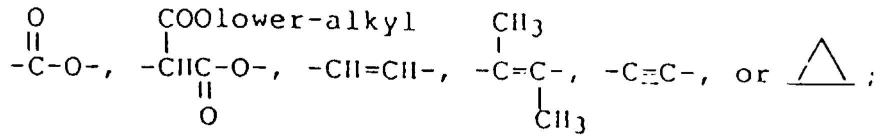

n is zero or one; and

$R^8$ is hydrogen;lower-alkyl; phenyl; furanyl; thienyl; pyridinyl; phenyl having one or two substituents chosen from the group consisting of halogen, lower-alkyl, nitro, hydroxy, lower-alkoxy, lower-alkylamido, lower-alkylsulfonamido dilower-alkylaminosulfonyl, and amino; or with a 5-membered heterocycle containing one or more oxygen atoms; or when n is zero and m is other than zero, $R^8$ is additionally halogen; benzyl(lower-alkyl)-amino; di-(lower-alkyl)amino; or a 5- or 6- membered heterocycle containing one or two nitrogens, said heterocycle being unsubstituted or substituted with one lower-alkyl group; or X and $R^8$ taken together are cyclohexylidine;

$R^4$ is hydrogen; lower-alkyl; allyl; lower-alkoxylower-alkyl; acetyl; lower-alkoxy-carbonyl, lower-alkoxy-carbonyl-alkyl; or α-hydroxy-lower .-alkyl; and

$R^5$ is hydrogen; lower-alkyl; naphthyl; thienyl; pyridinyl; benzyl; phenyl; or phenyl having one or two substituents chosen independently from the group consisting of lower-alkyl,lower-alkoxy, halogen, hydroxyl, amino, di (lower-alkyl)amino, lower-alkylsulfonamido and lower-acylamino;

with the proviso that the total number of carbon atoms in $R^1$ plus $R^2$ plus $R^4$ plus $R^5$ must be five or greater, it being understood that lower-alkyl designates a linear, branched or cyclic saturated carbon chain of eight to fewer carbons and lower-alkoxy designates linear or branched alkyloxy substituents containing eight to fewer carbon atoms.

## Patentansprüche

### Patentansprüche für folgende Vertragsstaaten : AT, BE, CH, DE, DK, FR, GB,GR, IT, LI, LU, NL, SE

**1.** Verbindungen der Formel XXXVI oder deren Additionssalze:

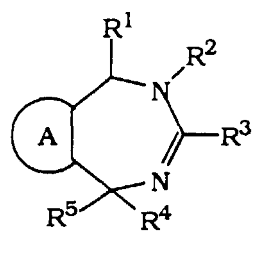

XXXVI

in der

A einen Ring ausgewählt aus der Gruppe, die Phenyl, Thienyl. Furanyl. Naphthyl, Pyridinyl. Cyclohexyl und Phenyl mit einem oder mehreren Substituenten ausgewählt aus der Amino, Niedrigalkyl, Niedrigalkoxy, Halogen. Nitro und Niedrigalkylsulfonamido umfassenden Gruppe. umfaßt:

$R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;

$R^2$ Wasserstoff; Niedrigalkyl; Benzyl; Phenyl; Phenyl substituiert mit Halogen, Niedrigalkyl oder Niedrigalkoxy; oder

$R^2$ $-CH_2CH_2R^7$, in der $R^7$ Niedrigalkoxy; Benzyl; Di(niedrigalkyl)-amino, Pyrrolidino; Piperidino; Morpholino; Phenyl; oder Phenyl substituiert mit Amino, Nitro oder Niedrigalkylsulfonamido darstellt;

$R^3$ $Yp - (CH_2)_m - X_n - R^8$, in der

Y -NH-,-O-,-S- oder

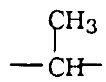 ;

p Null oder Eins.

m eine ganze Zahl mit einem Wert von Null bis Sieben;

X -S-,-O-,-$SO_2$-,

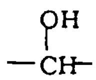, 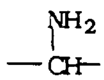, 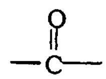, 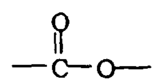,

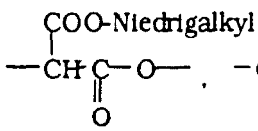, $-CH=CH-$, 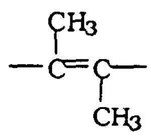, $-C\equiv C-$ oder 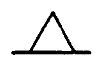 ;

n Null oder Eins; und

$R^8$ Wasserstoff; Niedrigalkyl; Phenyl; Furanyl; Thienyl; Pyridinyl; Phenyl mit einem oder zwei Substituenten ausgewählt aus der Halogen, Niedrigalkyl, Nitro, Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome aufweist; oder, wenn n Null und m von Null verschieden ist, $R^8$ zusätzlich Halogen; Benzyl-(niedrigalkyl)amino; Di(niedrigalkyl)-amino; oder einen 5- oder 6-gliedrigen Heterocylcus, der ein oder zwei Stickstoffatome aufweist, wobei der Heterocyclus unsubstituiert oder mit einer Niedrigalkylgruppe substituiert ist; oder X und $R^8$ gemeinsam Cyclohexylidin darstellen;

$R^4$ Wasserstoff; Niedrigalkyl; Allyl; Niedrigalkoxy-niedrigalkyl; Acetyl; Niedrigalkoxy-carbonyl; Niedrigalkoxy-carbonyl-alkyl; oder $\alpha$-Hydroxy-niedrigalkyl; und

$R^5$ Wasserstoff; Niedrigalkyl; Naphthyl; Thienyl; Pyridinyl; Benzyl; Phenyl; oder Phenyl mit einem oder zwei Substituenten ausgewählt unabhängig voneinander aus der Niedrigalkyl, Niedrigalkoxy, Halogen, Hydroxy, Amino, Di(niedrigalkyl)-amino, Niedrigalkylsulfonamido und Niedrigacylamino umfassenden Gruppe;

mit der Maßgabe bedeuten, daß die Gesamtzahl von Kohlenstoffatomen in $R^1$ plus $R^2$ plus $R^4$ plus $R^5$ Fünf oder größer sein muß, wobei es sich versteht, daß Niedrigalkyl für eine geradkettige. verzweigte oder cyclische gesättigte Kohlenstoffkette mit acht oder weniger Kohlenstoffatomen und Niedrigalkoxy für geradkettige oder verzweigte Alkyloxysubstituenten, die acht oder weniger Kohlenstoffatome enthalten, stehen.

2. Verbindungen nach Anspruch 1 der Formel

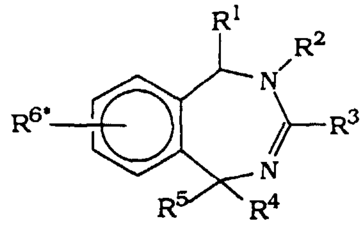

in der

$R^1$ Wasserstoff, Niedrigalkyl, Benzyl, Naphthyl, Thienyl. Pyridinyl. Phenyl oder Phenyl mit einem oder zwei Substituenten ausgewählt aus der Niedrigalkyl und Niedrigalkoxy umfassenden Gruppe;

$R^2$ Wasserstoff; Niedrigalkyl; Benzyl; Phenyl; Phenyl substituiert mit Halogen, Niedrigalkyl oder Niedrigalkoxy; oder

$R^2$ $-CH_2CH_2R^7$, in der $R^7$ Niedrigalkoxy; Benzyl; Di(niedrigalkyl)-amino, Pyrrolidino; Piperidino; Morpholino; Phenyl; oder Phenyl substituiert mit Amino. Nitro oder Niedrigalkylsulfonamido darstellt;

$R^3$ $Yp - (CH_2)_m - X_n - R^8$, in der

Y - NH-, - O -, - S - oder

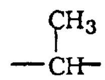 ;

p Null oder Eins,
m eine ganze Zahl mit einem Wert von Null bis Sieben;
X -S-, -O-, $-SO_2-$,

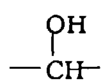 , 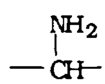 , 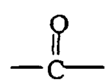 , 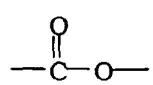 ,

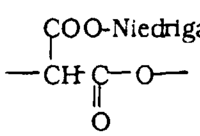 , $-CH=CH-$ , 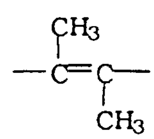 , $-C\equiv C-$ oder 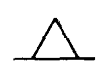 ;

n Null oder Eins; und

$R^8$ Wasserstoff; Niedrigalkyl; Phenyl; Furanyl; Thienyl; Pyridinyl; Phenyl mit einem oder zwei Substituenten ausgewählt aus der Halogen. Niedrigalkyl, Nitro, Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder, wenn n Null und m von Null verschieden sind. $R^8$ zusätzlich Halogen; Benzyl-(niedrigalkyl)amino; Di(niedrigalkyl)-amino; oder einen 5- oder 6-gliedrigen Heterocylcus, der ein oder zwei Stickstoffatome aufweist, wobei der Heterocyclus unsubstituiert oder mit einer Niedrigalkylgruppe substituiert ist; oder X und $R^8$ gemeinsam Cyclohexylidin darstellen;

$R^4$ Wasserstoff; Niedrigalkyl; Allyl; Niedrigalkoxy-niedrigalkyl; Acetyl; Niedrigalkoxy-carbonyl; Niedrigalkoxy-carbonyl-alkyl; oder α-Hydroxy-niedrigalkyl;

$R^5$ Wasserstoff; Niedrigalkyl; Naphthyl; Thienyl; Pyridinyl; Benzyl; Phenyl; oder Phenyl mit einem oder zwei Substituenten ausgewählt aus der Niedrigalkyl, Niedrigalkoxy, Halogen, Hydroxy, Amino, Di(niedrigalkyl)-amino, Niedrigalkylsulfonamido und Niedrigacylamino umfassenden Gruppe; und

$R^{6*}$ einen oder zwei Substituenten ausgewählt aus der Wasserstoff, Niedrigalkyl, Niedrigalkoxy, Amino, Halogen, Nitro und Niedrigalkylsulfonamido umfassenden Gruppe;

mit der Maßgabe bedeuten, daß die Gesamtzahl von Kohlenstoffatomen in $R^1$ plus $R^2$ plus $R^4$ plus $R^5$ Fünf oder mehr sein muß.

**3.** 4,5-Dihydro-4-methyl-1-phenyl-3-(2-phenylethyl)-1H-2,4-benzodiazepin,

R-(+)-4,5-Dihydro-4-methyl-1-phenyl-3-(2-phenylethyl)-1H-2,4-benzodiazepin.
N-[4-[2-(4,5-Dihydro-4-methyl-1-phenyl-1-H-2,4-benzodiazepin-3-yl)-ethyl)-phenyl]-methansulfonamid,
N-[4-[2-(4,5-Dihydro-3-ethyl-1-phenyl-1-H-2,4-benzodiazepin-4-yl)-ethyl)-phenyl]-methansulfonamid,
N-[4,5-Dihydro-4-methyl-1-phenyl-3-(2-phenylethyl)-1H-2,4-benzodiazepin-8-yl)-methansulfonamid,
1-(Chlorphenyl)-4,5-dihydro-4-methyl-3-(2-phenylethyl)-1H-2,4-benzodiazepin,
3-[2-(4-Chlorphenyl)-ethyl]-4,5-dihydro-4-methyl-1-phenyl-1H-2,4-benzodiazepin,
3-[2-(4-Chlorphenyl)-ethyl]-4,5-dihydro-1,4-dimethyl-1-phenyl-1H-2,4-benzodiazepin,
N,N-Diethyl-4-[4,5-dihydro-5-methyl-1-phenyl-1H-2,4-benzodiazepin-4-yl]-benzolsulfonamid,
4,5-Dihydro-1-(4-hydroxyphenyl)-4-methyl-3-(2-phenylethyl)-1H-2,4-benzodiazepin,
4,5-Dihydro-3-(2-(4-hydroxyphenyl)-ethyl]-4-methyl-1-phenyl-1H-2,4-benzodiazepin oder deren Säureadditionssalze nach Anspruch 2.

**4.** Verbindung nach Anspruch 1 der Formel:

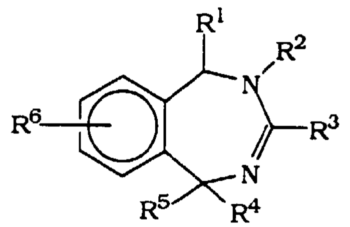

I

in der

$R^1$ Wasserstoff, Niedrigalkyl, Phenyl, Phenyl mit einem oder zwei Substituenten ausgewählt aus der Niedrigalkyl und Niedrigalkoxy umfassenden Gruppe, Naphthyl, Thienyl, Pyridinyl oder Benzyl:
$R^2$ Wasserstoff; Niedrigalkyl; Benzyl; Phenyl: Phenyl substituiert mit Halogen, Niedrigalkyl oder Niedrigalkoxy; oder
$R^2$ $-CH_2CH_2R^7$, in der $R^7$ Niedrigalkoxy; Phenyl, Benzyl, Di(niedrigalkyl)-amino, Pyrrolidino, Piperidino oder Morpholino darstellt;
$R^3$ $Yp - (CH_2)_m - X_n - R^8$, in der

Y - NH-, - O -, - S - oder

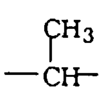 ;

p Null oder Eins,
m eine ganze Zahl mit einem Wert von Null bis Sieben;
X -S-,-O-,$-SO_2-$,

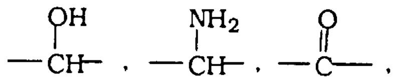

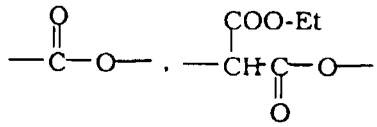

oder -C≡C- ;
n Null oder Eins; und
$R^8$ Niedrigalkyl; Phenyl; Phenyl mit einem oder zwei Substituenten ausgewählt aus der Halogen, Niedrigalkyl und Niedrigalkoxy umfassenden Gruppe; oder wenn n Null und m von Null verschieden sind, $R^8$ zusätzlich Wasserstoff; Halogen; Benzyl-(niedrigalkyl)-amino; Di(niedrigalkyl)-amino; oder einen 5- oder 6-gliedrigen Heterocylcus, der ein oder zwei Stickstoffatome aufweist. wobei der Heterocyclus unsubstituiert oder mit einer Niedrigalkylgruppe substituiert ist; darstellen;

$R^4$ Wasserstoff; Niedrigalkyl; Allyl; Niedrigalkoxy-niedrigalkyl; Acetyl; Niedrigalkoxy-carbonyl-alkyl; Niedrigalkylcarboxyl; oder α-Hydroxyniedrigalkyl;
$R^5$ Wasserstoff; Niedrigalkyl; Phenyl: Phenyl mit einem oder zwei Substituenten ausgewählt aus der Niedrigalkyl, Niedrigalkoxy, Halogen, Amino, Di(niedrigalkyl)-amino und Niedrigacylamino umfassenden Gruppe; Naphthyl; Thienyl; Pyridinyl; oder Benzyl; und
$R^6$ einen oder zwei Substituenten ausgewählt aus der Wasserstoff, Niedrigalkyl, Niedrigalkoxy und Halogen umfassenden Gruppe:

mit der Maßgabe bedeuten, daß die Gesamtzahl der Kohlenstoffatome in $R^1$ plus $R^2$ plus $R^4$ plus $R^5$ Fünf oder größer ist.

**5.** Verfahren zur Herstellung einer Verbindung nach Anspruch 1 der Formel V

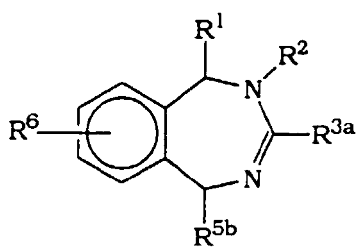

V

welches darin besteht

a) eine Verbindung der Formel VId

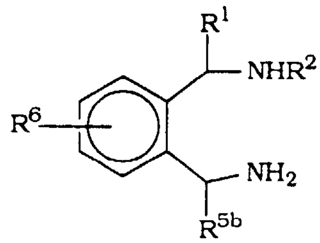

VI d

in der

$R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;

$R^2$ Wasserstoff; Niedrigalkyl; Benzyl; Phenyl; Phenyl substituiert mit Halogen, Niedrigalkyl oder Niederigalkoxy; oder

$R^2$ $-CH_2CH_2R^7$, in der $R^7$ Niedrigalkoxy, Benzyl, Di(niedrigalkyl)-amino, Pyrrolidino, Piperidino, Morpholino, Phenyl oder Phenyl substituiert mit Amino. Nitro oder Niedrigalkylsulfonamido, darstellt;

$R^{3a}$ $(Y^a)_p-(CH_2)_m-(X^a)_n-R^8$, in der

$Y^a$ -O-,-S- oder

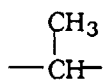 ;

p Null oder Eins;

m eine ganze Zahl mit einem Wert von Null bis Sieben;

$X^a$ -S-, $-SO_2-$, -O- oder -CH=CH-;

n Null oder Eins; und

$R^8$ Wasserstoff; Niedrigalkyl; Phenyl; Furanyl; Thienyl; Pyridinyl; Phenyl mit einem oder zwei Substituenten ausgewählt aus der Halogen. Niedrigalkyl, Nitro. Hydroxy, Niedrigalkoxy, Niedrigalkylamido. Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder wenn n Null und m von Null verschieden ist, $R^8$ zusätzlich Halogen; Benzyl-(niedrigalkyl)amino; oder einen 5- oder 6-gliedrigen Heterocyclus, der ein oder zwei Stickstoffatome enthält, wobei der Heterocyclus unsubstituiert oder mit einer Niedrigalkylgruppe substituiert ist; oder X und $R^8$ gemeinsam Cyclohexylidin darstellen:

$R^{5b}$ Wasserstoff; Niedrigalkyl; Phenyl; Phenyl mit einem oder zwei Substituenten ausgewählt aus der Niedrigalkyl, Niedrigalkoxy und Halogen umfassenden Gruppe; Naphthyl; Thienyl; Pyridinyl; oder Benzyl; und

$R^6$ einen oder zwei Substituenten unabhängig voneinander ausgewählt aus der Wasserstoff, Niedrigalkyl, Niedrigalkoxy, Halogen, Nitro und Niedrigalkylsulfonamido umfassenden Gruppe bedeuten;

1) mit einem Iminoether der Formel

$$R^{3a}C(OR^{12})NH$$

in der $R^{12}$ Methyl oder Ethyl bedeutet, oder einem Salz davon;

2) mit einem Orthoester der Formel $R^{3a}C(OR^{12})_3$

3) mit einem Ester der Formel $R^{3a}COOR^{12}$ in Gegenwart von Trialkylaluminium umzusetzen oder

b) eine Verbindung der Formel XXVII oder XXVIII

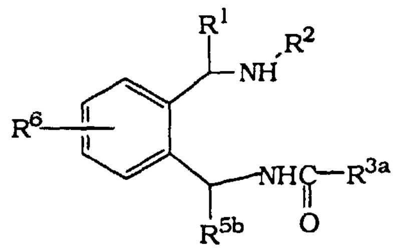

XXVII

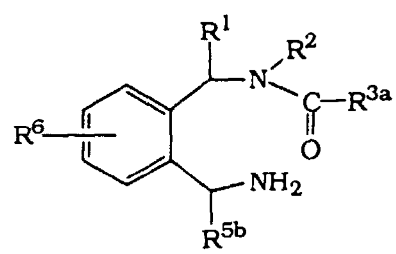

XXVIII

mit einem Trialkylaluminium umzusetzen.

**6.** Verfahren zur Herstellung einer Verbindung nach Anspruch 1 der Formel Ia

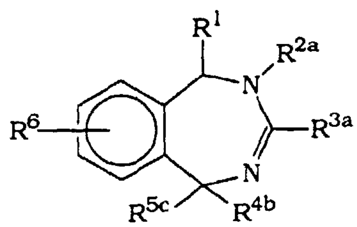

Ia

in der

$R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;
$R^{2a}$ Niedrigalkyl; Benzyl; Phenyl; Phenyl substituiert mit Halogen, Niedrigalkyl oder Niedrigalkoxy; oder
$R^{2a}$ $-CH_2CH_2R^7$, in der $R^7$ Niedrigalkoxy, Phenyl, Benzyl, Di(niedrigalkyl)-amino, Pyrrolidino Piperidino oder Morpholino darstellt;
$R^{3a}$ $(Y^a)p - (CH_2)_m - (X^a)_n - R^8$, in der

$Y^a$ -O-,-S- oder

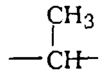

;

p Null oder Eins,
m eine ganze Zahl mit einem Wert von Null bis Sieben;
$X^a$ -S-, $-SO_2-$, -O- oder -CH=CH-;
n Null oder Eins; und
$R^8$ Wasserstoff; Niedrigalkyl; Phenyl; Furanyl; Thienyl; Pyridinyl; Phenyl mit einem oder zwei Substituenten ausgewählt aus der Halogen, Niedrigalkyl, Nitro, Hydroxy, Niedrigalkoxy, Niedrigalkylamido. Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder. wenn n Null und m von Null verschieden ist, $R^8$ zusätzlich Halogen; Benzyl-(niedrigalkyl)amino; Di(niedrigalkyl)-amino; oder $R^8$ einen 5- oder 6-gliedrigen Heterocyclus, der eines oder zwei Stickstoffatome enthält, wobei der Heterocyclus unsubstituiert oder mit einer Niedrigalkylgruppe substituiert ist; oder X und $R^8$ gemeinsam Cyclohexylidin darstellen;

$R^{4b}$ Niedrigalkyl; Allyl; Niedrigalkoxy-niedrigalkyl; Acetyl; Niedrigalkoxycarbonyl; Niedrigalkylcarboxyalkyl; oder α-Hydroxyniedrigalkyl:
$R^{5c}$ Phenyl; Phenyl mit einem oder zwei Substituenten ausgewählt aus der Niedrigalkyl, Niedrigalkoxy und Halogen umfassenden Gruppe; Naphthyl; Thienyl; oder Pyridinyl; und
$R^6$ einen oder zwei Substituenten unabhängig voneinander ausgewählt aus der Wasserstoff, Niedrigalkyl, Niedrigalkoxy, Halogen, Nitro und Niedrigalkylsulfonamido umfassenden Gruppe;

bedeuten,
welches darin besteht, eine Verbindung der Formel Va

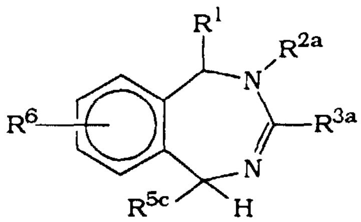

Va

mit einer starken Base umzusetzen und mit a) einem Niedrigalkylaldehyd oder b) einer Verbindung der Formel $R^{4b}Z$, in der Z eine einer nucleophilen Verdrängung unterliegende Gruppe darstellt, umzusetzen.

**7.** Verfahren zur Herstellung einer Verbindung nach Anspruch 1 der Formel VII

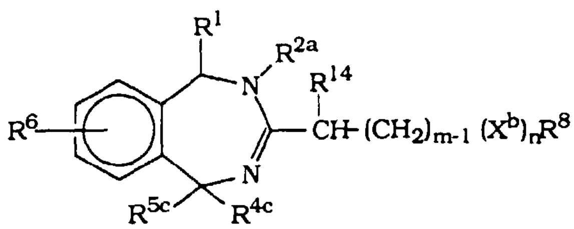

VII

in der

$R^1$ Wasserstoff, Niedrigalkyl, Benzyl, Naphthyl, Thienyl, Pyridinyl. Phenyl oder Phenyl mit einem oder zwei Substituenten ausgewählt aus der Niedrigalkyl und Niedrigalkoxy umfassenden Gruppe;
$R^{2a}$ Niedrigalkyl; Benzyl; Phenyl; Phenyl substituiert mit Halogen, Niedrigalkyl oder Niedrigalkoxy; oder
$R^{2a}$ $-CH_2CH_2R^7$, in der $R^7$ Niedrigalkoxy, Phenyl, Benzyl, Di(niedrigalkyl)-amino, Pyrrolidino, Piperidino oder Morpholino darstellt;
m eine ganze Zahl mit einem Wert von Null bis Sieben;

$X^b$ -S-,-$SO_2$-,-O-, -CH=CH-,

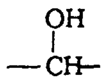 , 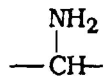 ,

oder

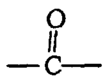 ;

n Null oder Eins;

$R^8$ Wasserstoff; Niedrigalkyl; Phenyl; Furanyl; Thienyl; Pyridinyl; Phenyl mit einem oder zwei Substituenten ausgewählt aus der Halogen, Niedrigalkyl, Nitro, Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder. wenn n Null und m von Null verschieden sind, $R^8$ zusätzlich Halogen; Benzyl-(niedrigalkyl)amino; Di(niedrigalkyl)-amino; oder $R^8$ einen 5- oder 6-gliedrigen Heterocyclus, der eines oder zwei Stickstoffatome enthält, wobei der Heterocyclus unsubstituiert oder mit einer Niedrigalkylgruppe substituiert ist; oder X und $R^8$ gemeinsam Cyclohexylidin darstellen;

$R^{4c}$ Niedrigalkyl, Allyl oder Niedrigalkoxyniedrigalkyl;

$R^{5c}$ Phenyl; Naphthyl; Thienyl; Pyridinyl; oder Phenyl mit einem oder zwei Substituenten ausgewählt aus der Niedrigalkyl, Niedrigalkoxy und Halogen umfassenden Gruppe;

$R^6$ einen oder zwei Substituenten unabhängig voneinander ausgewählt aus der Wasserstoff. Niedrigalkyl, Niedrigalkoxy, Halogen; Nitro und Niedrigalkylsulfonamido umfassenden Gruppe; und

$R^{14}$ Wasserstoff oder Methyl bedeuten.

welches darin besteht. eine Verbindung der Formel VIIa

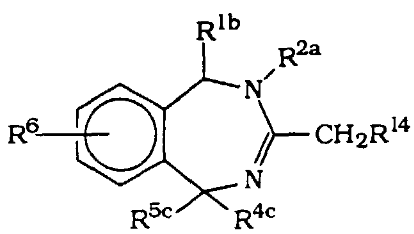

VIIa

mit einer starken Base umzusetzen gefolgt von einer Reaktion mit einem elektrophilen Mittel ausgewählt aus der Gruppe die $R^8COOR^{12}$, $R^8CHNR^{12}$, $R^8CHO$, $R^8SSR^8$, $R^8(X^b)_n(CH_2)_{m-1}Z$ umfaßt, worin $R^{12}$ Methyl oder Ethyl und Z eine einer nucleophilen Verdrängung unterliegende Gruppe bedeuten.

**8.** Verwendung einer Verbindung nach den Ansprüchen 1 bis 4 für die Herstellung eines Arzneimittels zur Behandlung von Herzarrhythmien bei einem eine solche Behandlung erfordernden Patienten.

**9.** Zubereitung für die Behandlung von Herzarrhythmien enthaltend eine antiarrhythmisch wirksame Menge einer Verbindung nach einem der Ansprüche 1 bis 4.

**10.** Verbindung nach Anspruch 1, in der A Naphthyl bedeutet.

**Patentansprüche für folgenden Vertragsstaat : ES**

1. Verfahren zur Herstellung einer Verbindung der Formel V

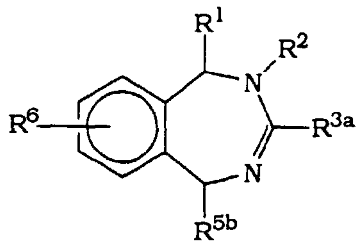

V

welches darin besteht

a) eine Verbindung der Formel VId

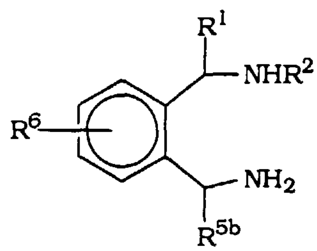

VI d

in der

$R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;

$R^2$ Wasserstoff; Niedrigalkyl; Benzyl; Phenyl; Phenyl substituiert mit Halogen, Niedrigalkyl oder Niederigalkoxy; oder

$R^2$ $-CH_2CH_2R^7$, in der $R^7$ Niedrigalkoxy, Benzyl, Di(niedrigalkyl)-amino, Pyrrolidino, Piperidino, Morpholino, Phenyl oder Phenyl substituiert mit Amino, Nitro oder Niedrigalkylsulfonamido. darstellt;

$R^{3a}$ $(Y^a)_p-(CH_2)_m-(X^a)_n-R^8$, in der

$Y^a$ -O-,-S- oder

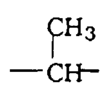 ;

p Null oder Eins;

m eine ganze Zahl mit einem Wert von Null bis Sieben;

$X^a$ -S-, $-SO_2-$, -O- oder -CH=CH-;

n Null oder Eins: und

$R^8$ Wasserstoff; Niedrigalkyl; Phenyl; Furanyl; Thienyl; Pyridinyl; Phenyl mit einem oder zwei Substituenten ausgewählt aus der Halogen, Niedrigalkyl, Nitro. Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder wenn n Null und m von Null verschieden ist, $R^8$ zusätzlich Halogen: Benzyl-(niedrigalkyl)amino: oder einen 5- oder 6-gliedrigen Heterocyclus, der ein oder zwei Stickstoffatome enthält. wobei der Heterocyclus

unsubstituiert oder mit einer Niedrigalkylgruppe substituiert ist; oder X und $R^8$ gemeinsam Cyclohexylidin darstellen;

$R^{5b}$ Wasserstoff; Niedrigalkyl; Phenyl; Phenyl mit einem oder zwei Substituenten ausgewählt aus der Niedrigalkyl, Niedrigalkoxy und Halogen umfassenden Gruppe; Naphthyl; Thienyl; Pyridinyl; oder Benzyl; und

$R^6$ einen oder zwei Substituenten unabhängig voneinander ausgewählt aus der Wasserstoff, Niedrigalkyl, Niedrigalkoxy, Halogen, Nitro und Niedrigalkylsulfonamido umfassenden Gruppe:

mit der Maßgabe bedeuten. daß die Gesamtzahl von Kohlenstoffatomen in $R^1$ plus $R^2$ plus $R^{5b}$ Fünf oder größer sein muß, wobei es sich versteht, daß Niedrigalkyl für eine geradkettige, verzweigte oder cyclische gesättigte Kohlenstoffkette mit acht oder weniger Kohlenstoffatomen und Niedrigalkoxy für geradkettige oder verzweigte Alkyloxysubstituenten, die acht oder weniger Kohlenstoffatome enthalten, stehen;

1) mit einem Iminoether der Formel

$$R^{3a}C(OR^{12})NH$$

in der $R^{12}$ Methyl oder Ethyl bedeutet, oder einem Salz davon ;

2) mit einem Orthoester der Formel $R^{3a}C(OR^{12})_3$

3) mit einem Ester der Formel $R^{3a}COOR^{12}$ in Gegenwart von Trialkylaluminium umzusetzen oder

b) eine Verbindung der Formel XXVII oder XXVIII

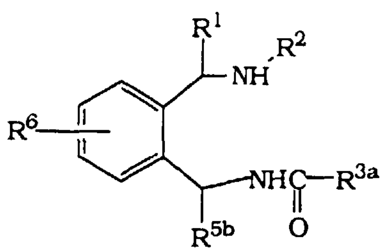

XXVII

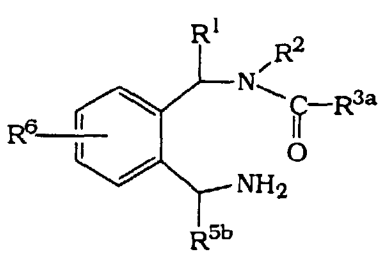

XXVIII

mit einem Trialkylaluminium umzusetzen,

gewünschtenfalls eine erhaltene Verbindung, in der $R^6$ Nitro oder $R^8$ Nitrophenyl bedeuten. zur Herstellung der entsprechenden Verbindung, in der $R^6$ Amino oder $R^8$ Aminophenyl bedeuten. zu reduzieren,

gewünschtenfalls eine erhaltene Verbindung, in der $R^6$ Amino oder $R^8$ Aminophenyl bedeuten, mit einem Niedrigalkylsulfonylhalogenid zur Herstellung der entsprechenden Verbindung, in der $R^6$ Niedrigalkylsulfonamido oder $R^8$ Niedrigalkylsulfonamidophenyl bedeuten. zu sulfonylieren,

gewünschtenfalls eine erhaltene Verbindung, in der $R^8$ Aminophenyl bedeutet, zur Herstellung der entsprechenden Verbindung, in der $R^8$ Niedrigalkylamidophenyl darstellt. zu acylieren.

gewünschtenfalls eine erhaltene Verbindung, in der $R^8$ Niedrigalkoxyphenyl darstellt. zur Herstellung der entsprechenden Verbindung, in der $R^8$ Hydroxyphenyl bedeutet, zu spalten,
gewünschtenfalls eine erhaltene Mischung von Enantiomeren, in denen $R^1$ oder $R^{5b}$ von Wasserstoff verschieden sind, zu Herstellung einer entsprechenden Mischung, die an einem einzigen Enantiomer in einer Menge von mehr als 90 % angereichert ist, aufzuspalten, und
gewünschtenfalls eine erhaltene freie Base in ein Säureadditionssalz davon umzuwandeln.

**2.** Verfahren nach Anspruch 1, worin $R^1$ Wasserstoff, $R^2$ Niedrigalkyl, $R^{5b}$ Phenyl. Naphthyl, Thienyl. Pyridinyl, Benzyl oder Phenyl mit einem oder zwei Substituenten unabhängig voneinander ausgewählt aus der Niedrigalkyl. Niedrigalkoxy und Halogen umfassenden Gruppe, $R^6$ Wasserstoff, Niedrigalkoxy, Nitro oder Niedrigalkylsulfonamido und in $R^{3a}$ p Null, m Null. Eins oder Zwei. $R^8$ Phenyl. Phenyl mit einem oder zwei Substituenten ausgewählt aus der Halogen. Niedrigalkyl, Nitro, Hydroxy, Niedrigalkoxy, Niedrigalkylsulfonamido und Di(niedrigalkyl)-aminosulfonyl umfassenden Gruppe, oder $R^8$ ein 5- oder 6-gliedriger Heterocyclus, der ein oder zwei Stickstoffatome aufweist, wobei der Heterocyclus unsubstituiert oder mit einer Niedrigalkylgruppe substituiert ist, bedeuten.

**3.** Verfahren nach Anspruch 2, worin $R^2$ Methyl, $R^{5b}$ Phenyl oder substituiertes Phenyl, $R^6$ Wasserstoff und in $R^{3a}$ m Null oder Zwei. $X^a$ -CH=CH- und $R^8$ Phenyl oder substituiertes Phenyl bedeuten.

**4.** Verfahren nach Anspruch 3, worin $R^{5b}$ Phenyl und $R^{3a}$

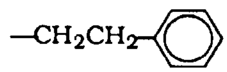

bedeuten.

**5.** Verfahren zur Herstellung einer Verbindung der Formel Ia

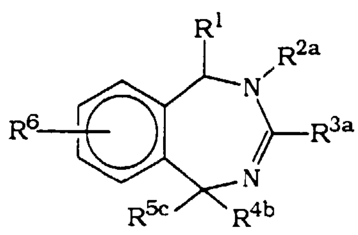

Ia

in der

$R^1$ Wasserstoff, Niedrigalkyl. Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe:
$R^{2a}$ Niedrigalkyl; Benzyl: Phenyl: Phenyl substituiert mit Halogen, Niedrigalkyl oder Niedrigalkoxy: oder
$R^{2a}$ $-CH_2CH_2R^7$, in der $R^7$ Niedrigalkoxy. Phenyl, Benzyl, Di(niedrigalkyl)-amino, Pyrrolidino, Piperidino oder Morpholino darstellt;
$R^{3a}$ $(Y^a)p - (CH_2)_m - (X^a)_n - R^8$, in der

$Y^a$ -O-,-S- oder

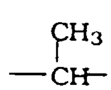 ;

p Null oder Eins.

m eine ganze Zahl mit einem Wert von Null bis Sieben;
$X^a$ -S-, $-SO_2-$, -O- oder -CH=CH-;
n Null oder Eins; und
$R^8$ Wasserstoff; Niedrigalkyl; Phenyl; Furanyl; Thienyl; Pyridinyl; Phenyl mit einem oder zwei Substituenten ausgewählt aus der Halogen, Niedrigalkyl, Nitro, Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder, wenn n Null und m von Null verschieden ist, $R^8$ zusätzlich Halogen; Benzyl-(niedrigalkyl)amino; Di(niedrigalkyl)-amino; oder $R^8$ einen 5- oder 6-gliedrigen Heterocyclus, der eines oder zwei Stickstoffatome enthält, wobei der Heterocyclus unsubstituiert oder mit einer Niedrigalkylgruppe substituiert ist; oder X und $R^8$ gemeinsam Cyclohexylidin darstellen;

$R^{4b}$ Niedrigalkyl; Allyl; Niedrigalkoxy-niedrigalkyl; Acetyl; Niedrigalkoxycarbonyl; Niedrigalkylcarbonylalkyl; oder α-Hydroxyniedrigalkyl;
$R^{5c}$ Phenyl; Phenyl mit einem oder zwei Substituenten ausgewählt aus der Niedrigalkyl, Niedrigalkoxy und Halogen umfassenden Gruppe; Naphthyl: Thienyl; oder Pyridinyl; und
$R^6$ einen oder zwei Substituenten unabhängig voneinander ausgewählt aus der Wasserstoff, Niedrigalkyl, Niedrigalkoxy, Halogen, Nitro und Niedrigalkylsulfonamido umfassenden Gruppe mit der Maßgabe bedeuten, daß die Gesamtzahl der Kohlenstoffatome in $R^1$ plus $R^{2a}$ plus $R^{4b}$ Z plus $R^{5c}$ Fünf oder mehr beträgt, wobei es sich versteht, daß Niedrigalkyl für eine geradkettige, verzweigte oder cyclische gesättigte Kohlenstoffkette mit acht oder weniger Kohlenstoffatomen und Niedrigalkoxy für geradkettige oder verzweigte Alkyloxysubstituenten, die acht oder weniger Kohlenstoffatome enthalten, stehen;

welches darin besteht, eine Verbindung der Formel Va

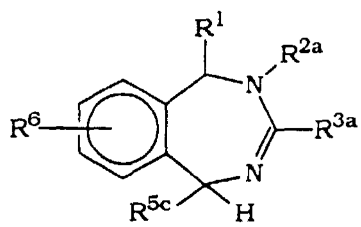

Va

mit einer starken Base umzusetzen und mit a) einem Niedrigalkylaldehyd oder b) einer Verbindung der Formel $R^{4b}Z$, in der Z eine einer nucleophilen Verdrängung unterliegende Gruppe darstellt, umzusetzen,
gewünschtenfalls eine erhaltene Verbindung, in der $R^6$ Nitro oder $R^8$ Nitrophenyl bedeuten, zur Herstellung der entsprechenden Verbindung, in der $R^6$ Amino oder $R^8$ Aminophenyl bedeuten. zu reduzieren,
gewünschtenfalls eine erhaltene Verbindung, in der $R^6$ Amino oder $R^8$ Aminophenyl bedeuten. mit einem Niedrigalkylsulfonylhalogenid zur Herstellung der entsprechenden Verbindung, in der $R^6$ Niedrigalkylsulfonamido oder $R^8$ Niedrigalkylsulfonamidophenyl bedeuten, zu sulfonylieren,
gewünschtenfalls eine erhaltene Verbindung, in der $R^8$ Aminophenyl bedeutet, zur Herstellung der entsprechenden Verbindung, in der $R^8$ Niedrigalkylamidophenyl darstellt, zu acylieren,
gewünschtenfalls eine erhaltene Verbindung, in der $R^8$ Niedrigalkoxyphenyl darstellt, zur Herstellung der entsprechenden Verbindung, in der $R^8$ Hydroxyphenyl bedeutet, zu spalten,
gewünschtenfalls eine erhaltene Mischung von Enantiomeren, in denen $R^1$ oder $R^{5b}$ von Wasserstoffverschieden sind, zur Herstellung einer entsprechenden Mischung, die an einem einzigen Enantiomer in einer Menge von mehr als 90 % angereichert ist, aufzuspalten, und
gewünschtenfalls eine erhaltene freie Base in ein Säureadditionssalz davon umzuwandeln.

**6.** Verfahren nach Anspruch 5, worin $R^1$ Wasserstoff, $R^{2a}$ Niedrigalkyl, $R^{4b}$ Niedrigalkyl, $R^{5c}$ Phenyl, Naphthyl, Thienyl, Pyridinyl, Benzyl oder Phenyl mit einem oder zwei Substituenten unabhängig voneinander ausgewählt aus der Niedrigalkyl, Niedrigalkoxy und Halogen umfassenden Gruppe, $R^6$ Wasserstoff, Niedrigalkoxy, Nitro oder Niedrigalkylsulfonamido, und in $R^{3a}$ p Null, m Null, Eins oder Zwei, $R^8$ Phenyl, Phenyl mit einem oder zwei Substituenten ausgewählt aus der Halogen, Niedrigalkyl, Nitro, Hydroxy, Niedrigalkoxy, Niedrigalkylsulfonamido und Di(niedrigalkyl)-aminosulfonyl umfassenden Gruppe. oder $R^8$ einen 5- oder 6-gliedrigen Heterocyclus, der ein oder zwei Stickstoffatome aufweist, wobei der Heterocyclus unsubstituiert oder mit einer Niedrigalkylgruppe substituiert ist, bedeuten.

**7.** Verfahren nach Anspruch 6, worin $R^{2a}$ Methyl, $R^{4b}$ Methyl. $R^{5c}$ Phenyl oder substituiertes Phenyl, $R^6$ Wasserstoff und in $R^{3a}$ m Null oder Zwei, $X^a$ -CH=CH- und $R^8$ Phenyl oder substituiertes Phenyl bedeuten.

**8.** Verfahren zur Herstellung einer Verbindung der Formel VII

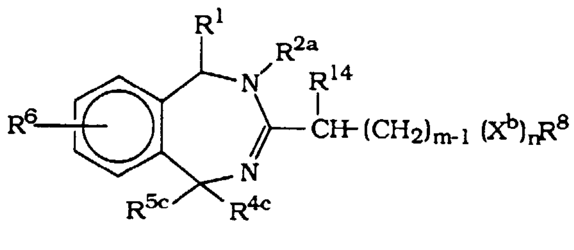

VII

in der

- $R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;
- $R^{2a}$ Niedrigalkyl; Benzyl; Phenyl; Phenyl substituiert mit Halogen, Niedrigalkyl oder Niedrigalkoxy; oder
- $R^{2a}$ $-CH_2CH_2R^7$, in der $R^7$ Niedrigalkoxy, Phenyl, Benzyl, Di(niedrigalkyl)-amino, Pyrrolidino. Piperidino oder Morpholino darstellt;
- m eine ganze Zahl mit einem Wert von Null bis Sieben;
- $X^b$ -S-,$-SO_2-$,-O-, -CH=CH- ,

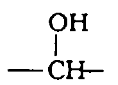 , 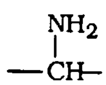 ,

oder

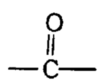 ;

- n Null oder Eins;
- $R^8$ Wasserstoff; Niedrigalkyl; Phenyl: Furanyl; Thienyl; Pyridinyl; Phenyl mit einem oder zwei Substituenten ausgewählt aus der Halogen, Niedrigalkyl, Nitro, Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder, wenn n Null und m von Null verschieden sind, $R^8$ zusätzlich Halogen; Benzyl-(niedrigalkyl)-amino; Di(niedrigalkyl)-amino; oder $R^8$ einen 5- oder 6-gliedrigen Heterocyclus, der ein oder zwei Stickstoffatome enthält, wobei der Heterocyclus unsubstituiert oder mit einer Niedrigalkylgruppe substituiert ist; oder X und $R^8$ gemeinsam Cyclohexylidin darstellen;
- $R^{4c}$ Niedrigalkyl, Allyl oder Niedrigalkoxyniedrigalkyl;
- $R^{5c}$ Phenyl; Naphthyl; Thienyl; Pyridinyl; oder Phenyl mit einem oder zwei Substituenten ausgewählt aus der Niedrigalkyl, Niedrigalkoxy und Halogen umfassenden Gruppe;
- $R^6$ einen oder zwei Substituenten unabhängig voneinander ausgewählt aus der Wasserstoff, Niedrigalkyl, Niedrigalkoxy, Halogen; Nitro und Niedrigalkylsulfonamido umfassenden Gruppe; und
- $R^{14}$ Wasserstoff oder Methyl,

mit der Maßgabe bedeuten, daß die Gesamtzahl von Kohlenstoffatomen in $R^1$ plus $R^{2a}$ plus $R^{4c}$ plus $R^{5b}$ Fünf

oder mehr betragen muß, wobei es sich versteht, daß Niedrigalkyl für eine geradkettige, verzweigte oder cyclische gesättigte Kohlenstoffkette mit acht oder weniger Kohlenstoffatomen und Niedrigalkoxy für geradkettige oder verzweigte Alkyloxysubstituenten, die acht oder weniger Kohlenstoffatome aufweisen, stehen;
welches darin besteht. eine Verbindung der Formel VIIa

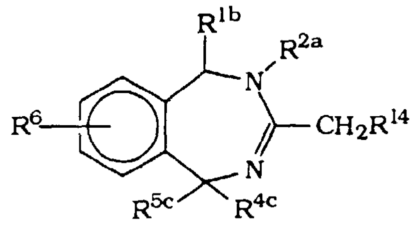

VIIa

mit einer starken Base umzusetzen gefolgt von einer Reaktion mit einem elektrophilen Mittel ausgewählt aus der Gruppe die $R^8COOR^{12}$, $R^8CHNR^{12}$, $R^8CHO$, $R^8SSR^8$, $R^8(X^b)_n(CH_2)_{m-1}Z$ umfaßt, worin $R^{12}$ Methyl oder Ethyl und Z eine einer nucleophilen Verdrängung unterliegende Gruppe bedeuten.
gewünschtenfalls eine erhaltene Verbindung, in der $R^6$ Nitro oder $R^8$ Nitrophenyl bedeuten, zur Herstellung der entsprechenden Verbindung, in der $R^6$ Amino oder $R^8$ Aminophenyl bedeuten, zu reduzieren.
gewünschtenfalls eine erhaltene Verbindung, in der $R^6$ Amino oder $R^8$ Aminophenyl bedeuten. mit einem Niedrigalkylsulfonylhalogenid zur Herstellung der entsprechenden Verbindung, in der $R^6$ Niedrigalkylsulfonamido oder $R^8$ Niedrigalkylsulfonamidophenyl bedeuten, zu sulfonylieren.
gewünschtenfalls eine erhaltene Verbindung, in der $R^8$ Aminophenyl bedeutet. zur Herstellung der entsprechenden Verbindung, in der $R^8$ Niedrigalkylamidophenyl darstellt, zu acylieren,
gewünschtenfalls eine erhaltene Verbindung, in der $R^8$ Niedrigalkoxyphenyl darstellt, zur Herstellung der entsprechenden Verbindung, in der $R^8$ Hydroxyphenyl bedeutet, zu spalten.
gewünschtenfalls eine erhaltene Mischung von Enantiomeren, in denen $R^1$ oder $R^{5b}$ von Wasserstoffverschieden sind, zur Herstellung einer entsprechenden Mischung, die an einem einzigen Enantiomer in einer Menge von mehr als 90 % angereichert ist, aufzuspalten, und
gewünschtenfalls eine erhaltene freie Base in ein Säureadditionssalz davon umzuwandeln.

**9.** Verfahren zur Herstellung einer Verbindung der Formel XXXX

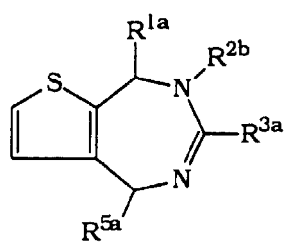

XXXX

in der

$R^{1a}$ Wasserstoff, Niedrigalkyl oder Phenyl;
$R^{2b}$ Wasserstoff; Niedrigalkyl; Di(niedrigalkyl)-aminoethyl; Benzyl; Phenyl; Phenethyl oder Phenyl substituiert mit Halogen, Niedrigalkyl oder Niedrigalkoxy;
$R^{3a}$ $(Y^a)_p$-$(CH_2)$m-$(X^a)_n$-$R^8$, in der

$Y^a$ -O-,-S- oder

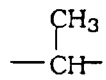 ;

p Null oder Eins,
m eine ganze Zahl mit einem Wert von Null bis Sieben:
$X^a$ -S-, $-SO_2-$, -O- oder -CH=CH-;
n Null oder Eins; und
$R^8$ Wasserstoff; Niedrigalkyl; Phenyl; Furanyl; Thienyl: Pyridinyl; Phenyl mit einem oder zwei Substituenten ausgewählt aus der Halogen, Niedrigalkyl, Nitro, Hydroxy, Niedrigalkoxy, Niedrigalkylamido, Niedrigalkylsulfonamido, Diniedrigalkylaminosulfonyl und Amino umfassenden Gruppe; oder mit einem 5-gliedrigen Heterocyclus, der ein oder mehrere Sauerstoffatome enthält; oder, wenn n Null und m von Null verschieden ist, $R^8$ zusätzlich Halogen; Benzyl-(niedrigalkyl)-amino; Di(niedrigalkyl)-amino; oder $R^8$ einen 5- oder 6-gliedrigen Heterocyclus, der eines oder zwei Stickstoffatome enthält. wobei der Heterocyclus unsubstituiert oder mit einer Niedrigalkylgruppe substituiert ist; oder $X^a$ und $R^8$ gemeinsam Cyclohexylidin darstellen;

$R^{5a}$ Wasserstoff; Phenyl; Phenyl mit einem oder zwei Substituenten ausgewählt aus der Halogen, Niedrigalkyl und Niedrigalkoxy umfassenden Gruppe; Naphthyl; Thienyl; Pyridinyl oder Benzyl;

mit der Maßgabe bedeuten, daß die Gesamtzahl von Kohlenstoffatomen in $R^{1a}$ plus $R^{2b}$ plus $R^{5a}$ Fünf oder mehr sein muß, wobei es sich versteht, daß Niedrigalkyl für eine geradkettige, verzweigte oder cyclische gesättigte Kohlenstoffkette mit acht oder weniger Kohlenstoffatomen und Niedrigalkoxy für geradkettige oder verzweigte Alkyloxysubstituenten, die acht oder weniger Kohlenstoffatome enthalten, stehen;
welches darin besteht, eine Verbindung der Formel XXXXI

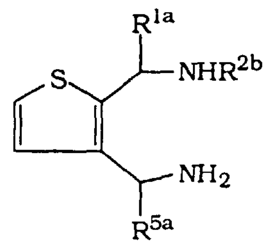

XXXX1

1) mit einem Iminoether der Formel $R^{3a}C(OR^{12})NH$, in der $R^{12}$ Methyl oder Ethyl darstellt, oder einem Salz davon
2) mit einem Orthoester der Formel $R^{3a}C(OR^{12})_3$ oder
3) mit einem Ester der Formel $R^{3a}COOR^{12}$ in Gegenwart von Trialkylaluminium umzusetzen und gewünschtenfalls eine erhaltene freie Base in ein Säureadditionssalz davon umzuwandeln.

**10.** Verfahren nach Anspruch 1 zur Herstellung einer Verbindung der Formel V

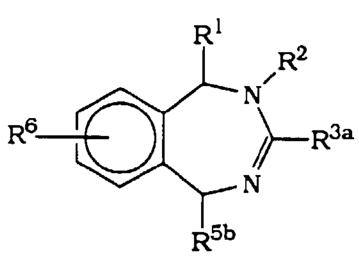

V

welches darin besteht a) eine Verbindung der Formel VId

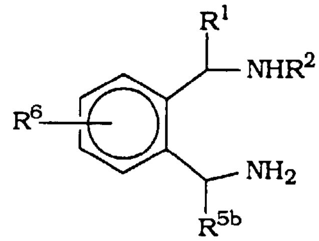

VI d

in der

- $R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;
- $R^2$ Wasserstoff; Niedrigalkyl; Benzyl; Phenyl; Phenyl substituiert mit Halogen, Niedrigalkyl oder Niederigalkoxy; oder
- $R^2$ $-CH_2CH_2R^7$, in der $R^7$ Niedrigalkoxy, Benzyl, Di(niedrigalkyl)-amino, Pyrrolidino, Piperidino, Morpholino, Phenyl oder Phenyl substituiert mit Amino, Nitro oder Niedrigalkylsulfonamido, darstellt;
- $R^{3a}$ $(Y^a)_p-(CH_2)_m-(X^a)_n-R^8$, in der

  $Y^a$ -O-,-S- oder

  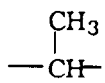 ;

  p Null oder Eins;
  m eine ganze Zahl mit einem Wert von Null bis Sieben;
  $X^a$ -S-, $-SO_2-$, -O- oder -CH=CH-;
  n Null oder Eins; und
  $R^8$ Niedrigalkyl; Phenyl; Phenyl mit einem oder zwei Substituenten ausgewählt aus der Halogen, Niedrigalkyl und Niedrigalkoxy umfassenden Gruppe; oder wenn n Null und m von Null verschieden ist, $R^8$ zusätzlich Wasserstoff; Halogen; Benzyl-(niedrigalkyl)-amino; Di(niedrigalkyl)-amino: oder einen 5- oder 6-gliedrigen Heterocyclus, der ein oder zwei Stickstoffatome enthält, wobei der Heterocyclus unsubstituiert oder mit einer Niedrigalkylgruppe substituiert ist; darstellen;

- $R^{5b}$ Wasserstoff; Niedrigalkyl; Phenyl; Phenyl mit einem oder zwei Substituenten ausgewählt aus der Niedrigalkyl, Niedrigalkoxy und Halogen umfassenden Gruppe; Naphthyl; Thienyl; Pyridinyl; oder Benzyl; und
- $R^6$ einen oder zwei Substituenten ausgewählt aus der Wasserstoff, Niedrigalkyl, Niedrigalkoxy und Halogen umfassenden Gruppe:

mit der Maßgabe bedeuten, daß die Gesamtzahl von Kohlenstoffatomen in $R^1$ plus $R^2$ plus $R^{5b}$ Fünf oder mehr betragen muß:
mit einer Verbindung ausgewählt aus der

1) einen Iminoether der Formel

$$R^{3a}C(OR^{12})NH$$

in der $R^{12}$ Methyl oder Ethyl bedeutet, oder ein Salz davon;

2) einen Orthoester der Formel $R^{3a}C(OR^{12})_3$, und

3) einen Ester der Formel $R^{3a}COOR^{12}$ umfassenden Gruppe, in Gegenwart von Trialkylaluminium umzusetzen oder

b) eine Verbindung der Formel XXVII oder XXVIII

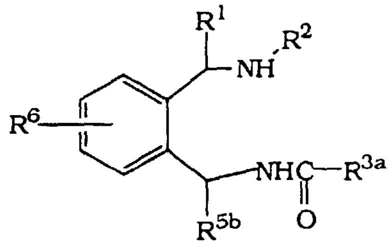

XXVII

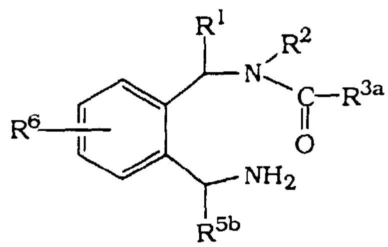

XXVIII

mit einem Trialkylaluminium umzusetzen.

**11.** Verfahren nach Anspruch 5 zur Herstellung einer Verbindung der Formel Ia

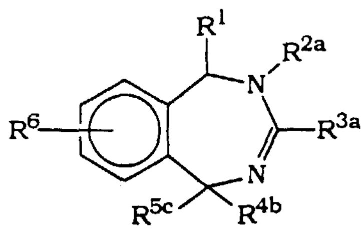

Ia

in der

$R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;
$R^{2a}$ Niedrigalkyl; Benzyl; Phenyl; Phenyl substituiert mit Halogen, Niedrigalkyl oder Niedrigalkoxy; oder
$R^{2a}$ $-CH_2CH_2R^7$, in der $R^7$ Niedrigalkoxy, Phenyl, Benzyl, Di(niedrigalkyl)-amino, Pyrrolidino Piperidino oder Morpholino darstellt;
$R^{3a}$ $(Y^a)p - (CH_2)_m - (X^a)_n - R^8$, in der

$Y^a$ -O-,-S- oder

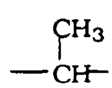 ;

p Null oder Eins,

m eine ganze Zahl mit einem Wert von Null bis Sieben;
$X^a$ -S-, $-SO_2-$, -O- oder -CH=CH-;
n Null oder Eins: und
$R^8$ Niedrigalkyl; Phenyl; Phenyl mit einem oder zwei Substituenten ausgewählt aus der Halogen, Niedrigalkyl und Niedrigalkoxy umfassenden Gruppe; oder. wenn n Null und m von Null verschieden ist, $R^8$ zusätzlich Wasserstoff; Halogen; Benzyl-(niedrigalkyl)-amino; Di(niedrigalkyl)-amino; oder
$R^8$ einen 5- oder 6-gliedrigen Heterocyclus, der ein oder zwei Stickstoffatome enthält. wobei der Heterocyclus unsubstituiert oder mit einer Niedrigalkylgruppe substituiert ist; darstellen;

$R^{4b}$ Niedrigalkyl: Allyl; Niedrigalkoxy-niedrigalkyl; Acetyl; Niedrigalkoxycarbonyl; Niedrigalkylcarboxyalkyl; oder α-Hydroxyniedrigalkyl;
$R^{5c}$ Phenyl; Phenyl mit einem oder zwei Substituenten ausgewählt aus der Niedrigalkyl. Niedrigalkoxy und Halogen umfassenden Gruppe; Naphthyl; Thienyl; oder Pyridinyl; und
$R^6$ einen oder zwei Substituenten ausgewählt aus der Wasserstoff, Niedrigalkyl, Niedrigalkoxy und Halogen umfassenden Gruppe;

mit der Maßgabe bedeuten, daß die Gesamtzahl von Kohlenstoffatomen in $R^1$ plus $R^{2a}$ plus $R^{4b}$ plus $R^{5c}$ Fünf oder größer sein muß;
welches darin besteht, eine Verbindung der Formel Va

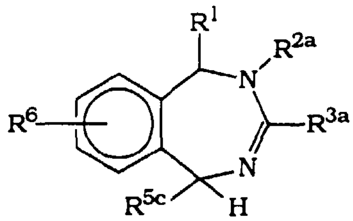

Va

mit einer starken Base umzusetzen und mit einem geeigneten Elektrophil umzusetzen.

**12.** Verfahren nach Anspruch 8 zur Herstellung einer Verbindung der Formel VII

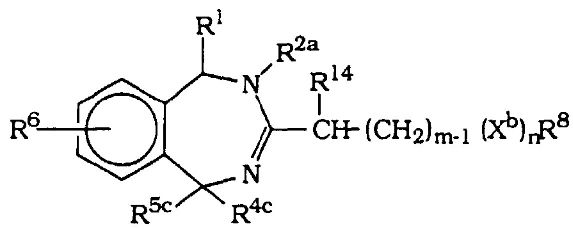

VII

in der

$R^1$ Wasserstoff, Niedrigalkyl, Benzyl, Naphthyl, Thienyl, Pyridinyl, Phenyl oder Phenyl mit einem oder zwei Substituenten ausgewählt aus der Niedrigalkyl und Niedrigalkoxy umfassenden Gruppe;
$R^{2a}$ Niedrigalkyl; Benzyl: Phenyl: Phenyl substituiert mit Halogen, Niedrigalkyl oder Niedrigalkoxy: oder
$R^{2a}$ $-CH_2CH_2R^7$, in der $R^7$ Niedrigalkoxy, Phenyl, Benzyl, Di(niedrigalkyl)-amino, Pyrrolidino, Piperidino oder Morpholino darstellt:
m eine ganze Zahl mit einem Wert von Null bis Sieben:
$X^b$ -S-,$-SO_2-$,-O-, -CH=CH- ,

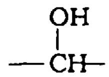 , 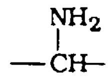 ,

oder

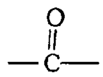 ;

n Null oder Eins:

$R^8$ Niedrigalkyl: Phenyl; Phenyl mit einem oder zwei Substituenten ausgewählt aus der Halogen, Niedrigalkyl und Niedrigalkoxy umfassenden Gruppe: oder, wenn n Null und m von Null verschieden sind, $R^8$ zusätzlich Wasserstoff; Halogen: Benzyl-(niedrigalkyl)-amino; Di(niedrigalkyl)-amino: oder $R^8$ einen 5- oder 6-gliedrigen Heterocyclus, der ein oder zwei Stickstoffatome enthält, wobei der Heterocyclus unsubstituiert oder mit einer Niedrigalkylgruppe substituiert ist; darstellen;

$R^{4c}$ Niedrigalkyl, Allyl oder Niedrigalkoxyniedrigalkyl;

$R^{5c}$ Phenyl; Naphthyl; Thienyl; Pyridinyl; oder Phenyl mit einem oder zwei Substituenten ausgewählt aus der Niedrigalkyl, Niedrigalkoxy und Halogen umfassenden Gruppe;

$R^6$ einen oder zwei Substituenten ausgewählt aus der Wasserstoff, Niedrigalkyl, Niedrigalkoxy und Halogen umfassenden Gruppe; und

$R^{14}$ Wasserstoff oder Methyl,

mit der Maßgabe bedeuten, daß die Gesamtzahl von Kohlenstoffatomen in $R^1$ plus $R^{2a}$ plus $R^{5c}$ Fünf oder größer sein muß,
welches darin besteht. eine Verbindung der Formel VIIa

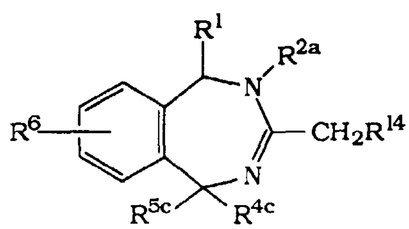

VIIa

mit einer starken Base umzusetzen gefolgt von einer Reaktion mit einem elektrophilen Mittel ausgewählt aus der Gruppe die $R^8COOR^{12}$, $R^8CHNR^{12}$, $R^8CHO$, $R^8SSR^8$, $R^8(X^b)_n(CH_2)_{m-1}Z$ umfaßt, worin $R^{12}$ Methyl oder Ethyl und Z eine einer nucleophilen Verdrängung unterliegende Gruppe bedeuten.

**13.** Verfahren zur Herstellung einer Verbindung der allgemeinen Formel XXXVI

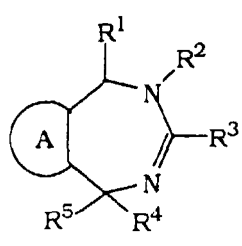

XXXVI

in der

A einen Ring ausgewählt aus der Gruppe, die Phenyl. Thienyl, Furanyl, Naphthyl, Pyridinyl, Cyclohexyl und Phenyl mit einem oder mehreren Substituenten ausgewählt aus derAmino, Niedrigalkyl, Niedrigalkoxy. Halogen. Nitro und Niedrigalkylsulfonamido umfassenden Gruppe. umfaßt:

$R^1$ Wasserstoff, Niedrigalkyl. Benzyl. Naphthyl. Thienyl, Pyridinyl, Phenyl oder Phenyl mit einem oder zwei Substituenten ausgewählt aus der Niedrigalkyl und Niedrigalkoxy umfassenden Gruppe;

$R^2$ Wasserstoff; Niedrigalkyl; Benzyl; Phenyl; Phenyl substituiert mit Halogen, Niedrigalkyl oder Niedrigalkoxy; oder

$R^2$ $-CH_2CH_2R^7$, in der $R^7$ Niedrigalkoxy; Benzyl; Di(niedrigalkyl)-amino. Pyrrolidino; Piperidino; Morpholino; Phenyl; oder Phenyl substituiert mit Amino, Nitro oder Niedrigalkylsulfonamido darstellt;

$R^3$ $Yp - (CH_2)_m - X_n - R^8$, in der

Y -NH-,-O-,-S- oder

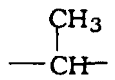 ;

p Null oder Eins,
m eine ganze Zahl mit einem Wert von Null bis Sieben;
X -S-,-O-,$-SO_2-$,

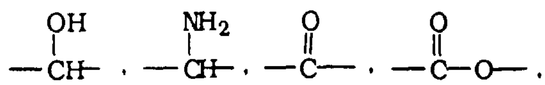

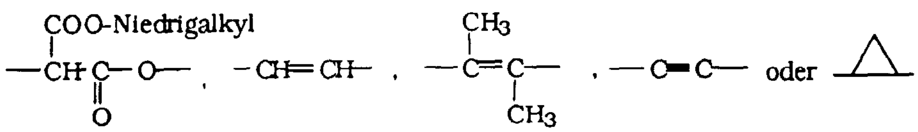 ;

n Null oder Eins; und

$R^8$ Wasserstoff; Niedrigalkyl; Phenyl; Furanyl; Thienyl; Pyridinyl; Phenyl mit einem oder zwei Substituenten ausgewählt aus der Gruppe, die Halogen, Niedrigalkyl, Nitro, Hydroxy, Niedrigalkoxy, Niedrigalkylamido, Niedrigalkylsulfonamido, Diniedrigalkylaminosulfonyl und Amino umfaßt; oder mit einem 5-gliedrigen Heterocyclus, der ein oder mehrere Sauerstoffatome aufweist; oder, wenn n Null und m von Null verschieden ist. $R^8$ zusätzlich Halogen; Benzyl-(niedrigalkyl)-amino; Di(niedrigalkyl)-amino; oder einen 5- oder 6-gliedrigen Heterocylcus, der ein oder zwei Stickstoffatome aufweist, wobei der Heterocyclus unsubstituiert oder mit einer Niedrigalkylgruppe substituiert ist; oder X und $R^8$ gemeinsam Cyclohexylidin darstellen;

$R^4$ Wasserstoff; Niedrigalkyl; Allyl; Niedrigalkoxy-niedrigalkyl; Acetyl; Niedrigalkoxy-carbonyl; Niedrigalkoxy-carbonyl-alkyl; oder $\alpha$-Hydroxy-niedrigalkyl; und

$R^5$ Wasserstoff; Niedrigalkyl; Naphthyl; Thienyl; Pyridinyl; Benzyl; Phenyl; oder Phenyl mit einem oder zwei Substituenten ausgewählt unabhängig voneinander aus der Niedrigalkyl, Niedrigalkoxy, Halogen, Hydroxy. Amino, Di(niedrigalkyl)-amino, Niedrigalkylsulfonamido und Niedrigacylamino umfassenden Gruppe:

mit der Maßgabe bedeuten, daß die Gesamtzahl von Kohlenstoffatomen in $R^1$ plus $R^2$ plus $R^4$ plus $R^5$ Fünf oder größer sein muß, wobei es sich versteht, daß Niedrigalkyl für eine geradkettige, verzweigte oder cyclische gesättigte Kohlenstoffkette mit acht oder weniger Kohlenstoffatomen und Niedrigalkoxy für geradkettige oder verzweigte Alkyloxysubstituenten, die acht oder weniger Kohlenstoffatome enthalten, stehen,
welches darin besteht, eine Verbindung der Formel

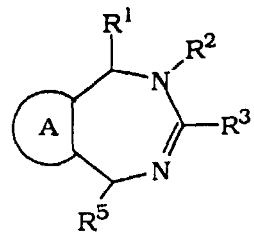

mit Butyllithium umzusetzen und dann die erhaltene Verbindung mit $R^4Z$ umzusetzen, worin Z eine einer nucleophilen Verdrängung unterliegende Gruppe darstellt.

**14.** Verbindungen der Formel XXXVI oder deren Säureadditionssalze:

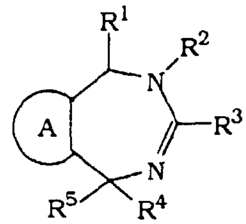

XXXVI

in der

A einen Ring ausgewählt aus der Gruppe, die Phenyl, Thienyl, Furanyl, Naphthyl, Pyridinyl, Cyclohexyl und Phenyl mit einem oder mehreren Substituenten ausgewählt aus derAmino, Niedrigalkyl, Niedrigalkoxy, Halogen, Nitro und Niedrigalkylsulfonamido umfassenden Gruppe, umfaßt:

$R^1$ Wasserstoff, Niedrigalkyl, Benzyl, Naphthyl, Thienyl. Pyridinyl. Phenyl oder Phenyl mit einem oder zwei Substituenten ausgewählt aus der Niedrigalkyl und Niedrigalkoxy umfassenden Gruppe;

$R^2$ Wasserstoff; Niedrigalkyl; Benzyl; Phenyl; Phenyl substituiert mit Halogen, Niedrigalkyl oder Niedrigalkoxy; oder

$R^2$ $-CH_2CH_2R^7$, in der $R^7$ Niedrigalkoxy; Benzyl; Di(niedrigalkyl)-amino, Pyrrolidino; Piperidino; Morpholino; Phenyl; oder Phenyl substituiert mit Amino, Nitro oder Niedrigalkylsulfonamido darstellt;

$R^3$ $Yp - (CH_2)_m - X_n - R^8$, in der

Y - NH-, - O -, - S - oder

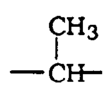 ;

p Null oder Eins.
m eine ganze Zahl mit einem Wert von Null bis Sieben;
X -S-, -O-, $-SO_2-$,

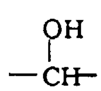 , 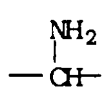 , 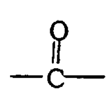 , 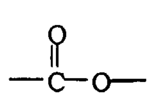 ,

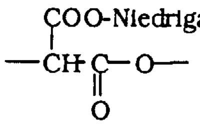, 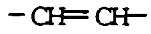, 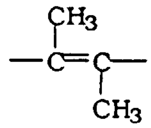, 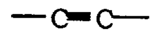 oder 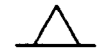 ;

n Null oder Eins: und

$R^8$ Wasserstoff; Niedrigalkyl: Phenyl: Furanyl; Thienyl; Pyridinyl; Phenyl mit einem oder zwei Substituenten ausgewählt aus der Gruppe, die Halogen, Niedrigalkyl, Nitro, Hydroxy, Niedrigalkoxy, Niedrigalkylamido, Niedrigalkylsulfonamido, Diniedrigalkylaminosulfonyl und Amino umfaßt; oder mit einem 5-gliedrigen Heterocyclus, der ein oder mehrere Sauerstoffatome aufweist; oder, wenn n Null und m von Null verschieden ist, $R^8$ zusätzlich Halogen; Benzyl-(niedrigalkyl)-amino; Di(niedrigalkyl)-amino; oder einen 5- oder 6-gliedrigen Heterocylcus, der ein oder zwei Stickstoffatome aufweist, wobei der Heterocyclus unsubstituiert oder mit einer Niedrigalkylgruppe substituiert ist; oder X und $R^8$ gemeinsam Cyclohexylidin darstellen;

$R^4$ Wasserstoff; Niedrigalkyl; Allyl; Niedrigalkoxy-niedrigalkyl; Acetyl; Niedrigalkoxy-carbonyl; Niedrigalkoxy-carbonyl-alkyl; oder $\alpha$-Hydroxy-niedrigalkyl; und

$R^5$ Wasserstoff: Niedrigalkyl; Naphthyl; Thienyl; Pyridinyl; Benzyl; Phenyl; oder Phenyl mit einem oder zwei Substituenten ausgewählt unabhängig voneinander aus der Niedrigalkyl, Niedrigalkoxy, Halogen, Hydroxy, Amlno, Di(niedrigalkyl)-amino, Niedrigalkylsulfonamido und Niedrigacylamino umfassenden Gruppe;

mit der Maßgabe bedeuten, daß die Gesamtzahl von Kohlenstoffatomen in $R^1$ plus $R^2$ plus $R^4$ plus $R^5$ Fünf oder größer sein muß, wobei es sich versteht, daß Niedrigalkyl für eine geradkettige, verzweigte oder cyclische gesättigte Kohlenstoffkette mit acht oder weniger Kohlenstoffatomen und Niedrigalkoxy für geradkettige oder verzweigte Alkyloxysubstltuenten, die acht oder weniger Kohlenstoffatome enthalten, stehen.

## Revendications

**Revendications pour les Etats contractants suivants : AT, BE, CH, DE, DK, FR, GB, GR, IT, LI, LU, NL, SE**

**1.** Composés de formule XXXVI ou leurs sels d'addition avec des acides:

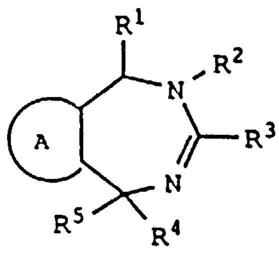

XXXVI

formule dans laquelle

A est un cycle choisi dans l'ensemble constitué par les radicaux phényle, thiényle, furannyle, naphtyle, pyridinyle, cyclohexyle et phényle portant un ou deux substituants choisis parmi des atomes d'halogène et des groupes amino, alkyle inférieur, alcoxy inférieur, nitro et (alkyl inférieur)sulfonamido;

$R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;

$R^2$ est un atome d'hydrogène ou un radical alkyle inférieur, benzyle, phényle ou phényle substitué par halogène ou par alkyle inférieur ou alcoxy inférieur; ou

$R^2$ est un radical $-CH_2CH_2R^7$, dans lequel $R^7$ est un groupe alcoxy inférieur, benzyle, di(alkyl inférieur)amino, pyrrolidino, pipéridino, morpholino, phényle ou phényle substitué par un groupe amino, nitro ou (alkyl inférieur)

sulfonamido;

$R^3$ est un groupe $Y_p$-$(CH_2)_m$-$X_n$-$R^8$, dans lequel

Y représente -NH-, -O-, -S- ou

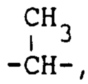

p est zéro ou un,

m est un nombre entier allant de zéro à sept,

X représente -S-, -O-, $-SO_2-$,

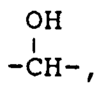 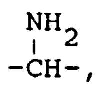 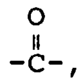 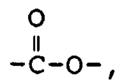

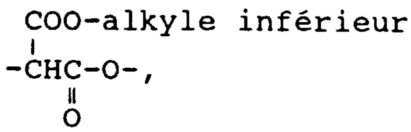 -CH=CH-, 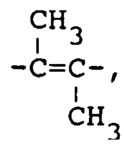 -C≡C- ou 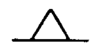,

n est zéro ou un, et

$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur)aminosulfonyle et amino, ou avec

un hétérocycle à 5 chaînons contenant un ou plusieurs atomes d'oxygène, ou

lorsque n est zéro et m est différent de zéro, $R^8$ est un outre un atome d'halogène ou un radical benzyl-(alkyl inférieur)amino, di(alkyl inférieur)amino ou un hétérocycle à 5 ou 6 chaînons contenant un ou deux atomes d'azote, ledit hétérocycle étant non substitué ou substitué par un groupe alkyle inférieur, ou

X et $R^8$ forment ensemble un radical cyclohexylidine;

$R^4$ est un atome d'hydrogène ou un radical alkyle inférieur, allyle, (alcoxy inférieur)-alkyle inférieur, acétyle, (alcoxy inférieur)-carbonyle, (alcoxy inférieur)-carbonyl-alkyle ou $\alpha$-hydroxyalkyle inférieur; et

$R^5$ est un atome d'hydrogène ou un radical alkyle inférieur, naphtyle, thiényle, pyridinyle, benzyle, phényle, ou phényle portant un ou deux substituants choisis indépendamment parmi des atomes d'halogène et des groupes alkyle inférieur, alcoxy inférieur, hydroxyle, amino, di(alkyl inférieur)amino, (alkyl inférieur)sulfonamido et (acyl inférieur)amino;

avec la condition que le nombre total des atomes de carbone dans $R^1$ plus $R^2$ plus $R^4$ plus $R^5$ doit être égal à cinq ou plus,

étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins.

**2.** Composés selon la revendication 1, de formule

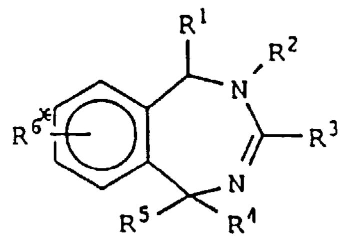

dans laquelle

- $R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;
- $R^2$ est un atome d'hydrogène ou un radical alkyle inférieur, benzyle, phényle ou phényle substitué par un halogène ou par alkyle inférieur ou alcoxy inférieur; ou
- $R^2$ est un radical $-CH_2CH_2R^7$, dans lequel $R^7$ est un groupe alcoxy inférieur, benzyle, di(alkyl inférieur)amino, pyrrolidino, pipéridino, morpholino, phényle ou phényle substitué par amino, nitro ou (alkyl inférieur)sulfonamido;
- $R^3$ est un groupe $Y_p$-$(CH_2)_m$-$X_n$-$R^8$, dans lequel

  Y représente -NH-, -O-, -S- ou

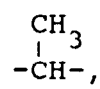

  p est zéro ou un,
  m est un nombre entier allant de zéro à sept,
  X représente -S-, -O-, $-SO_2-$,

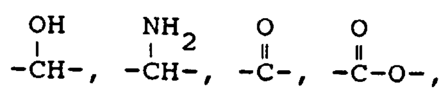

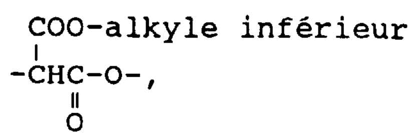

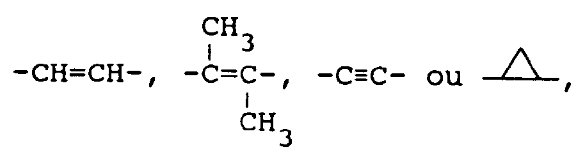

ou

- n est zéro ou un, et
- $R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido,(alkyl inférieur)sulfonamido, di(alkyl inférieur)aminosulfonyle et amino, ou avec

  un hétérocycle à 5 chaînons contenant un ou plusieurs atomes d'oxygène, ou
  lorsque n est zéro et m est différent de zéro, $R^8$ est un outre un atome d'halogène ou un radical benzyl-(alkyl inférieur)amino, di(alkyl inférieur)amino ou un hétérocycle à 5 ou 6 chaînons contenant un ou deux atomes d'azote, ledit hétérocycle étant non substitué ou substitué par un groupe alkyle inférieur, ou
  X et $R^8$ forment ensemble un radical cyclohexylidine;

- $R^4$ est un atome d'hydrogène ou un radical alkyle inférieur, allyle, (alcoxy inférieur)-alkyle inférieur, acétyle, (alcoxy inférieur)-carbonyle, (alcoxy inférieur)-carbonyl-alkyle ou α-hydroxyalkyle inférieur; et
- $R^5$ est un atome d'hydrogène ou un radical alkyle inférieur, naphtyle, thiényle, pyridinyle, benzyle, phényle, ou phényle portant un ou deux substituants choisis parmi des atomes d'halogène et des groupes alkyle inférieur, alcoxy inférieur, hydroxyle, amino, di(alkyl inférieur)amino, (alkyl inférieur)sulfonamido et (acyl inférieur)ami-

no; et

$R^{6*}$ représente un ou deux substituants choisis dans l'ensemble constitué par des atomes d'hydrogène et d'halogène et des groupes alkyle inférieur, alcoxy inférieur, amino, nitro et (alkyl inférieur)sulfonylamido;

avec la condition que le nombre total des atomes de carbone dans $R^1$ plus $R^2$ plus $R^4$ plus $R^5$ doit être égal à cinq ou plus.

**3.** 4,5-dihydro-4-méthyl-1-phényl-3-(2-phényléthyl)-1H-2,4-benzodiazépine,

R-(+)-4,5-dihydro-4-méthyl-1-phényl-3-(2-phényléthyl)-1H-2,4-benzodiazépine,
N-[4-[2-(4,5-dihydro-4-méthyl-1-phényl-1H-2,4-benzodiazépine-3-yl)éthyl]phényl]méthanesulfonamide,
N-[4-[2-(4,5-dihydro-3-éthyl-1-phényl-1H-2,4-benzodiazépine-4-yl)éthyl]phényl]méthanesulfonamide,
N-[4,5-dihydro-4-méthyl-l-phényl-3-(2-phényléthyl)-1H-2,4-benzodiazépine-8-yl]méthanesulfonamide,
1-(4-chlorophényl)-4,5-dihydro-4-méthyl-3-(2-phényléthyl)-1H-2,4-benzodiazépine,
3-[2-(4-chlorophényl)éthyl]-4,5-dihydro-4-méthyl-1-phényl-1H-2,4-benzodiazépine,
3-[2-(4-chlorophényl)éthyl]-4,5-dihydro-1,4-diméthyl-1-phényl-1H-2,4-benzodiazépine,
N,N-diéthyl-4-[4,5-dihydro-5-méthyl-1-phényl-1H-2,4-benzodiazépine-4-yl]benzènesulfonamide,
4,5-dihydro-1-(4-hydroxyphényl)-4-méthyl-3-(2-phényléthyl)-1H-2,4-benzodiazépine,
4,5-dihydro-3-[2-(4-hydroxyphényl)éthyl]-4-méthyl-1-phényl-1H-2,4-benzodiazépine,

ou leurs sels d'addition avec des acides, selon la revendication 2.

**4.** Composé selon la revendication 1, de formule

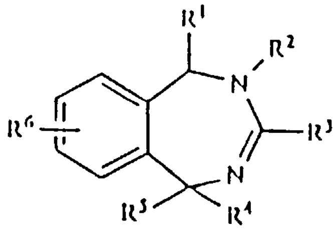

I

dans laquelle

$R^1$ est un atome d'hydrogène ou un radical alkyle inférieur, phényle ou phényle portant un ou deux substituants choisis parmi des groupes alkyle inférieur et alcoxy inférieur, ou le radical naphtyle, thiényle, pyridinyle ou benzyle;

$R^2$ est un atome d'hydrogène ou un radical alkyle inférieur, benzyle, phényle ou phényle substitué par halogène ou par alkyle inférieur ou alcoxy inférieur; ou

$R^2$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;

$R^3$ est un groupe $Y_p-(CH_2)_m-X_n-R^8$, dans lequel

Y représente -NH-, -O-, -S- ou

$$\overset{CH_3}{\underset{|}{}}\!\!-CH-,$$

p est zéro ou un,
m est un nombre entier allant de zéro à sept,
X représente -S-, -O-, $-SO_2-$,

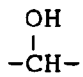, 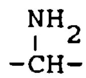, 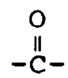, 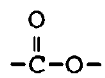,

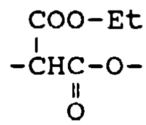

ou -CH=CH-,

n est zéro ou un, et

$R^8$ est un radical alkyle inférieur, phényle, phényle portant un ou deux substituants choisis parmi des atomes d'halogène et des groupes alkyle inférieur et alcoxy inférieur, ou

lorsque n est zéro et m est différent de zéro,
$R^8$ est un outre un atome d'hydrogène ou d'halogène ou un radical benzyl-(alkyl inférieur)amino, di(alkyl inférieur)amino ou un hétérocycle à 5 ou 6 chaînons contenant un ou deux atomes d'azote, ledit hétérocycle étant non substitué ou substitué par un groupe alkyle inférieur;

$R^4$ est un atome d'hydrogène ou un radical alkyle inférieur, allyle, (alcoxy inférieur)-alkyle inférieur, acétyle, (alcoxy inférieur)-carbonyl-alkyle, (alkyl inférieur)-carboxyle ou $\alpha$-hydroxyalkyle inférieur;

$R^5$ est un atome d'hydrogène ou un radical alkyle inférieur, phényle, ou phényle portant un ou deux substituants choisis parmi des atomes d'halogène et des groupes alkyle inférieur, alcoxy inférieur, amino, di(alkyl inférieur) amino et (acyl inférieur)amino; ou est le radical naphtyle, thiényle, pyridinyle ou benzyle; et

$R^6$ représente un ou deux substituants choisis dans l'ensemble constitué par des atomes d'hydrogène et d'halogène et des groupes alkyle inférieur et alcoxy inférieur;

avec la condition que le nombre total des atomes de carbone dans $R^1$ plus $R^2$ plus $R^4$ plus $R^5$ doit être égal à cinq ou plus.

**5.** Procédé pour la préparation d'un composé selon la revendication 1, de formule (V)

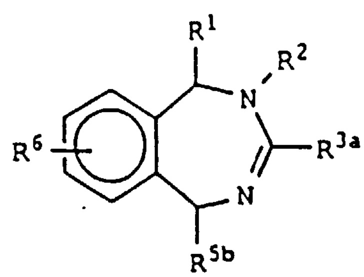

**V**

comprenant a) la mise en réaction d'un composé de formule VI

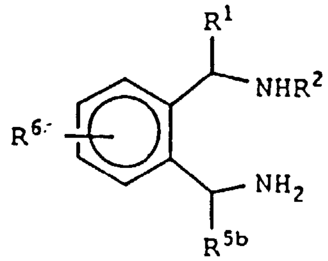

VI d

formules dans lesquelles

- $R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;
- $R^2$ est un atome d'hydrogène ou un radical alkyle inférieur, benzyle, phényle ou phényle substitué par halogène ou par alkyle inférieur ou alcoxy inférieur; ou
- $R^2$ est un radical $-CH_2CH_2R^7$, dans lequel $R^7$ est un groupe alcoxy inférieur, benzyle, di(alkyl inférieur)amino, pyrrolidino, pipéridino, morpholino, phényle ou phényle substitué par amino, nitro ou (alkyl inférieur)sulfonamido;
- $R^{3a}$ est un groupe $(Y^a)_p-(CH_2)_m-(X^a)_n-R^8$, dans lequel
  $Y^a$ représente -O-, -S- ou

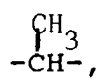

- p est zéro ou un,
- m est un nombre entier allant de zéro à sept,
- $X^a$ représente -S-, $-SO_2-$, -O- ou -CH=CH-,
- n est zéro ou un, et
- $R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur)aminosulfonyle et amino, ou avec

  un hétérocycle à 5 chaînons contenant un ou plusieurs atomes d'oxygène, ou
  lorsque n est zéro et m est différent de zéro,
  $R^8$ est un outre un atome d'halogène ou un radical benzyl-(alkyl inférieur)amino, di(alkyl inférieur)amino ou un hétérocycle à 5 ou 6 chaînons contenant un ou deux atomes d'azote, ledit hétérocycle étant non substitué ou substitué par un groupe alkyle inférieur, ou
  X et $R^8$ forment ensemble un radical cyclohexylidine;

- $R^{5b}$ est un atome d'hydrogène ou un radical alkyle inférieur ou phényle, phényle portant un ou deux substituants choisis dans l'ensemble constitué par des atomes d'halogène et des groupes alkyle inférieur, alcoxy inférieur, ou est le radical naphtyle, thiényle, pyridinyle ou benzyle;
- $R^6$ représente un ou deux substituants choisis indépendamment dans l'ensemble constitué par des atomes d'hydrogène et d'halogène et des groupes alkyle inférieur, alcoxy inférieur, nitro et (alkyl inférieur)sulfonamido;

1) avec un iminoéther, ou un sel de celui-ci, de formule

$$R^{3a}C(OR^{12})NH$$

dans laquelle $R^{12}$ est le groupe méthyle ou éthyle;
2) avec un orthoester de formule $R^{3a}C(OR^{12})_3$;

3) avec un ester de formule $R^{3a}COOR^{12}$ en présence d'un composé de type trialkylaluminium ou
b) la mise en réaction d'un composé de formule XXVII ou XXVIII

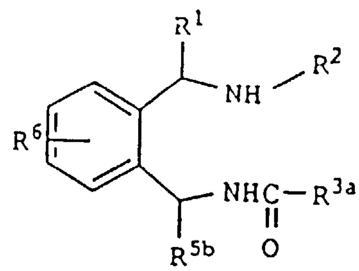

XXVII

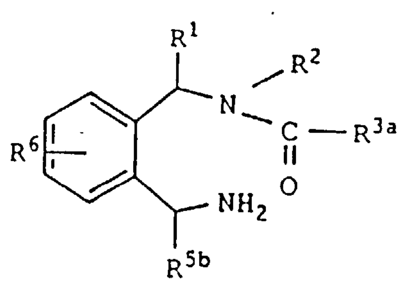

XXVIII

avec un composé de type trialkylaluminium.

**6.** Procédé pour la préparation d'un composé selon la revendication 1, de formule la

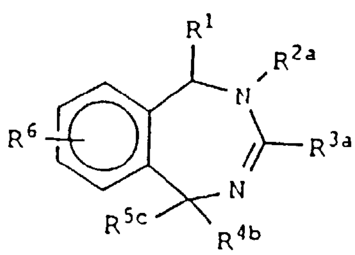

Ia

dans laquelle

$R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;
$R^{2a}$ est un radical alkyle inférieur, benzyle, phényle ou phényle substitué par halogène ou par alkyle inférieur ou alcoxy inférieur; ou
$R^{2a}$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;
$R^{3a}$ est un groupe $(Y^a)_p-(CH_2)_m-(X^a)_n-R^8$, dans lequel

$Y^a$ représente -O-, -S- ou

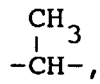

p est zéro ou un,
m est un nombre entier allant de zéro à sept,
$X^a$ représente -S-, $-SO_2-$, -O- ou -CH=CH-,
n est zéro ou un, et
$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur) aminosulfonyle et amino, ou avec

un hétérocycle à 5 chaînons contenant un ou plusieurs atomes d'oxygène, ou
lorsque n est zéro et m est différent de zéro, $R^8$ est un outre un atome d'halogène ou un radical benzyl-(alkyl inférieur)amino, di(alkyl inférieur)amino ou
$R^8$ est un hétérocycle à 5 ou 6 chaînons contenant un ou deux atomes d'azote, ledit hétérocycle étant non substitué ou substitué par un groupe alkyle inférieur, ou
X et $R^8$ forment ensemble un radical cyclohexylidine;

$R^{4b}$ Z est un radical alkyle inférieur, allyle, (alcoxy inférieur)-alkyle inférieur, acétyle, (alcoxy inférieur)carbonyle; (alkyl inférieur)-carboxy-alkyle ou $\alpha$-hydroxyalkyle inférieur;
$R^{5c}$ est un radical phényle, phényle portant un ou deux substituants choisis dans l'ensemble constitué par des atomes d'halogène et des groupes alkyle inférieur, alcoxy inférieur, ou est le radical naphtyle, thiényle ou pyridinyle; et
$R^6$ représente un ou deux substituants choisis indépendamment dans l'ensemble constitué par des atomes d'hydrogène et d'halogène et des groupes alkyle inférieur, alcoxy inférieur, nitro et (alkyl inférieur)-sulfonamido;

comprenant la mise en réaction d'un composé de formule Va

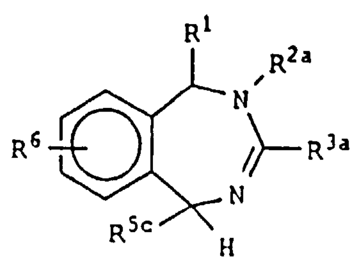

Va

avec une base forte et la mise en réaction avec a) un (alkyl inférieur)aldéhyde ou b) un composé de formule $R^{4b}Z$ dans laquelle Z est un groupe susceptible de déplacement nucléophile.

**7.** Procédé pour la préparation d'un composé selon la revendication 1, de formule VII

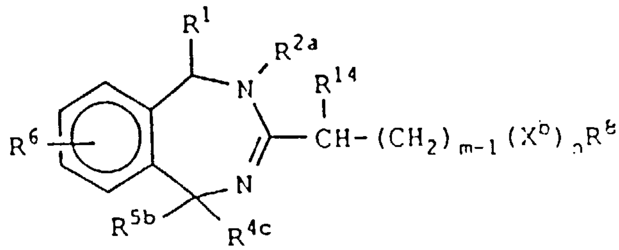

VII

dans laquelle

- $R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;
- $R^{2a}$ est un radical alkyle inférieur, benzyle, phényle ou phényle substitué par halogène ou par alkyle inférieur ou alcoxy inférieur; ou
- $R^{2a}$ est un radical $-CH_2CH_2R^7$, dans lequel $R^7$ est un groupe alcoxy inférieur, phényle, benzyle, di(alkyl inferieur) amino, pyrrolidino, pipéridino ou morpholino;
- m est un nombre entier allant de zéro à sept,
- $X^b$ représente -S-, $-SO_2-$, -O-, -CH=CH-,

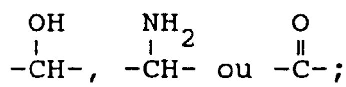

- n est zéro ou un, et
- $R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur)aminosulfonyle et amino, ou avec

  un hétérocycle à 5 chaînons contenant un ou plusieurs atomes d'oxygène, ou
  lorsque n est zéro et m est différent de zéro,
  $R^8$ est un outre un atome d'halogène ou un radical benzyl-(alkyl inférieur)amino, di(alkyl inférieur)amino ou
  $R^8$ est un hétérocycle à 5 ou 6 chaînons contenant un ou deux atomes d'azote, ledit hétérocycle étant non substitué ou substitué par un groupe alkyle inférieur, ou
  X et $R^8$ forment ensemble un radical cyclohexylidine;

- $R^{4c}$ est un radical alkyle inférieur, allyle ou (alcoxy inférieur)-alkyle inférieur;
- $R^{5c}$ est un radical phényle, naphtyle, thiényle, pyridinyle ou phényle portant un ou deux substituants choisis dans l'ensemble constitué par des atomes d'halogène et des groupes alkyle inférieur, alcoxy inférieur;
- $R^6$ représente un ou deux substituants choisis indépendamment dans l'ensemble constitué par des atomes d'hydrogène et d'halogène et des groupes alkyle inférieur, alcoxy inférieur, nitro et (alkyl inférieur)sulfonamido; et
- $R^{14}$ est un atome d'hydrogène ou le groupe méthyle,

comprenant la mise en réaction d'un composé de formule VIIa

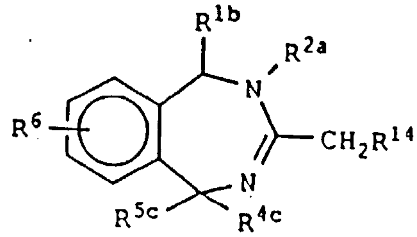

VIIa

avec une base forte, suivie d'une réaction avec un composé électrophile choisi parmi $R^8COOR^{12}$, $R^8CHNR^{12}$, $R^8CHO$, $R^8SSR^8$, $R^8\,(X^b)_n(CH_2)_{m-1}Z$, $R^{12}$ étant le groupe méthyle ou éthyle, et Z étant un groupe susceptible de déplacement nucléophile.

**8.** Utilisation d'un composé selon les revendications 1 à 4, pour la fabrication d'un médicament destiné au traitement de l'arythmie cardiaque chez un patient nécessitant un tel traitement.

**9.** Composition pour le traitement de l'arythmie cardiaque, comprenant une quantité à activité anti-arythmique d'un composé selon l'une quelconque des revendications 1 à 4.

**10.** Composé selon la revendication 1, dans lequel A est le groupe naphtyle.

**Revendications pour l'Etat contractant suivant : ES**

**1.** Procédé pour la préparation d'un composé de formule V

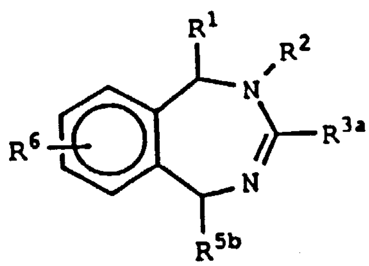

**V**

comprenant a) la mise en réaction d'un composé de formule VId

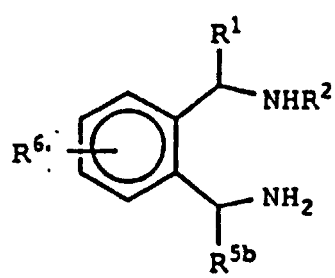

**VI d**

formules dans lesquelles

$R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;

$R^2$ est un atome d'hydrogène ou un radical alkyle inférieur, benzyle, phényle ou phényle substitué par halogène ou par alkyle inférieur ou alcoxy inférieur; ou

$R^2$ est un radical $-CH_2CH_2R^7$, dans lequel $R^7$ est un groupe alcoxy inférieur, benzyle, di(alkyl inférieur)amino, pyrrolidino, pipéridino, morpholino, phényle ou phényle substitué par amino, nitro ou (alkyl inférieur)sulfonamido;

$R^{3a}$ est un groupe $(Y^a)_p-(CH_2)_m-(X^a)_n-R^8$, dans lequel

$Y^a$ représente -O-, -S- ou

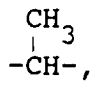

p est zéro ou un,

m est un nombre entier allant de zéro à sept,

$X^a$ représente -S-, $-SO_2-$, -O- ou -CH=CH-,

n est zéro ou un, et

$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur) aminosulfonyle et amino, ou avec

un hétérocycle à 5 chaînons contenant un ou plusieurs atomes d'oxygène, ou

lorsque n est zéro et m est différent de zéro,

$R^8$ est un outre un atome d'halogène ou un radical benzyl-(alkyl inférieur)amino, di(alkyl inférieur) amino, ou $R^8$ est un hétérocycle à 5 ou 6 chaînons contenant un ou deux atomes d'azote, ledit hétérocycle étant non substitué ou substitué par un groupe alkyle inférieur, ou

X et $R^8$ forment ensemble un radical cyclohexylidine;

$R^{5b}$ est un atome d'hydrogène ou un radical alkyle inférieur ou phényle, phényle portant un ou deux substituants choisis dans l'ensemble constitué par des atomes d'halogène et des groupes alkyle inférieur, alcoxy inférieur, ou est le radical naphtyle, thiényle, pyridinyle ou benzyle;

$R^6$ représente un ou deux substituants choisis indépendamment dans l'ensemble constitué par des atomes d'hydrogène et d'halogène et des groupes alkyle inférieur, alcoxy inférieur, nitro et (alkyl inférieur)sulfonamido;

avec la condition que le nombre total des atomes de carbone dans $R^1$ plus $R^2$ plus $R^{5b}$ doit être égal à cinq ou plus, étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins,

1) avec un iminoéther, ou un sel de celui-ci, de formule

$$R^{3a}C(OR^{12})NH$$

dans laquelle $R^{12}$ est le groupe méthyle ou éthyle;

2) avec un orthoester de formule $R^{3a}C(OR^{12})_3$;

3) avec un ester de formule $R^{3a}COOR^{12}$ en présence d'un composé de type trialkylaluminium ou

b) la mise en réaction d'un composé de formule XXVII ou XXVIII

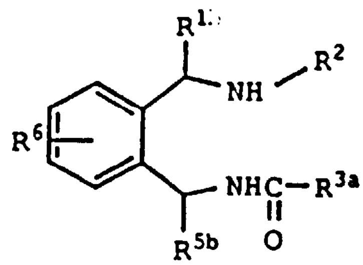

XXVII

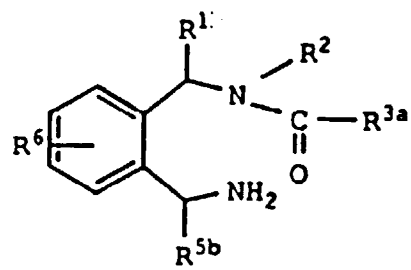

XXVIII

avec un composé de type trialkylaluminium,

si on le désire, la réduction d'un composé obtenu dans lequel $R^6$ est le groupe nitro ou $R^8$ est le groupe nitrophényle, pour préparer le composé correspondant dans lequel $R^6$ est le groupe amino et $R^8$ est le groupe amino-phényle,
si on le désire, la sulfonylation d'un composé obtenu, dans lequel $R^6$ est le groupe amino ou $R^8$ est le groupe aminophényle, à l'aide d'un halogénure d'(alkyl inférieur)sulfonyle, pour préparer le composé correspondant dans lequel $R^6$ est un groupe (alkyl inférieur)sulfonamido ou $R^8$ est un groupe (alkyl inférieur)sulfonamidophényle,
si on le désire, l'acylation d'un composé obtenu, dans lequel $R^8$ est le groupe aminophényle, pour préparer le composé correspondant dans lequel $R^8$ est un groupe (alkyl inférieur)amidophényle,
si on le désire, la coupure d'un composé obtenu, dans lequel $R^8$ est un groupe (alcoxy inférieur)phényle, pour préparer le composé correspondant dans lequel $R^8$ est le groupe hydroxyphényle,
si on le désire, la résolution d'un mélange d'énantiomères obtenus, dans lesquels $R^1$ ou $R^{5b}$ est différent d'un atome d'hydrogène, pour préparer un mélange correspondant enrichi à plus de 90 % en un seul énantiomère, et si on le désire, la conversion d'une base libre obtenue en un sel d'addition avec un acide de celle-ci.

2. Procédé selon la revendication 1, dans lequel $R^1$ est un atome d'hydrogène, $R^2$ est un radical alkyle inférieur, $R^{5b}$ est le radical phényle, naphtyle, thiényle, pyridinyle, benzyle ou un radical phényle portant un ou deux substituants choisis indépendamment parmi des atomes d'halogène et des groupes alkyle inférieur et alcoxy inférieur, $R^6$ est un atome d'hydrogène ou un groupe alcoxy inférieur, nitro ou (alkyl inférieur)sulfonamido, et, dans $R^{3a}$, p est zéro, m est zéro, un ou deux, $R^8$ est le radical phényle ou un radical phényle portant un ou deux substituants choisis parmi des atomes d'halogène et des groupes alkyle inférieur, nitro, hydroxyle, alcoxy inférieur, (alkyl inférieur) sulfonamido et di(alkyl inférieur)aminosulfonyle, ou $R^8$ est un hétérocycle à 5 ou 6 chaînons contenant un ou deux atomes d'azote, ledit hétérocycle étant non substitué ou substitué par un groupe alkyle inférieur.

3. Procédé selon la revendication 2, dans lequel $R^2$ est le groupe méthyle, $R^{5b}$ est le groupe phényle ou un groupe phényle substitué, $R^6$ est un atome d'hydrogène et, dans $R^{3a}$, m est zéro ou deux, $X^a$ est -CH=CH- et $R^8$ est le groupe phényle ou un groupe phényle substitué.

4. Procédé selon la revendication 3, dans lequel $R^{5b}$ est le groupe phényle et $R^{3a}$ est

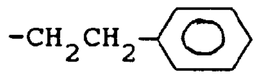 .

**5.** Procédé pour la préparation d'un composé de formule la

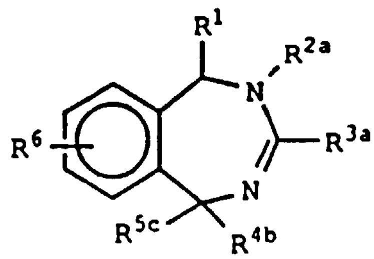

Ia

dans laquelle

$R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;

$R^{2a}$ est un radical alkyle inférieur, benzyle, phényle ou phényle substitué par halogène ou par alkyle inférieur ou alcoxy inférieur; ou

$R^{2a}$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;

$R^{3a}$ est un groupe $(Y^a)_p-(CH_2)_m-(X^a)_n-R^8$, dans lequel

$Y^a$ représente -O-, -S- ou

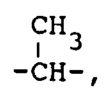

p est zéro ou un,

m est un nombre entier allant de zéro à sept,

$X^a$ représente -S-, $-SO_2-$, -O- ou -CH=CH-,

n est zéro ou un, et

$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur) aminosulfonyle et amino, ou avec

un hétérocycle à 5 chaînons contenant un ou plusieurs atomes d'oxygène, ou lorsque n est zéro et m est différent de zéro,

$R^8$ est un outre un atome d'halogène ou un radical benzyl-(alkyl inférieur)amino, di(alkyl inférieur) amino ou

$R^8$ est un hétérocycle à 5 ou 6 chaînons contenant un ou deux atomes d'azote, ledit hétérocycle étant non substitué ou substitué par un groupe alkyle inférieur, ou

X et $R^8$ forment ensemble un radical cyclohexylidine;

$R^{4b}$ est un radical alkyle inférieur, allyle, (alcoxy inférieur)-alkyle inférieur, acétyle, (alcoxy inférieur)carbonyle; (alcoxy inférieur)-carbonylalkyle ou $\alpha$-hydroxyalkyle inférieur;

$R^{5c}$ est un radical phényle, phényle portant un ou deux substituants choisis dans l'ensemble constitué par des atomes d'halogène et des groupes alkyle inférieur, alcoxy inférieur, ou est le radical naphtyle, thiényle ou

pyridinyle; et

$R^6$ représente un ou deux substituants choisis indépendamment dans l'ensemble constitué par des atomes d'hydrogène et d'halogène et des groupes alkyle inférieur, alcoxy inférieur, nitro et (alkyl inférieur)sulfonamido;

avec la condition que le nombre total des atomes de carbone dans $R^1$ plus $R^{2a}$ plus $R^{4b}$ plus $R^{5c}$ soit égal à cinq ou plus,
étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins,
comprenant la mise en réaction d'un composé de formule Va

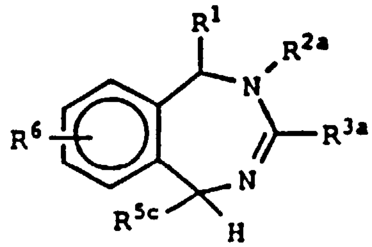

Va

avec une base forte et la mise en réaction avec a) un (alkyl inférieur)aldéhyde ou b) un composé de formule $R^{4b}Z$ dans laquelle Z est un groupe susceptible de déplacement nucléophile,

si on le désire, la réduction d'un composé obtenu dans lequel $R^6$ est le groupe nitro ou $R^8$ est le groupe nitrophényle, pour préparer le composé correspondant dans lequel $R^6$ est le groupe amino et $R^8$ est le groupe aminophényle,
si on le désire, la sulfonylation d'un composé obtenu, dans lequel $R^6$ est le groupe amino ou $R^8$ est le groupe aminophényle, à l'aide d'un halogénure d'(alkyl inférieur)sulfonyle, pour préparer le composé correspondant dans lequel $R^6$ est un groupe (alkyl inférieur)sulfonamido ou $R^8$ est un groupe (alkyl inférieur)sulfonamidophényle,
si on le désire, l'acylation d'un composé obtenu, dans lequel $R^8$ est le groupe aminophényle, pour préparer le composé correspondant dans lequel $R^8$ est un groupe (alkyl inférieur)amidophényle,
si on le désire, la coupure d'un composé obtenu, dans lequel $R^8$ est un groupe (alcoxy inférieur)phényle, pour préparer le composé correspondant dans lequel $R^8$ est le groupe hydroxyphényle,
si on le désire, la résolution d'un mélange d'énantiomères obtenus, dans lesquels $R^1$ est différent d'un atome d'hydrogène ou $R^{5b}$ est différent de $R^{4b}$, pour préparer un mélange correspondant enrichi à plus de 90 % en un seul énantiomère, et
si on le désire, la conversion d'une base libre obtenue en un sel d'addition avec un acide de celle-ci.

**6.** Procédé selon la revendication 5, dans lequel $R^1$ est un atome d'hydrogène, $R^{2a}$ est un radical alkyle inférieur, $R^{4b}$ est un radical alkyle inférieur , $R^{5c}$ est le radical phényle, naphtyle, thiényle, pyridinyle, benzyle ou un radical phényle portant un ou deux substituants choisis indépendamment parmi des atomes d'halogène et des groupes alkyle inférieur et alcoxy inférieur, $R^6$ est un atome d'hydrogène ou un groupe alcoxy inférieur, nitro ou (alkyl inférieur)sulfonamido, et, dans $R^{3a}$, p est zéro, m est zéro, un ou deux, $R^8$ est le radical phényle ou un radical phényle portant un ou deux substituants choisis parmi des atomes d'halogène et des groupes alkyle inférieur, nitro, hydroxyle, alcoxy inférieur, (alkyl inférieur)sulfonamido et di(alkyl inférieur)aminosulfonyle, ou $R^8$ est un hétérocycle à 5 ou 6 chaînons contenant un ou deux atomes d'azote, ledit hétérocycle étant non substitué ou substitué par un groupe alkyle inférieur.

**7.** Procédé selon la revendication 6, dans lequel $R^{2a}$ est le groupe méthyle, $R^{4b}$ est le groupe méthyle, $R^{5c}$ est le groupe phényle ou un groupe phényle substitué, $R^6$ est un atome d'hydrogène et, dans $R^{3a}$, m est zéro ou deux, $X^a$ est -CH=CH- et $R^8$ est le groupe phényle ou un groupe phényle substitué.

**8.** Procédé pour la préparation d'un composé de formule VII

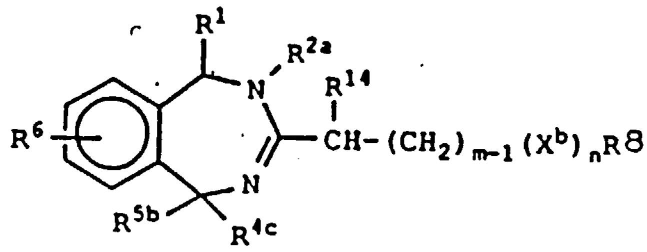

VII

dans laquelle

$R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;

$R^{2a}$ est un radical alkyle inférieur, benzyle, phényle ou phényle substitué par halogène ou par alkyle inférieur ou alcoxy inférieur; ou

$R^{2a}$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;

m est un nombre entier allant de zéro à sept,

$X^b$ représente -S-, $-SO_2-$, -O-, -CH=CH-,

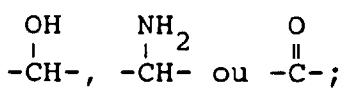

n est zéro ou un, et

$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur)aminosulfonyle et amino, ou avec

un hétérocycle à 5 chaînons contenant un ou plusieurs atomes d'oxygène, ou
lorsque n est zéro et m est différent de zéro,
$R^8$ est un outre un atome d'halogène ou un radical benzyl-(alkyl inférieur)amino, di(alkyl inférieur)amino ou
$R^8$ est un hétérocycle à 5 ou 6 chaînons contenant un ou deux atomes d'azote, ledit hétérocycle étant non substitué ou substitué par un groupe alkyle inférieur, ou
X et $R^8$ forment ensemble un radical cyclohexylidine;

$R^{4c}$ est un radical alkyle inférieur, allyle ou (alcoxy inférieur)-alkyle inférieur;

$R^{5c}$ est un radical phényle, naphtyle, thiényle, pyridinyle ou phényle portant un ou deux substituants choisis dans l'ensemble constitué par des atomes d'halogène et des groupes alkyle inférieur, alcoxy inférieur;

$R^6$ représente un ou deux substituants choisis indépendamment dans l'ensemble constitué par des atomes d'hydrogène et d'halogène et des groupes alkyle inférieur, alcoxy inférieur, nitro et (alkyl inférieur)sulfonamido; et

$R^{14}$ est un atome d'hydrogène ou le groupe méthyle, avec la condition que le nombre total des atomes de carbone dans $R^1$ plus $R^{2a}$ plus $R^{4c}$ plus $R^{5b}$ doit être égal à cinq ou plus,

étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins.
comprenant la mise en réaction d'un composé de formula VIIa

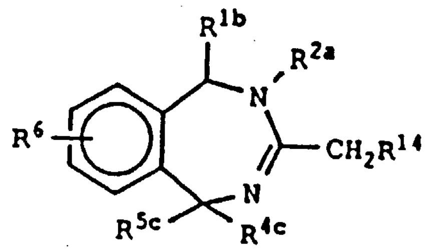

VIIa

avec une base forte, suivie d'une réaction avec un composé électrophile choisi parmi $R^8COOR^{12}$, $R^8CHNR^{12}$, $R^8CHO$, $R^8SSR^8$, $R^8(X^b)_n(CH_2)_{m-1}Z$, $R^{12}$ étant le group méthyle ou éthyle, et Z étant un groupe susceptible de déplacement nucléophile,

si on le désire, la réduction d'un composé obtenu dans lequel $R^6$ est le groupe nitro ou $R^8$ est le groupe nitrophényle, pour préparer le composé correspondant dans lequel $R^6$ est le groupe amino et $R^8$ est le groupe aminophényle,
si on le désire, la sulfonylation d'un composé obtenu, dans lequel $R^6$ est le groupe amino ou $R^8$ est le groupe aminophényle, à l'aide d'un halogénure d'(alkyl inférieur)sulfonyle, pour préparer le composé correspondant dans lequel $R^6$ est un groupe (alkyl inférieur)sulfonamido ou $R^8$ est un groupe (alkyl inférieur)sulfonamidophényle,
si on le désire, l'acylation d'un composé obtenu, dans lequel $R^8$ est le groupe aminophényle, pour préparer le composé correspondant dans lequel $R^8$ est un groupe (alkyl inférieur)amidophényle,
si on le désire, la coupure d'un composé obtenu, dans lequel $R^8$ est un groupe (alcoxy inférieur)phényle, pour préparer le composé correspondant dans lequel $R^8$ est le groupe hydroxyphényle,
si on le désire, la résolution d'un mélange d'énantiomères obtenus, dans lesquels $R^1$ ou $R^{5b}$ est différent d'un atome d'hydrogène, pour préparer un mélange correspondant enrichi à plus de 90 % en un seul énantiomère, et
si on le désire, la conversion d'une base libre obtenue en un sel d'addition avec un acide de celle-ci.

**9.** Procédé pour la préparation d'un composé de formule XXXX

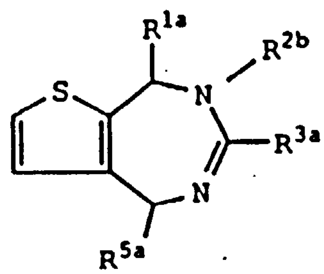

XXXX

dans laquelle

$R^{1a}$ est un atome d'hydrogène ou un radical alkyle inférieur ou phényle;
$R^{2b}$ est un atome d'hydrogène ou un radical alkyle inférieur, di(alkyl inférieur)aminoéthyle, benzyle, phényle, phénéthyle ou phényle substitué par halogène ou par alkyle inférieur ou alcoxy inférieur;
$R^{3a}$ est un groupe $(Y^a)_p$-$(CH_2)_m$-$(X^a)_n$-$R^8$, dans lequel

$Y^a$ représente -O-, -S- ou

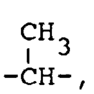

p est zéro ou un,
m est un nombre entier allant de zéro à sept,
$X^a$ représente -S-, $-SO_2-$, -O- ou -CH=CH-,
n est zéro ou un, et
$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur) aminosulfonyle et amino, ou avec

un hétérocycle à 5 chaînons contenant un ou plusieurs atomes d'oxygène, ou
lorsque n est zéro et m est différent de zéro,
$R^8$ est un outre un atome d'halogène ou un radical benzyl-(alkyl inférieur)amino, di(alkyl inférieur) amino ou
un hétérocycle à 5 ou 6 chaînons contenant un ou deux atomes d'azote, ledit hétérocycle étant non substitué ou substitué par un groupe alkyle inférieur, ou
$X^a$ et $R^8$ forment ensemble un radical cyclohexylidine;

$R^{5a}$ est un atome d'hydrogène ou un radical phényle, phényle portant un ou deux substituants choisis dans l'ensemble constitué par des atomes d'halogène et des groupes alkyle inférieur et alcoxy inférieur, ou est le radical naphtyle, thiényle, pyridinyle ou benzyle;

avec la condition que le nombre total des atomes de carbone dans $R^{1a}$ plus $R^{2b}$ plus $R^{5a}$ doit être égal à cinq ou plus, étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins,
comprenant la mise en réaction d'un composé de formule XXXXI

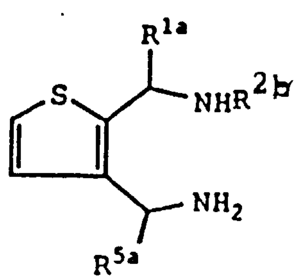

XXXXI

1) avec un iminoéther, ou un sel de celui-ci, de formule $R^{3a}C(OR^{12})NH$, dans laquelle $R^{12}$ est le groupe méthyle ou éthyle,
2) avec un orthoester de formule $R^{3a}C(OR^{12})_3$ ou
3) avec un ester de formule $R^{3a}COOR^{12}$ en présence d'un composé de type trialkylaluminium

et, si on le désire, la conversion d'une base libre obtenue en un sel d'addition avec un acide de celle-ci.

**10.** Procédé selon la revendication 1, pour la préparation d'un composé de formule V

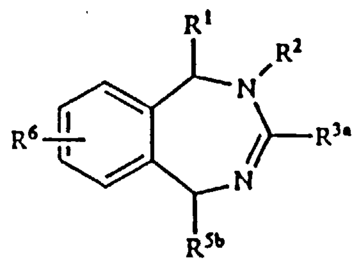

V

comprenant a) la mise en réaction d'un composé de formule VId

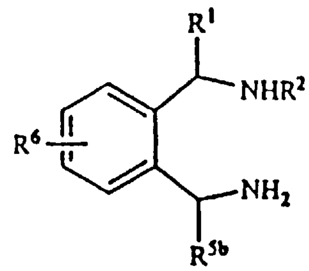

dans laquelle

$R^1$ est un atome d'hydrogène ou un radical alkyle inférieur, phényle ou phényle portant un ou deux substituants choisis parmi des groupes alkyle inférieur et alcoxy inférieur, ou est le radical naphtyle, thiényle, pyridinyle ou benzyle ;

$R^2$ est un atome d'hydrogène ou un radical alkyle inférieur, benzyle, phényle ou phényle substitué par halogène ou par alkyle inférieur ou alcoxy inférieur; ou

$R^2$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;

$R^{3a}$ est un groupe $(Y^a)_p-(CH_2)_m-(X^a)_n-R^8$, dans lequel

- Y représente -O-, -S- ou

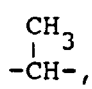

- p est zéro ou un,
- m est un nombre entier allant de zéro à sept,
- $X^a$ représente -S-, $-SO_2-$, -O- ou -CH=CH-,
- n est zéro ou un, et
- $R^8$ est un radical alkyle inférieur, phényle, phényle portant un ou deux substituants choisis parmi des atomes d'halogène et des groupes alkyle inférieur et alcoxy inférieur, ou

  lorsque n est zéro et m est différent de zéro,
  $R^8$ est un outre un atome d'hydrogène ou d'halogène ou un radical benzyl-(alkyl inférieur)amino, di(alkyl inférieur)amino ou
  $R^8$ est un hétérocycle à 5 ou 6 chaînons contenant un ou deux atomes d'azote, ledit hétérocycle étant non substitué ou substitué par un groupe alkyle inférieur;

$R^{5b}$ est un atome d'hydrogène ou un radical alkyle inférieur ou phényle, phényle portant un ou deux substituants choisis dans l'ensemble constitué par des atomes d'halogène et des groupes alkyle inférieur, alcoxy inférieur, ou est le radical naphtyle, thiényle, pyridinyle ou benzyle;

$R^6$ représente un ou deux substituants choisis indépendamment dans l'ensemble constitué par des atomes d'hydrogène et d'halogène et des groupes alkyle inférieur, alcoxy inférieur;

avec la condition que le nombre total des atomes de carbone dans $R^1$ plus $R^2$ plus $R^{5b}$ doit être égal à cinq ou plus, avec un composé choisi dans l'ensemble constitué par

1) un iminoéther, ou un sel de celui-ci, de formule

$$R^{3a}C(OR^{12})NH$$

dans laquelle $R^{12}$ est le groupe méthyle ou éthyle;
2) un orthoester de formule $R^{3a}C(OR^{12})_3$;
3) un ester de formule $R^{3a}COOR^{12}$ en présence d'un composé de type trialkylaluminium ou

comprenant b) la mise en réaction d'un composé de formule XXVII ou XXVIII

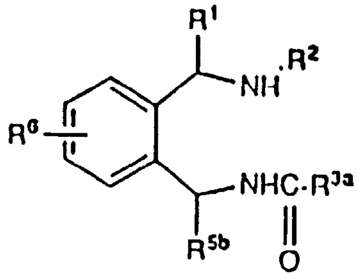

XXVII

ou

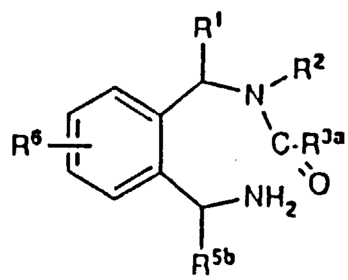

XXVIII

avec un composé de type trialkylaluminium.

**11.** Procédé pour la préparation d'un composé de formule Ia

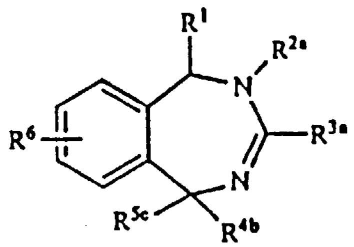

Ia

dans laquelle

$R^1$ est un atome d'hydrogène ou un radical alkyle inférieur, phényle ou phényle portant un ou deux substituants choisis parmi des groupes alkyle inférieur et alcoxy inférieur, ou le radical naphtyle, thiényle, pyridinyle ou benzyle;

$R^{2a}$ est un radical alkyle inférieur, benzyle, phényle ou phényle substitué par halogène ou par alkyle inférieur ou alcoxy inférieur; ou

$R^{2a}$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;

$R^{3a}$ est un groupe $(Y^a)_p-(CH_2)_m-(X^a)_n-R^8$, dans lequel

$Y^a$ représente -O-, -S- ou

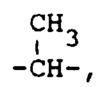

p est zéro ou un,

m est un nombre entier allant de zéro à sept,

$X^a$ représente -S-, $-SO_2-$, -O- ou -CH=CH-,

n est zéro ou un, et

$R^8$ est un radical alkyle inférieur, phényle, phényle portant un ou deux substituants choisis parmi des atomes d'halogène et des groupes alkyle inférieur et alcoxy inférieur, ou

lorsque n est zéro et m est différent de zéro,
$R^8$ est un outre un atome d'hydrogène ou d'halogène ou un radical benzyl-(alkyl inférieur)amino, di(alkyl inférieur)amino ou
$R^8$ est un hétérocycle à 5 ou 6 chaînons contenant un ou deux atomes d'azote, ledit hétérocycle étant non substitué ou substitué par un groupe alkyle inférieur;

$R^{4b}$ est un radical alkyle inférieur, allyle, (alcoxy inférieur)-alkyle inférieur, acétyle, (alcoxy inférieur)carbonyle, (alcoxy inférieur)-carbonylalkyle ou $\alpha$-hydroxyalkyle inférieur;

$R^{5c}$ est un radical phényle, phényle portant un ou deux substituants choisis dans l'ensemble constitué par des atomes d'halogène et des groupes alkyle inférieur, alcoxy inférieur, ou est le radical naphtyle, thiényle ou pyridinyle; et

$R^6$ représente un ou deux substituants choisis dans l'ensemble constitué par des atomes d'hydrogène et d'halogène et des groupes alkyle inférieur et alcoxy inférieur;

avec la condition que le nombre total des atomes de carbone dans $R^1$ plus $R^{2a}$ plus $R^{4b}$ plus $R^{5c}$ doive être égal à cinq ou plus;
comprenant la mise en réaction d'un composé de formule Va

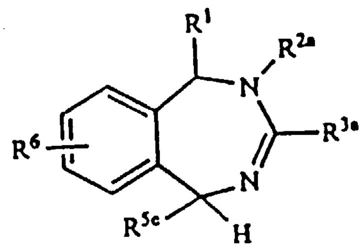

Va

avec une base forte et la mise en réaction avec un composé électrophile approprié.

**12.** Procédé selon la revendication 8, pour la préparation d'un composé de formule VII

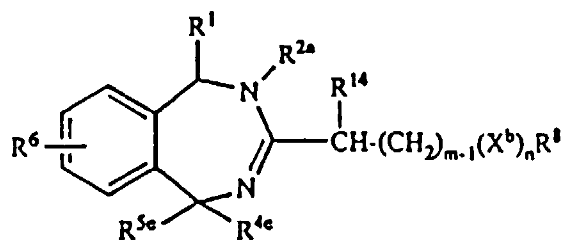

VII

dans laquelle

- $R^1$ est un atome d'hydrogène ou un radical alkyle inférieur, phényle ou phényle portant un ou deux substituants choisis parmi des groupes alkyle inférieur et alcoxy inférieur, ou est le radical naphtyle, thiényle, pyridinyle ou benzyle;
- $R^{2a}$ est un radical alkyle inférieur, benzyle, phényle ou phényle substitué par halogène ou par alkyle inférieur ou alcoxy inférieur; ou
- $R^{2a}$ est un radical $-CH_2CH_2R^7$, dans lequel $R^7$ est un groupe alcoxy inférieur, phényle, benzyle, di(alkyl inférieur) amino, pyrrolidino, pipéridino ou morpholino;
- m est un nombre entier allant de zéro à sept,
- $X^b$ représente -S-, $-SO_2-$, -O-, -CH=CH-,

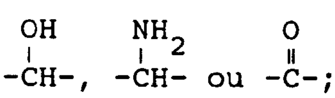

- n est zéro ou un, et
- $R^8$ est un radical alkyle inférieur, phényle, phényle portant un ou deux substituants choisis parmi des atomes d'halogène et des groupes alkyle inférieur et alcoxy inférieur, ou lorsque n est zéro et m est différent de zéro, $R^8$ est un outre un atome d'hydrogène ou d'halogène ou un radical benzyl-(alkyl inférieur)amino, di (alkyl inférieur)amino; ou $R^8$ est un hétérocycle à 5 ou 6 chaînons contenant un ou deux atomes d'azote, ledit hétérocycle étant non substitué ou substitué par un groupe alkyle inférieur;
- $R^{4c}$ est un radical alkyle inférieur, allyle ou (alcoxy inférieur)-alkyle inférieur;
- $R^{5c}$ est le radical phényle ou un radical phényle portant un ou deux substituants choisis dans l'ensemble constitué par des atomes d'halogène et des groupes alkyle inférieur et alcoxy inférieur, ou est le radical naphtyle, thiényle ou pyridinyle;
- $R^6$ représente un ou deux substituants choisis dans l'ensemble constitué par des atomes d'hydrogène et d'halogène et des groupes alkyle inférieur et alcoxy inférieur;

et
$R^{14}$ est un atome d'hydrogène ou le groupe méthyle, avec la condition que le nombre total des atomes de carbone dans $R^1$ plus $R^{2a}$ plus $R^{5c}$ doit être égal à cinq ou plus,
comprenant la mise en réaction d'un composé de formule VIIa

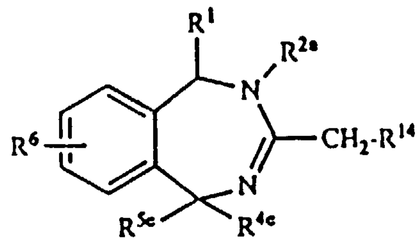

VIIa

avec une base forte, suivie d'une réaction avec un composé électrophile choisi parmi $R^8COOR^{12}$, $R^8CHNR^{12}$, $R^8CHO$, $R^8SSR^8$, $R^8(X^b)_n(CH_2)_{m-1}Z$, $R^{12}$ étant le groupe méthyle ou éthyle, et Z étant un groupe susceptible de déplacement nucléophile,

**13.** Procédé pour la préparation d'un composé de formule générale XXXVI

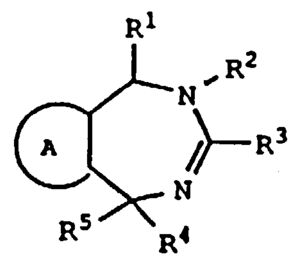

XXXVI

dans laquelle

- A est un cycle choisi dans l'ensemble constitué par les radicaux phényle, thiényle, furannyle, naphtyle, pyridinyle, cyclohexyle et phényle portant un ou deux substituants choisis parmi des atomes d'halogène et des groupes amino, alkyle inférieur, alcoxy inférieur, nitro et (alkyl inférieur)sulfonamido;
- $R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;
- $R^2$ est un atome d'hydrogène ou un radical alkyle inférieur, benzyle, phényle ou phényle substitué par halogène ou par alkyle inférieur ou alcoxy inférieur; ou
- $R^2$ est un radical $-CH_2CH_2R^7$, dans lequel $R^7$ est un groupe alcoxy inférieur, benzyle, di(alkyl inférieur)amino, pyrrolidino, pipéridino, morpholino, phényle ou phényle substitué par amino, nitro ou (alkyl inférieur)sulfonamido;
- $R^3$ est un groupe $Y_p-(CH_2)_m-X_n-R^8$, dans lequel

  - Y représente -NH-, -O-, -S- ou

$$\begin{array}{c} CH_3 \\ | \\ -CH- \end{array},$$

  - p est zéro ou un,
  - m est un nombre entier allant de zéro à sept,
  - X représente -S-, -O-, $-SO_2-$,

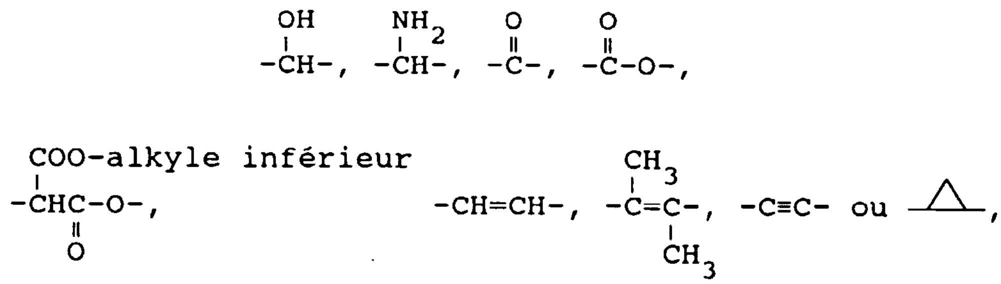

n est zéro ou un, et

$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur) aminosulfonyle et amino, ou avec

un hétérocycle à 5 chaînons contenant un ou plusieurs atomes d'oxygène, ou
lorsque n est zéro et m est différent de zéro,
$R^8$ est un outre un atome d'halogène ou un radical benzyl-(alkyl inférieur)amino, di(alkyl inférieur) amino ou un hétérocycle à 5 ou 6 chaînons contenant un ou deux atomes d'azote, ledit hétérocycle étant non substitué ou substitué par un groupe alkyle inférieur, ou
X et $R^8$ forment ensemble un radical cyclohexylidine;

$R^4$ est un atome d'hydrogène ou un radical alkyle inférieur, allyle, (alcoxy inférieur)-alkyle inférieur, acétyle, (alcoxy inférieur)-carbonyle, (alcoxy inférieur)-carbonyl-alkyle ou $\alpha$-hydroxyalkyle inférieur; et

$R^5$ est un atome d'hydrogène ou un radical alkyle inférieur, naphtyle, thiényle, pyridinyle, benzyle, phényle, ou phényle portant un ou deux substituants choisis indépendamment parmi des atomes d'halogène et des groupes alkyle inférieur, alcoxy inférieur, hydroxyle, amino, di(alkyl inférieur)amino, (alkyl inférieur)sulfonamido et (acyl inférieur)amino;

avec la condition que le nombre total des atomes de carbone dans $R^1$ plus $R^2$ plus $R^4$ plus $R^5$ doit être égal à cinq ou plus,
étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins,
comprenant la mise en réaction d'un composé de formule:

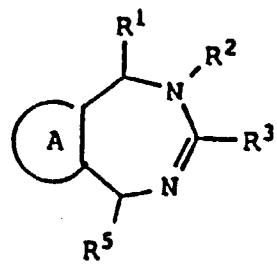

avec du butyllithium et ensuite la mise en réaction du composé résultant avec $R^4Z$, Z étant un groupe susceptible de déplacement nucléophile.

**14.** Composés de formule XXXVI ou leurs sels d'addition avec des acides:

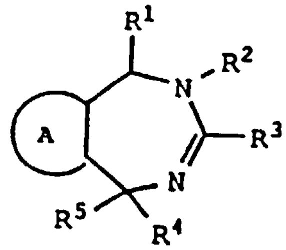

XXXVI

formule dans laquelle

A est un cycle choisi dans l'ensemble constitué par les radicaux phényle, thiényle, furannyle, naphtyle, pyridinyle, cyclohexyle et phényle portant un ou deux substituants choisis parmi des atomes d'halogène et des groupes amino, alkyle inférieur, alcoxy inférieur, nitro et (alkyl inférieur)sulfonamido;

$R^1$ est un atome d'hydrogène ou un radical alkyle inférieur, benzyle, naphtyle, thiényle, pyridinyle, phényle ou phényle portant un ou deux substituants choisis parmi des groupes alkyle inférieur et alcoxy inférieur;

$R^2$ est un atome d'hydrogène ou un radical alkyle inférieur, benzyle, phényle ou phényle substitué par halogène ou par alkyle inférieur ou alcoxy inférieur; ou

$R^2$ est un radical $-CH_2CH_2R^7$, dans lequel $R^7$ est un groupe alcoxy inférieur, benzyle, di(alkyl inférieur)amino, pyrrolidino, pipéridino, morpholino, phényle ou phényle substitué par amino, nitro ou (alkyl inférieur)sulfonamido;

$R^3$ est un groupe $Y_p-(CH_2)_m-X_n-R^8$, dans lequel

Y représente -NH-, -O-, -S- ou

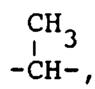

p est zéro ou un,

m est un nombre entier allant de zéro à sept,

X représente -S-, -O-, $-SO_2-$,

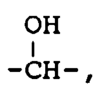 , 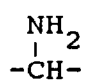 , 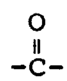 , 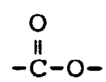 ,

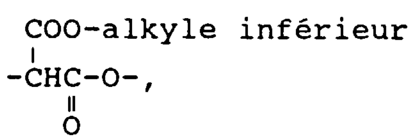 -CH=CH-, 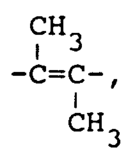 -C≡C- ou 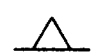 ,

n est zéro ou un, et

$R^8$ est un atome d'hydrogène ou un radical alkyle inférieur, phényle, furannyle, thiényle, pyridinyle, phényle portant un ou deux substituants choisis parmi des atomes d'halogène et des groupes alkyle inférieur, nitro, hydroxyle, alcoxy inférieur, (alkyl inférieur)amido, (alkyl inférieur)sulfonamido, di(alkyl inférieur) aminosulfonyle et amino, ou avec

un hétérocycle à 5 chaînons contenant un ou plusieurs atomes d'oxygène, ou

lorsque n est zéro et m est différent de zéro,

$R^8$ est un outre un atome d'halogène ou un radical benzyl-(alkyl inférieur)amino, di(alkyl inférieur) amino ou un hétérocycle à 5 ou 6 chaînons contenant un ou deux atomes d'azote, ledit hétérocycle étant non substitué ou substitué par un groupe alkyle inférieur, ou

X et $R^8$ forment ensemble un radical cyclohexylidine;

$R^4$ est un atome d'hydrogène ou un radical alkyle inférieur, allyle, (alcoxy inférieur)-alkyle inférieur, acétyle, (alcoxy inférieur)-carbonyle, (alcoxy inférieur)-carbonyl-alkyle ou α-hydroxyalkyle inférieur; et

$R^5$ est un atome d'hydrogène ou un radical alkyle inférieur, naphtyle, thiényle, pyridinyle, benzyle, phényle, ou phényle portant un ou deux substituants choisis indépendamment parmi des atomes d'halogène et des groupes alkyle inférieur, alcoxy inférieur, hydroxyle, amino, di(alkyl inférieur)amino, (alkyl inférieur)sulfonamido et (acyl inférieur)amino;

avec la condition que le nombre total des atomes de carbone dans $R^1$ plus $R^2$ plus $R^4$ plus $R^5$ doit être égal à cinq ou plus, étant entendu que l'expression "alkyle inferieur" désigne une chaîne carbonée saturée linéaire, ramifiée ou cyclique ayant huit atomes de carbone ou moins, et que l'expression "alcoxy inférieur" désigne des substituants alkyloxy linéaires ou ramifiés contenant huit atomes de carbone ou moins.